(19) Europäisches Patentamt
European Patent Office
Office européen des brevets

(11) **EP 4 169 915 A1**

(12) **EUROPEAN PATENT APPLICATION**
published in accordance with Art. 153(4) EPC


(43) Date of publication:
**26.04.2023 Bulletin 2023/17**

(21) Application number: **21829316.5**

(22) Date of filing: **23.06.2021**

(51) International Patent Classification (IPC):
***C07D 403/04*** *(2006.01)* ***A61K 31/53*** *(2006.01)*
***A61P 35/00*** *(2006.01)*

(52) Cooperative Patent Classification (CPC):
**A61K 31/53; A61P 35/00; C07D 403/04**

(86) International application number:
**PCT/CN2021/101779**

(87) International publication number:
**WO 2021/259316 (30.12.2021 Gazette 2021/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.06.2020 CN 202010579560**
**22.05.2021 CN 202110563762**



(71) Applicant: **Shenzhen Forward Pharmaceuticals Co., Limited**
**Shenzhen, Guangdong 518067 (CN)**

(72) Inventors:
- **ZHU, Chenggang**
  **Shenzhen, Guangdong 518067 (CN)**
- **YANG, Xuan**
  **Shenzhen, Guangdong 518067 (CN)**
- **ZHANG, Chaochun**
  **Shenzhen, Guangdong 518067 (CN)**
- **XU, Liangliang**
  **Shenzhen, Guangdong 518067 (CN)**

(74) Representative: **Pharma Patents International AG**
**Leonhardsgraben 52**
**Postfach**
**4001 Basel (CH)**


(54) **CRYSTALLINE FORM OF COMPOUND**


(57) The present application relates to the crystalline form of the mesylate salt, the crystalline form of the hydrochloride salt and the crystalline form of the maleate salt of a compound of formula (I):

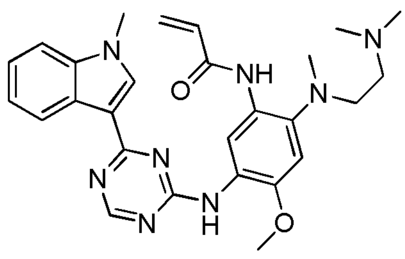

(I)

The present application also relates to a method for treating non-small cell lung cancer (NSCLC) with EGFR exon 20 insertion mutation using the crystalline forms. The present application also relates to synthetic routes for the compound of formula (I).


EP 4 169 915 A1

## Description

### Technical Field

The present application relates to the crystalline form of the mesylate salt, the crystalline form of the hydrochloride salt and the crystalline form of the maleate salt of a compound of formula (I):

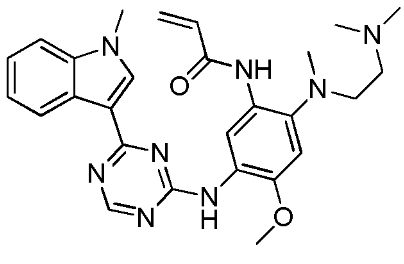

(I)

The present application also relates to a method for treating non-small cell lung cancer (NSCLC) with EGFR exon 20 insertion mutation using the crystalline forms. The present application also relates to synthetic routes for the compound of formula (I).

### Background Art

In the field of anticancer drugs, EGFR is known as a member of the transmembrane protein tyrosine kinase of the erbB receptor family. Homodimerization and/or heterodimerization of the erbB receptor leads to phosphorylation of certain tyrosine residues in the intracellular domain and activates multiple intracellular signaling pathways involved in cell proliferation and survival. Dysregulation of erbB family signaling can lead to cell proliferation, invasion, metastasis, and angiogenesis, and has been reported in cancers such as lung, head and neck, and breast cancer. Therefore, as targets for anticancer drug development, many drugs targeting EGFR have been clinically applied.

Chinese patent CN105461695B discloses a compound of formula (I), which have multiple basic centers. The inhibitory activity of the compound against EGFR activating mutation (such as exon 19 deletion activating mutation, L858R activating mutation, T790M drug resistance mutation and exon 20 insertion mutation) is significantly higher than the inhibitory activity against wild-type EGFR (WT EGFR), and therefore, the compound has higher selectivity and safety, and lower toxic and side effects.

Salt formation studies are usually carried out on organic basic compounds having activity. However, those skilled in the art cannot predict with which acids a specific organic basic compound can form stable salts, or whether a specific organic basic compound or its acid addition salt is more suitable for further drug development, let alone which salt formed has better chemical stability, physical stability or solubility, and which salt has all these better properties. In particular, if an organic basic compound has multiple basic centers, it is impossible for those skilled in the art to predict whether its salts formed with a specific acid in various equivalent ratios have the same properties or different properties, let alone which equivalent ratio of the organic basic compound to the acid is more suitable for further drug development.

After the salt formation study of the organic basic compounds having activity, the crystallization study of the screened salt is usually further carried out, because in addition to the properties brought by the salt formation, the crystallization is likely to provide the salt with additional properties that are more suitable for further drug development, such as better stability, processability, and druggability. However, although there is general guidance on crystallization in the prior art, such general guidance does not enable a person skilled in the art to reasonably determine whether a specific salt formed from a specific organic basic compound and a specific acid can form a particular crystalline form, let alone whether such a particular crystalline form has additional properties that are more suitable for further drug development.

### Summary of the Invention

The present application seeks to find salts of the compound of formula (I) suitable for further drug development. In particular, the present application seeks to find salts with properties suitable for further drug development, including a reasonable salt-forming equivalent ratio, better chemical stability, better physical stability and/or better solubility. The present application is further intended to seek crystalline forms of the salt of the compound of formula (I) suitable for further drug development based on the salts of the compound of formula (I) screened out.

In one aspect according to the invention, the present application relates to a salt formed from the compound of formula (I) with an acid:

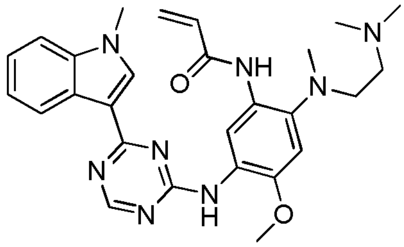

(I)

wherein the acid is selected from hydrochloric acid, methanesulfonic acid, benzenesulfonic acid, ethanesulfonic acid, maleic acid, hydrobromic acid, citric acid, L-tartaric acid, and p-toluenesulfonic acid.

In another aspect according to the invention, the present application relates to a crystalline form of the mesylate salt of the compound of formula (I), a crystalline form of the hydrochloride salt of the compound of formula (I) and a crystalline form of the maleate salt of the compound of formula (I).

Preferably, the crystalline form of the mesylate salt of the compound of formula (I) is crystalline form II-A, which is characterized in that the crystalline form II-A at least has an X-ray powder diffraction pattern having characteristic peaks expressed in 2θ° of 15.12°±0.2°, 22.28°±0.2° and 25.82°±0.2°.

Preferably, the crystalline form of the hydrochloride salt of the compound of formula (I) is crystalline form III-A, which is characterized in that the crystalline form III-A at least has an X-ray powder diffraction pattern having characteristic peaks expressed in 2θ° of 24.85°±0.2°, 14.08°±0.2° and 14.45°±0.2°.

Preferably, the crystalline form of the maleate salt of the compound of formula (I) is crystalline form IV-C, which is characterized in that the crystalline form IV-C at least has an X-ray powder diffraction pattern having characteristic peaks expressed in 2θ° of 20.71°±0.2°, 25.76°±0.2° and 17.00°±0.2°.

In another aspect according to the invention, the present application relates to a pharmaceutical composition comprising the salt or crystalline form according to the invention, which comprises the salt or crystalline form according to the invention and a pharmaceutically acceptable carrier.

In still another aspect according to the invention, the present application relates to a method of treating non-small cell lung cancer (NSCLC) with EGFR exon 20 insertion mutation, comprising administering the salt or crystalline form according to the invention to a patient.

In a further aspect according to the invention, the present application relates to the use of the salt or crystalline form according to the invention in the preparation of a medicament for the treatment of non-small cell lung cancer (NSCLC) with EGFR exon 20 insertion mutation.

## Brief Description of the Drawings

The accompanying drawings are used for a better understanding of the present application, and do not constitute a limitation to the present application.

Figure 1: XRPD pattern of the amorphous free base (Sample No. Y11526-45-RV-FWD1509-AF-SU12).
Figure 2: DSC profile of the amorphous free base (Sample No. Y11526-45-RV-FWD1509-AF-SU12).
Figure 3: TGA profile of the amorphous free base (Sample No. Y11526-45-RV-FWD1509-AF-SU12).
Figure 4: $^1$H-NMR spectrum of the amorphous free base (Sample No. Y11526-45-RV FWD1509-AF-SU12).
Figure 5: PLM photograph of the amorphous free base (Sample No. Y11526-45-RV-FWD1509-AF-SU12).
Figure 6: XRPD pattern of the amorphous 1 eq. (equivalent) hydrochloride salt (Sample No. Y11526-42-SU11-methanol-dichloromethane).
Figure 7: DSC profile of the amorphous 1 eq. hydrochloride salt (Sample No. Y11526-42-SU11-methanol-dichloromethane).
Figure 8: TGA profile of the amorphous 1 eq. hydrochloride salt (Sample No. Y11526-42-SU11-methanol-dichloromethane).
Figure 9: $^1$H-NMR spectrum of the amorphous 1 eq. hydrochloride salt (Sample No. Y11526-42-SU11-methanol-dichloromethane).
Figure 10: PLM photograph of the amorphous 1 eq. hydrochloride salt (Sample No. Y11526-42-SU11-methanol-dichloromethane).

Figure 11: XRPD pattern of the amorphous 2 eq. hydrochloride salt (Sample No. Y11526-28-SUS-methanol-dichloromethane).
Figure 12: DSC profile of the amorphous 2 eq. hydrochloride salt (Sample No. Y11526-28-SUS-methanol-dichloromethane).
Figure 13: TGA profile of the amorphous 2 eq. hydrochloride salt (Sample No. Y11526-28-SUS-methanol-dichloromethane).
Figure 14: $^1$H-NMR spectrum of the amorphous 2 eq. hydrochloride salt (Sample No. Y11526-28-SUS-methanol-dichloromethane).
Figure 15: PLM photograph of the amorphous 2 eq. hydrochloride salt (Sample No. Y11526-28-SUS-methanol-dichloromethane).
Figure 16: XRPD pattern of the amorphous 1 eq. mesylate salt (Sample No. Y11526-42-SU10-methanol-dichloromethane).
Figure 17: DSC profile of the amorphous 1 eq. mesylate salt (Sample No. Y11526-42-SU10-methanol-dichloromethane).
Figure 18: TGA profile of the amorphous 1 eq. mesylate salt (Sample No. Y11526-42-SU10-methanol-dichloromethane).
Figure 19: $^1$H-NMR spectrum of the amorphous 1 eq. mesylate salt (Sample No. Y11526-42-SU10-methanol-dichloromethane).
Figure 20: PLM photograph of the amorphous 1 eq. mesylate salt (Sample No. Y11526-42-SU10-methanol-dichloromethane).
Figure 21: XRPD pattern of the amorphous 2 eq. mesylate salt (Sample No. Y11526-28-SU4-methanol-dichloromethane).
Figure 22: DSC profile of the amorphous 2 eq. mesylate salt (Sample No. Y11526-28-SU4-methanol-dichloromethane).
Figure 23: TGA profile of the amorphous 2 eq. mesylate salt (Sample No. Y11526-28-SU4-methanol-dichloromethane).
Figure 24: $^1$H-NMR spectrum of the amorphous 2 eq. mesylate salt (Sample No. Y11526-28-SU4-methanol-dichloromethane).
Figure 25: PLM photograph of the amorphous 2 eq. mesylate salt (Sample No. Y11526-28-SU4-methanol-dichloromethane).
Figure 26: XRPD pattern of the amorphous 1 eq. besylate salt (Sample No. Y11526-42-SU9-methanol-dichloromethane).
Figure 27: DSC profile of the amorphous 1 eq. besylate salt (Sample No. Y11526-42-SU9-methanol-dichloromethane).
Figure 28: TGA profile of the amorphous 1 eq. besylate salt (Sample No. Y11526-42-SU9-methanol-dichloromethane).
Figure 29: $^1$H-NMR spectrum of the amorphous 1 eq. besylate salt (Sample No. Y11526-42-SU9-methanol-dichloromethane).
Figure 30: PLM photograph of the amorphous 1 eq. besylate salt (Sample No. Y11526-42-SU9-methanol-dichloromethane).
Figure 31: XRPD pattern of the amorphous 2 eq. besylate salt (Sample No. Y11526-30-SU1-water).
Figure 32: DSC profile of the amorphous 2 eq. besylate salt (Sample No. Y11526-30-SU1-water).
Figure 33: TGA profile of the amorphous 2 eq. besylate salt (Sample No. Y11526-30-SU1-water).
Figure 34: $^1$H-NMR spectrum of the amorphous 2 eq. besylate salt (Sample No. Y11526-30-SU1-water).
Figure 35: PLM photograph of the amorphous 2 eq. besylate salt (Sample No. Y11526-30-SU1-water).
Figure 36: XRPD pattern of the amorphous 1 eq. esylate salt (Sample No. Y11526-28-SU7-methanol-dichloromethane).
Figure 37: DSC profile of the amorphous 1 eq. esylate salt (Sample No. Y11526-28-SU7-methanol-dichloromethane).
Figure 38: TGA profile of the amorphous 1 eq. esylate salt (Sample No. Y11526-28-SU7-methanol-dichloromethane).
Figure 39: $^1$H-NMR spectrum of the amorphous 1 eq. esylate salt (Sample No. Y11526-28-SU7-methanol-dichloromethane).
Figure 40: PLM photograph of the amorphous 1 eq. esylate salt (Sample No. Y11526-28-SU7-methanol-dichloromethane).
Figure 41: XRPD pattern of the amorphous 1 eq. maleate salt (Sample No. Y11526-30-SU2-water).
Figure 42: DSC profile of the amorphous 1 eq. maleate salt (Sample No. Y11526-30-SU2-water).
Figure 43: TGA profile of the amorphous 1 eq. maleate salt (Sample No. Y11526-30-SU2-water).
Figure 44: $^1$H-NMR spectrum of the amorphous 1 eq. maleate salt (Sample No. Y11526-30-SU2-water).
Figure 45: PLM photograph of the amorphous 1 eq. maleate salt (Sample No. Y11526-30-SU2-water).

Figure 46: XRPD pattern of the crystalline 1 eq. hydrobromide salt (Sample No. Y11526-33-SU8-methanol-dichloromethane).
Figure 47: DSC profile of the crystalline 1 eq. hydrobromide salt (Sample No. Y11526-33-SU8-methanol-dichloromethane).
Figure 48: TGA profile of the crystalline 1 eq. hydrobromide salt (Sample No. Y11526-33-SU8-methanol-dichloromethane).
Figure 49: $^1$H-NMR spectrum of the crystalline 1 eq. hydrobromide salt (Sample No. Y11526-33-SU8-methanol-dichloromethane).
Figure 50: PLM photograph of the crystalline 1 eq. hydrobromide salt (Sample No. Y11526-33-SU8-methanol-dichloromethane).
Figure 51: XRPD pattern of the amorphous nitrate salt (Sample No. Y11526-18-RV7-methanol-dichloromethane).
Figure 52: $^1$H-NMR spectrum of the amorphous nitrate salt (Sample No. Y11526-18-RV7-methanol-dichloromethane).
Figure 53: XRPD pattern of the amorphous sulfate salt (Sample No. Y11526-15-RV3-methanol-dichloromethane).
Figure 54: DSC profile of the amorphous sulfate salt (Sample No. Y11526-15-RV3-methanol-acetonitrile).
Figure 55: TGA profile of the amorphous sulfate salt (Sample No. Y11526-15-RV3-methanol-acetonitrile).
Figure 56: $^1$H-NMR spectrum of the amorphous sulfate salt (Sample No. Y11526-15-RV3-methanol-acetonitrile).
Figure 57: PLM photograph of the amorphous sulfate salt (Sample No. Y11526-15-RV3-methanol-acetonitrile).
Figure 58: XRPD pattern of the amorphous 2 eq. p-tosylate salt (Sample No. Y11526-23-FD11-water).
Figure 59: DSC profile of the amorphous 2 eq. p-tosylate salt (Sample No. Y11526-23-FD11-water).
Figure 60: TGA profile of the amorphous 2 eq. p-tosylate salt (Sample No. Y11526-23-FD11-water).
Figure 61: $^1$H-NMR spectrum of the amorphous 2 eq. p-tosylate salt (Sample No. Y11526-23-FD11-water).
Figure 62: PLM photograph of the amorphous 2 eq. p-tosylate salt (Sample No. Y11526-23-FD11-water).
Figure 63: photograph of the amorphous 2 eq. p-tosylate salt (Sample No. Y11526-23-FD11-water).
Figure 64: XRPD pattern of the amorphous sulfosalicylate salt (Sample No. Y11526-25-FD13-1,4-dioxane).
Figure 65: DSC profile of the amorphous sulfosalicylate salt (Sample No. Y11526-25-FD13-1,4-dioxane).
Figure 66: TGA profile of the amorphous sulfosalicylate salt (Sample No. Y11526-25-FD13-1,4-dioxane).
Figure 67: $^1$H-NMR spectrum of the amorphous sulfosalicylate salt (Sample No. Y11526-25-FD13-1,4-dioxane).
Figure 68: PLM photograph of the amorphous sulfosalicylate salt (Sample No. Y11526-25-FD 13-1,4-dioxane).
Figure 69: XRPD pattern of the amorphous sulfosalicylate salt (Sample No. Y11526-25-FD12-1,4-dioxane).
Figure 70: DSC profile of the amorphous sulfosalicylate salt (Sample No. Y11526-25-FD12-1,4-dioxane).
Figure 71: TGA profile of the amorphous sulfosalicylate salt (Sample No. Y11526-25-FD12-1,4-dioxane).
Figure 72: $^1$H-NMR spectrum of the amorphous sulfosalicylate salt (Sample No. Y11526-25-FD12-1,4-dioxane).
Figure 73: PLM photograph of the amorphous sulfosalicylate salt (Sample No. Y11526-25-FD12-1,4-dioxane).
Figure 74: XRPD pattern of the amorphous L-malate salt (Sample No. Y11526-17-RV6-methanol-dichloromethane).
Figure 75: DSC profile of the amorphous L-malate salt (Sample No. Y11526-17-RV6-methanol-dichloromethane).
Figure 76: TGA profile of the amorphous L-malate salt (Sample No. Y11526-17-RV6-methanol-dichloromethane).
Figure 77: $^1$H-NMR spectrum of the amorphous L-malate salt (Sample No. Y11526-17-RV6-methanol-dichloromethane).
Figure 78: PLM photograph of the amorphous L-malate salt (Sample No. Y11526-17-RV6-methanol-dichloromethane).
Figure 79: XRPD pattern of the amorphous 1 eq. citrate salt (Sample No. Y11526-10-FD6-1,4-dioxane).
Figure 80: DSC profile of the amorphous 1 eq. citrate salt (Sample No. Y11526-10-FD6-1,4-dioxane).
Figure 81: TGA profile of the amorphous 1 eq. citrate salt (Sample No. Y11526-10-FD6-1,4-dioxane).
Figure 82: $^1$H-NMR spectrum of the amorphous 1 eq. citrate salt (Sample No. Y11526-10-FD6-1,4-dioxane).
Figure 83: PLM photograph of the amorphous 1 eq. citrate salt (Sample No. Y11526-10-FD6-1,4-dioxane).
Figure 84: XRPD pattern of the amorphous 1 eq. L-tartrate salt (Sample No. Y11526-10-FD7-1,4-dioxane).
Figure 85: DSC profile of the amorphous 1 eq. L-tartrate salt (Sample No. Y11526-10-FD7-1,4-dioxane).
Figure 86: TGA profile of the amorphous 1 eq. L-tartrate salt (Sample No. Y11526-10-FD7-1,4-dioxane).
Figure 87: $^1$H-NMR spectrum of the amorphous 1 eq. L-tartrate salt (Sample No. Y11526-10-FD7-1,4-dioxane).
Figure 88: HPLC chromatogram overlay of the amorphous 1 eq. L-tartrate salt (Sample No. Y11526-10-FD7-1,4-dioxane).
Figure 89: XRPD overlay of the amorphous free base in a solid storage stability test, in which from top to bottom, the first trace is the XRPD of the crystalline free base as a control, the second trace is the solid obtained in Test BS2, the third trace is the XRPD of the solid obtained in Test BS1, and the fourth trace is the XRPD of the original solid.
Figure 90: XRPD overlay of the amorphous 1 eq. hydrochloride salt in a solid storage stability test, in which the upper trace is the XRPD of the solid obtained in Test BS2, the middle trace is the XRPD of the solid obtained in Test BS1, and the lower trace is the XRPD of the original solid.

Figure 91: XRPD overlay of the amorphous 2 eq. hydrochloride salt in a solid storage stability test, in which the upper trace is the XRPD of the solid obtained in Test BS2, the middle trace is the XRPD of the solid obtained in Test BS1, and the lower trace is the XRPD of the original solid.

Figure 92: Photograph of the amorphous 2 eq. hydrochloride salt in a solid storage stability test, in which the left picture is the photograph of the original solid, and the right picture is the photograph of the solid obtained in Test BS1.

Figure 93: XRPD overlay of the amorphous 1 eq. mesylate salt in a solid storage stability test, in which the upper trace is the XRPD of the solid obtained in Test BS2, the middle trace is the XRPD of the solid obtained in Test BS1, and the lower trace is the XRPD of the original solid.

Figure 94: XRPD overlay of the amorphous 2 eq. mesylate salt in a solid storage stability test, in which the upper trace is the XRPD of the solid obtained in Test BS2, the middle trace is the XRPD of the solid obtained in Test BS1, and the lower trace is the XRPD of the original solid.

Figure 95: Photograph of the amorphous 2 eq. mesylate salt in a solid storage stability test, in which the left picture is the photograph of the original solid, the middle picture is the photograph of the solid obtained in Test BS1, and the right picture is the photograph of the solid obtained in Test BS2.

Figure 96: XRPD overlay of the amorphous 1 eq. besylate salt in a solid storage stability test, in which the upper trace is the XRPD of the solid obtained in Test BS2, the middle trace is the XRPD of the solid obtained in Test BS1, and the lower trace is the XRPD of the original solid.

Figure 97: Photograph of the amorphous 2 eq. besylate salt in a solid storage stability test, in which the left picture is the photograph of the original solid, and the right picture is the photograph of the solid obtained in Test BS1.

Figure 98: XRPD overlay of the amorphous 2 eq. besylate salt in a solid storage stability test, in which the upper trace is the XRPD of the solid obtained in Test BS2, and the lower trace is the XRPD of the original solid.

Figure 99: Photograph of the amorphous 1 eq. esylate salt in a solid storage stability test, in which the left picture is the photograph of the original solid, and the right picture is the photograph of the solid obtained in Test BS1.

Figure 100: XRPD overlay of the amorphous 1 eq. esylate salt in a solid storage stability test, in which the upper trace is the XRPD of the solid obtained in Test BS2, and the lower trace is the XRPD of the original solid.

Figure 101: XRPD overlay of the amorphous 1 eq. maleate salt in a solid storage stability test, in which the upper trace is the XRPD of the solid obtained in Test BS2, the middle trace is the XRPD of the solid obtained in Test BS1, and the lower trace is the XRPD of the original solid.

Figure 102: Photograph of the amorphous 1 eq. maleate salt in a solid storage stability test, in which the left picture is the photograph of the original solid, and the right picture is the photograph of the solid obtained in Test BS1.

Figure 103: XRPD overlay of the crystalline 1 eq. hydrobromide salt in a solid storage stability test, in which the upper trace is the XRPD of the solid obtained in Test BS2, the middle trace is the XRPD of the solid obtained in Test BS1, and the lower trace is the XRPD of the original solid.

Figure 104: XRPD pattern of the solid of the crystalline 1 eq. hydrobromide salt obtained in a 2 h solid solubility test, in which the upper trace is the XRPD of the solid obtained in the 2 h solid solubility test, and the lower trace is the XRPD of the original solid.

Figure 105: X-ray powder diffraction pattern of crystalline form II-A of the mesylate salt.

Figure 106: Thermogravimetric analysis (TGA)/differential scanning calorimetry (DSC) profile of crystalline form II-A of the mesylate salt.

Figure 107: X-ray powder diffraction pattern of crystalline form III-A of the hydrochloride salt.

Figure 108: Thermogravimetric analysis (TGA)/differential scanning calorimetry (DSC) profile of crystalline form III-A of the hydrochloride salt.

Figure 109: X-ray powder diffraction pattern of crystalline form IV-C of the maleate salt.

Figure 110: Thermogravimetric analysis (TGA)/differential scanning calorimetry (DSC) profile of crystalline form IV-C of the maleate salt.

## Detailed Description of the Invention

In one aspect, the present application relates to a salt formed from a compound of formula (I) with an acid:

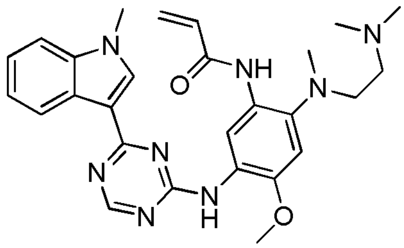

(I)

wherein the acid is selected from hydrochloric acid, methanesulfonic acid, benzenesulfonic acid, ethanesulfonic acid, maleic acid, hydrobromic acid, citric acid, L-tartaric acid, and p-toluenesulfonic acid. Preferably, the acid is selected from hydrochloric acid, methanesulfonic acid, and maleic acid. More preferably, the equivalent ratio of the compound of formula (I) to the acid is 1:1 or 1:2. Further preferably, the acid is methanesulfonic acid. More further preferably, the equivalent ratio of the compound of formula (I) to the acid is 1:1.

The inventors of the present application have found that when the compound of formula (I) is reacted with an organic or inorganic acid, it is unexpected that the compound of formula (I) can form salts with some acids, but cannot form salts with other acids at all.

More surprisingly, when the compound of formula (I) is reacted with an acid selected from hydrochloric acid, methanesulfonic acid, benzenesulfonic acid, ethanesulfonic acid, maleic acid, hydrobromic acid, sulfosalicylic acid, L-malic acid, citric acid, or L-tartaric acid in a molar charge ratio of compound of formula (I):acid of 1:1, some of the salts formed have a stoichiometric ratio of compound of formula (I):acid of 1:1, while the compound of formula (I) and some acids cannot form salts having a stoichiometric ratio of compound of formula (I): acid of 1:1. Specifically:
hydrochloric acid, methanesulfonic acid, benzenesulfonic acid, ethanesulfonic acid, maleic acid, hydrobromic acid, citric acid, and L-tartaric acid enable the resulting salts to have a stoichiometric ratio of compound of formula (I):acid of 1:1 (the corresponding salts are hereinafter referred to as 1 eq. hydrochloride salt, 1 eq. mesylate salt, 1 eq. besylate salt, 1 eq. esylate salt, 1 eq. maleate salt, 1 eq. hydrobromide salt, 1 eq. citrate salt and 1 eq. L-tartrate, respectively), while sulfosalicylic acid and L-malic acid cannot enable the resulting salts to have a stoichiometric ratio of compound of formula (I): acid of 1:1.

In addition, more surprisingly, when the compound of formula (I) is reacted with an acid selected from hydrochloric acid, methanesulfonic acid, benzenesulfonic acid, nitric acid, sulfuric acid, p-toluenesulfonic acid, or sulfosalicylic acid in a molar charge ratio of compound of formula (I):acid of 1:2, some of the salts formed have a stoichiometric ratio of compound of formula (I):acid of 1:2, while the compound of formula (I) and some acids cannot form salts having a stoichiometric ratio of compound of formula (I):acid of 1:2. Specifically:
hydrochloric acid, methanesulfonic acid, benzenesulfonic acid, and p-toluenesulfonic acid enable the resulting salts to have a stoichiometric ratio of compound of formula (I):acid of 1:2 (the corresponding salts are hereinafter referred to as 2 eq. hydrochloride salt, 2 eq. mesylate salt, 2 eq. besylate salt, and 2 eq. p-tosylate salt, respectively), while nitric acid, sulfuric acid, and sulfosalicylic acid cannot enable the resulting salts to have a stoichiometric ratio of compound of formula (I):acid of 1:2.

More specifically, the salts obtained as above include 1 eq. hydrochloride salt, 1 eq. mesylate salt, 1 eq. besylate salt, 1 eq. esylate salt, 1 eq. maleate salt, 1 eq. hydrobromide salt, 1 eq. citrate salt, 1 eq. L-tartrate, 2 eq. hydrochloride salt, 2 eq. mesylate salt, 2 eq. besylate salt and 2 eq. p-tosylate salt, and sulfosalicylate salt, L-malate salt, nitrate salt, and sulfate salt that do not have a reasonable salt-forming equivalent ratio. For example, the salts obtained as above include amorphous 1 eq. hydrochloride salt, amorphous 1 eq. mesylate salt, amorphous 1 eq. besylate salt, amorphous 1 eq. esylate salt, amorphous 1 eq. maleate salt, crystalline 1 eq. hydrobromide salt, amorphous 1 eq. citrate salt, amorphous 1 eq. L-tartrate, amorphous 2 eq. hydrochloride salt, amorphous 2 eq. mesylate salt, amorphous 2 eq. besylate salt and amorphous 2 eq. p-tosylate salt, and amorphous sulfosalicylate salt, amorphous L-malate salt, amorphous nitrate salt, and amorphous sulfate salt that do not have a reasonable salt-forming equivalent ratio.

It is crucial for drug development whether the salts obtained as above have chemical and physical stability at the end of the preparation. The chemical and physical stability of the salts at the end of the preparation includes:

the chemical stability of the salts at the end of the preparation, that is, the purity of the salts obtained at the end of the preparation is not significantly reduced compared with the purity of the free base used to prepare the salts; and the physical stability of the salts at the end of the preparation, that is, the salts do not undergo crystal phase transformation, moisture absorption and/or color change, etc. immediately at the end of the preparation.

Different salts behave differently in terms of chemical and physical stability at the end of the preparation,

indicating that it is unpredictable how a certain salt behaves. Specifically:

1 eq. citrate salt, 1 eq. L-tartrate, nitrate salt and L-malate salt are chemically unstable at the end of the preparation;
2 eq. p-tosylate salt and sulfate salt are physically unstable at the end of the preparation; and
1 eq. hydrochloride salt, 2 eq. hydrochloride salt, 1 eq. mesylate salt, 2 eq. mesylate salt, 1 eq. besylate salt, 2 eq. besylate salt, 1 eq. esylate salt, 1 eq. maleate salt, 1 eq. hydrobromide salt, and sulfosalicylate salt are chemically and physically stable at the end of the preparation.

The salts as above which have a reasonable salt-forming equivalent ratio and are chemically and physically stable at the end of the preparation include 1 eq. hydrochloride salt, 2 eq. hydrochloride salt, 1 eq. mesylate salt, 2 eq. mesylate salt, 1 eq. besylate salt, 2 eq. besylate salt, 1 eq. esylate salt, 1 eq. maleate salt, and 1 eq. hydrobromide salt. It is also crucial for drug development whether these salts remain chemically and physically stable after storage. The chemical and physical stability of the salts after storage includes:

the chemical stability of the salts after storage, that is, the purity of the salts after storage is not significantly reduced compared with the purity of the salts before storage; and
the physical stability of the salts after storage, that is, the salts do not undergo crystal phase transformation, moisture absorption and/or color change, etc. after storage.

Different salts behave differently in terms of chemical and physical stability after storage, indicating that it is unpredictable how a certain salt behaves. Specifically:

1 eq. hydrochloride salt, 1 eq. besylate salt, and 1 eq. maleate salt are chemically unstable under storage conditions of "solid/25°C/60%RH/open/1 week" and/or "solid/60°C/closed container/1 week";
2 eq. hydrochloride salt, 2 eq. mesylate salt, 2 eq. besylate salt, 1 eq. esylate salt, and 1 eq. maleate salt are physically unstable under storage conditions of "solid/25°C/60%RH/open/1 week" and/or "solid/60°C/closed container/1 week"; and
1 eq. mesylate salt and 1 eq. hydrobromide salt are chemically and physically stable under storage conditions of "solid/25°C/60%RH/open/1 week" and "solid/60°C/closed container/1 week".

The salts as above which have a reasonable salt-forming equivalent ratio and are chemically and physically stable at the end of the preparation include 1 eq. hydrochloride salt, 2 eq. hydrochloride salt, 1 eq. mesylate salt, 2 eq. mesylate salt, 1 eq. besylate salt, 2 eq. besylate salt, 1 eq. esylate salt, 1 eq. maleate salt, and 1 eq. hydrobromide salt. It is also crucial for drug development whether these salts have better solubility. Different salts have different solubility, indicating that it is unpredictable what solubility a certain salt may have. Specifically:

2 eq. besylate salt and 1 eq. hydrobromide salt do not achieve a solubility of 2 mg/mL in some solvents that mimic physiological conditions; and
1 eq. hydrochloride salt, 2 eq. hydrochloride salt, 1 eq. mesylate salt, 2 eq. mesylate salt, 1 eq. besylate salt, 1 eq. esylate salt, and 1 eq. maleate salt have a solubility greater than 2 mg/mL in various solvents that mimic physiological conditions.

As can be seen from the above contents, 1 eq. mesylate salt surprisingly and unexpectedly has a reasonable salt-forming equivalent ratio, better chemical stability, better physical stability and better solubility simultaneously, which makes it as a salt of the compound of formula (I) suitable for further drug development.
In another aspect according to the invention, the present application relates to a pharmaceutical composition comprising the salt according to the invention, which comprises the salt according to the invention and a pharmaceutically acceptable carrier.
In still another aspect according to the invention, the present application relates to a method of treating non-small cell lung cancer (NSCLC) with EGFR exon 20 insertion mutation, comprising administering the salt according to the invention to a patient.
In a further aspect according to the invention, the present application relates to the use of the salt according to the invention in the preparation of a medicament for the treatment of non-small cell lung cancer (NSCLC) with EGFR exon 20 insertion mutation.
In addition to the properties brought about by the salt formation, since crystallization may provide the salt with additional properties that are more suitable for further drug development, such as better stability, processability, and druggability, crystallization studies were carried out on some of the salts prepared after the above salt formation study and screening of the compound of formula (I). After conducting crystallization studies on the prepared salts, the inventors

of the present application have found that the mesylate, hydrochloride salt and maleate salts of the compound of formula (I) can form crystals and have obtained the crystalline forms of the salts, and have found that the crystalline forms of the mesylate, hydrochloride salt and maleate salts of the compound of formula (I) have improved bioavailability and increased dynamic solubility.

The present invention therefore provides the following technical solutions relating to the crystalline forms.

In one aspect according to the invention, the present application relates to a crystalline form II-A of the mesylate salt of the compound of formula (I), characterized in that the crystalline form II-A at least has an X-ray powder diffraction pattern having characteristic peaks expressed in 2θ° of 15.12°±0.2°, 22.28°±0.2° and 25.82°±0.2°.

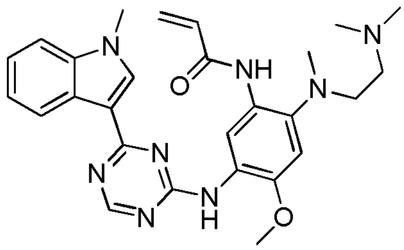

(I)

Further, the above crystalline form II-A is characterized in that the crystalline form II-A at least has an X-ray powder diffraction pattern having characteristic peaks expressed in 2θ° of one or all of 19.62°±0.2° and 26.24°±0.2°.

Further, the above crystalline form II-A is characterized in that the crystalline form II-A at least has an X-ray powder diffraction pattern having characteristic peaks expressed in 2θ° of one or all of 10.36°±0.2° and 18.94°±0.2°.

Further, the above crystalline form II-A is characterized in that the crystalline form II-A at least has an X-ray powder diffraction pattern having characteristic peaks expressed in 2θ° of one or all of 7.22°±0.2°, 8.62°±0.2°, 9.48°±0.2°, 15.74°±0.2°, 16.46°±0.2°, 16.92°±0.2°, 17.70°±0.2°, 19.30°±0.2°, 20.36°±0.2°, 20.81°±0.2°, 24.06°±0.2°, 24.78°±0.2°, and 25.54°±0.2°.

Further, the above crystalline form II-A is characterized in that the X-ray powder diffraction pattern thereof has characteristic peaks at diffraction angle 2θ° of 7.22°±0.2°, 8.62°±0.2°, 9.48°±0.2°, 10.36°±0.2°, 15.12°±0.2°, 15.74°±0.2°, 16.46°±0.2°, 16.92°±0.2°, 17.70°±0.2°, 18.94°±0.2°, 19.30°±0.2°, 19.62°±0.2°, 20.36°±0.2°, 20.81°±0.2°, 22.28°±0.2°, 24.06°±0.2°, 24.78°±0.2°, 25.54°±0.2°, 25.82°±0.2°, and 26.24°±0.2°.

Further, the above crystalline form II-A is characterized in that the X-ray powder diffraction pattern of the crystalline form II-A is substantially as shown in Figure 105.

Further, the above crystalline form II-A is characterized in that the crystalline form II-A has a weight loss of 0.56% at 200.0°C.

Further, the above crystalline form II-A is characterized in that the crystalline form II-A begins to show an endothermic peak when heated to 255.9°C.

Further, the above crystalline form II-A is characterized in that its thermogravimetric analysis (TGA)/differential scanning calorimetry (DSC) profile is substantially as shown in Figure 106.

Further, the above crystalline form II-A is characterized in that the molar ratio of the compound of formula (I) to methanesulfonic acid is 1:1.

In addition, the present invention provides a method for preparing the crystalline form II-A of the mesylate salt of the compound of formula (I), comprising:

a) suspending the compound of formula (I) in a first solvent;
b) increasing the temperature to 20-80°C, and adding dropwise a methanesulfonic acid solution dissolved in a second solvent beforehand; and
c) crystallizing the mixture and filtrating to obtain the crystalline form II-A.

Further, the first solvent is water, ketone, cyclic ether or nitrile solvent or a mixed solvent thereof; and the second solvent is water, ketone, cyclic ether or nitrile solvent or a mixed solvent thereof. Yet further, the first solvent is water, ketone, cyclic ether or nitrile solvent or a mixed solvent thereof; and the second solvent is a ketone, cyclic ether or nitrile solvent, or a mixed solvent of water and a ketone, cyclic ether or nitrile solvent. Yet further, the ketone solvent includes, but is not limited to, acetone, the cyclic ether solvent includes, but is not limited to, tetrahydrofuran or 1,4-dioxane, and the nitrile solvent includes, but is not limited to, acetonitrile.

Further, in the mixed solvent of the ketone, cyclic ether or nitrile solvent and water, the volume ratio of the ketone, cyclic ether or nitrile solvent to water is 5:1-25:1, and still further, the volume ratio of the ketone, cyclic ether or

nitrile solvent to water is 9:1-15:1.

Further, the temperature is increased in step b) to 20-50°C.

The present invention provides a method for preparing the crystalline form II-A of the mesylate salt of the compound of formula (I), comprising:

a) suspending the compound of formula (I) in a first solvent;
b) increasing the temperature to 20-80°C, and adding dropwise a methanesulfonic acid solution dissolved in a second solvent beforehand; and
c) adding dropwise a third solvent;
d) crystallizing the mixture and filtrating to obtain the crystalline form II-A.

Further, the first solvent is water, ketone, cyclic ether or nitrile solvent or a mixed solvent thereof; and the second solvent is water, ketone, cyclic ether or nitrile solvent or a mixed solvent thereof. Yet further, the first solvent is water, ketone, cyclic ether or nitrile solvent or a mixed solvent thereof; and the second solvent is a ketone, cyclic ether or nitrile solvent, or a mixed solvent of water and a ketone, cyclic ether or nitrile solvent. Yet further, the ketone solvent includes, but is not limited to, acetone, the cyclic ether solvent includes, but is not limited to, tetrahydrofuran or 1,4-dioxane, and the nitrile solvent includes, but is not limited to, acetonitrile.

Further, in the mixed solvent of the ketone, cyclic ether or nitrile solvent and water, the volume ratio of the ketone, cyclic ether or nitrile solvent to water is 5:1-25:1, and still further, the volume ratio of the ketone, cyclic ether or nitrile solvent to water is 9:1-16:1.

Further, the temperature is increased in step b) to 20-50°C.

Further, the third solvent is a $C_{6-7}$ alkane, ether or ester solvent. Yet further, the $C_{6-7}$ alkane solvent includes, but is not limited to, n-heptane; the ether solvent includes, but is not limited to, methyl tert-butyl ether; and the ester solvent includes, but is not limited to, methyl formate, ethyl acetate, isopropyl acetate, propyl acetate, or butyl acetate.

In one aspect, the present application relates to a crystalline form III-A of the hydrochloride salt of the compound of formula (I), characterized in that the crystalline form III-A at least has an X-ray powder diffraction pattern having characteristic peaks expressed in 2θ° of 24.85°±0.2°, 14.08°±0.2° and 14.45°±0.2°.

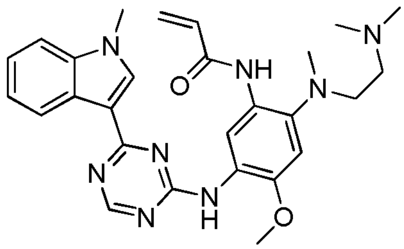

(I)

Further, the above crystalline form III-A is characterized in that the crystalline form III-A at least has an X-ray powder diffraction pattern having characteristic peaks expressed in 2θ° of one or all of 25.87°±0.2 and 6.55°±0.2°.

Further, the above crystalline form III-A is characterized in that the crystalline form III-A at least has an X-ray powder diffraction pattern having characteristic peaks expressed in 2θ° of one or all of 13.10°±0.2° and 19.07°±0.2°.

Further, the above crystalline form III-A is characterized in that the crystalline form III-A at least has an X-ray powder diffraction pattern having characteristic peaks expressed in 2θ° of one or all of 10.36°±0.2°, 12.28°±0.2°, 15.98°±0.2°, 17.33°±0.2°, 19.57°±0.2°, 21.15°±0.2°, 21.72°±0.2°, 24.30°±0.2°, and 33.29°±0.2°.

Further, the above crystalline form III-A is characterized in that the X-ray powder diffraction pattern thereof has characteristic peaks at diffraction angle 2θ° of 6.55°±0.2°, 10.36°±0.2°, 12.28°±0.2°, 13.10°±0.2°, 14.08°±0.2°, 14.45°±0.2°, 15.98°±0.2°, 17.33°±0.2°, 19.07°±0.2°, 19.57°±0.2°, 21.15°±0.2°, 21.72°±0.2°, 24.30°±0.2°, 24.85°±0.2°, 25.87°±0.2°, and 33.29°±0.2°.

Further, the above crystalline form III-A is characterized in that the X-ray powder diffraction pattern of the crystalline form III-A is substantially as shown in Figure 107.

Further, the above crystalline form III-A is characterized in that the crystalline form III-A has a weight loss of 1.05% at 150.0°C.

Further, the above crystalline form III-A is characterized in that the crystalline form III-A begins to show an endothermic peak when heated to 193.3°C.

Further, the above crystalline form III-A is characterized in that its thermogravimetric analysis (TGA)/differential scanning calorimetry (DSC) profile is substantially as shown in Figure 108.

Further, the above crystalline form III-A is characterized in that the molar ratio of the compound of formula (I) to hydrochloric acid is 1: 1.

In addition, the present invention provides a method for preparing the crystalline form III-A of the hydrochloride salt of the compound of formula (I), comprising:

a) suspending the compound of formula (I) in a first solvent;
b) increasing the temperature to 20-80°C, and adding dropwise a concentrated hydrochloric acid (about 37%) solution dissolved in a second solvent beforehand; and
c) crystallizing the mixture and filtrating to obtain the crystalline form III-A.

Further, the first solvent is water, ketone, cyclic ether or nitrile solvent or a mixed solvent thereof; and the second solvent is water, ketone, cyclic ether or nitrile solvent or a mixed solvent thereof. Yet further, the first solvent is water, ketone, cyclic ether or nitrile solvent or a mixed solvent thereof; and the second solvent is an alcohol or nitrile solvent, or a mixed solvent of water and an alcohol or nitrile solvent. Yet further, the ketone solvent includes, but is not limited to, acetone, the cyclic ether solvent includes, but is not limited to, tetrahydrofuran or 1,4-dioxane, the alcohol solvent includes, but is not limited to, ethanol, and the nitrile solvent includes, but is not limited to, acetonitrile.

Further, in the mixed solvent of the alcohol or nitrile solvent and water, the volume ratio of the alcohol or nitrile solvent to water is 5:1-25:1, and still further, the volume ratio of the alcohol or nitrile solvent to water is 9:1-15:1.

Further, the temperature is increased in step b) to 20-50°C.

The present invention provides a method for preparing the crystalline form III-A of the hydrochloride salt of the compound of formula (I), comprising:

a) suspending the compound of formula (I) in a first solvent;
b) increasing the temperature to 20-80°C, and adding dropwise a concentrated hydrochloric acid (about 37%) solution dissolved in a second solvent beforehand; and
c) adding dropwise a third solvent;
d) crystallizing the mixture and filtrating to obtain the crystalline form III-A.

Further, the first solvent is water, ketone, cyclic ether or nitrile solvent or a mixed solvent thereof; and the second solvent is water, ketone, cyclic ether or nitrile solvent or a mixed solvent thereof. Yet further, the first solvent is water, ketone, cyclic ether or nitrile solvent or a mixed solvent thereof; and the second solvent is an alcohol or nitrile solvent, or a mixed solvent of water and an alcohol or nitrile solvent. Yet further, the ketone solvent includes, but is not limited to, acetone, the cyclic ether solvent includes, but is not limited to, tetrahydrofuran or 1,4-dioxane, the alcohol solvent includes, but is not limited to, ethanol, and the nitrile solvent includes, but is not limited to, acetonitrile.

Further, in the mixed solvent of the alcohol or nitrile solvent and water, the volume ratio of the alcohol or nitrile solvent to water is 5:1-25:1, and still further, the volume ratio of the alcohol or nitrile solvent to water is 9:1-15:1.

Further, the temperature is increased in step b) to 20-50°C.

Further, the third solvent is a $C_{6-7}$ alkane, ether or ester solvent. Yet further, the $C_{6-7}$ alkane solvent includes, but is not limited to, n-heptane; the ether solvent includes, but is not limited to, methyl tert-butyl ether; and the ester solvent includes, but is not limited to, methyl formate, ethyl acetate, isopropyl acetate, propyl acetate, or butyl acetate.

In one aspect, the present application relates to a crystalline form IV-C of the maleate salt of the compound of formula (I), characterized in that the crystalline form IV-C at least has an X-ray powder diffraction pattern having characteristic peaks expressed in 2θ° of 20.71°±0.2°, 25.76°±0.2° and 17.00°±0.2°.

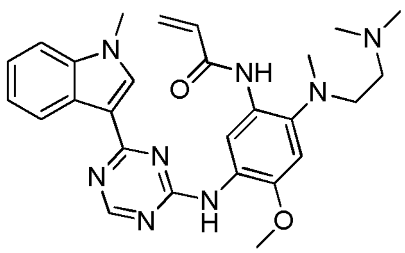

(I)

Further, the above crystalline form IV-C is characterized in that the crystalline form IV-C at least has an X-ray powder diffraction pattern having characteristic peaks expressed in 2θ° of one or all of 6.47°±0.2° and 10.63°±0.2°.

Further, the above crystalline form IV-C is characterized in that the crystalline form IV-C at least has an X-ray

powder diffraction pattern having characteristic peaks expressed in 2θ° of one or all of 28.70°±0.2° and 16.42°±0.2°.

Further, the above crystalline form IV-C is characterized in that the crystalline form IV-C at least has an X-ray powder diffraction pattern having characteristic peaks expressed in 2θ° of one or all of 8.44°±0.2°, 9.15°±0.2°, 12.70°±0.2°, 15.48°±0.2°, 18.19°±0.2°, 22.43°±0.2°, and 31.39°±0.2°.

Further, the above crystalline form IV-C is characterized in that the X-ray powder diffraction pattern thereof has characteristic peaks at diffraction angle 2θ° of 6.47°±0.2°, 8.44°±0.2°, 9.15°±0.2°, 10.63°±0.2°, 12.70°±0.2°, 15.48°±0.2°, 16.42°±0.2°, 17.00°±0.2°, 18.19°±0.2°, 20.71°±0.2°, 22.43°±0.2°, 25.76°±0.2°, 28.70°±0.2°, and 31.39°±0.2°.

Further, the above crystalline form IV-C is characterized in that the X-ray powder diffraction pattern of the crystalline form IV-C is substantially as shown in Figure 109.

Further, the above crystalline form IV-C is characterized in that the crystalline form IV-C has a weight loss of 1.96% at 220.0°C.

Further, the above crystalline form IV-C is characterized in that the crystalline form IV-C begins to show an endothermic peak when heated to 255.5°C.

Further, the above crystalline form IV-C is characterized in that its thermogravimetric analysis (TGA)/differential scanning calorimetry (DSC) profile is substantially as shown in Figure 110.

Further, the above crystalline form IV-C is characterized in that the molar ratio of the compound of formula (I) to maleic acid is 1: 1.

In addition, the present invention provides a method for preparing the crystalline form IV-C of the maleate of the compound of formula (I), comprising:

a) suspending the compound of formula (I) in a first solvent;
b) increasing the temperature to 20-80°C, and adding dropwise a maleic acid solution dissolved in a second solvent beforehand; and
c) crystallizing the mixture and filtrating to obtain the crystalline form IV-C.

Further, the first solvent is water, ketone, cyclic ether or nitrile solvent or a mixed solvent thereof; and the second solvent is water, ketone, cyclic ether or nitrile solvent or a mixed solvent thereof. Yet further, the first solvent is water, ketone, cyclic ether or nitrile solvent or a mixed solvent thereof; and the second solvent is a ketone, cyclic ether or nitrile solvent, or a mixed solvent of water and a ketone, cyclic ether or nitrile solvent. Yet further, the ketone solvent includes, but is not limited to, acetone, the cyclic ether solvent includes, but is not limited to, tetrahydrofuran or 1,4-dioxane, and the nitrile solvent includes, but is not limited to, acetonitrile.

Further, in the mixed solvent of the ketone, cyclic ether or nitrile solvent and water, the volume ratio of the ketone, cyclic ether or nitrile solvent to water is 5:1-25:1, and still further, the volume ratio of the ketone, cyclic ether or nitrile solvent to water is 9:1-15:1.

Further, the temperature is increased in step b) to 20-50°C.

The present invention provides a method for preparing the crystalline form IV-C of the maleate of the compound of formula (I), comprising:

a) suspending the compound of formula (I) in a first solvent;
b) increasing the temperature to 20-80°C, and adding dropwise a maleic acid solution dissolved in a second solvent beforehand; and
c) adding dropwise a third solvent;
d) crystallizing the mixture and filtrating to obtain the crystalline form IV-C.

Further, the first solvent is water, ketone, cyclic ether or nitrile solvent or a mixed solvent thereof; and the second solvent is water, ketone, cyclic ether or nitrile solvent or a mixed solvent thereof. Yet further, the first solvent is water, ketone, cyclic ether or nitrile solvent or a mixed solvent thereof; and the second solvent is a ketone, cyclic ether or nitrile solvent, or a mixed solvent of water and a ketone, cyclic ether or nitrile solvent. Yet further, the ketone solvent includes, but is not limited to, acetone, the cyclic ether solvent includes, but is not limited to, tetrahydrofuran or 1,4-dioxane, and the nitrile solvent includes, but is not limited to, acetonitrile.

Further, in the mixed solvent of the ketone, cyclic ether or nitrile solvent and water, the volume ratio of the ketone, cyclic ether or nitrile solvent to water is 5:1-25:1, and still further, the volume ratio of the ketone, cyclic ether or nitrile solvent to water is 9:1-16:1.

Further, the temperature is increased in step b) to 20-50°C.

Further, the third solvent is a $C_{6-7}$ alkane, ether or ester solvent. Yet further, the $C_{6-7}$ alkane solvent includes, but is not limited to, n-heptane; the ether solvent includes, but is not limited to, methyl tert-butyl ether; and the ester solvent includes, but is not limited to, methyl formate, ethyl acetate, isopropyl acetate, propyl acetate, or butyl acetate.

Since the formation of the crystalline form is accidental and unpredictable, the obtaining of the crystalline form II-A of the mesylate salt of the compound of formula (I), the crystalline form III-A of the hydrochloride salt of the compound of formula (I) and the crystalline form IV-C of the maleate salt of the compound of formula (I) is in itself surprising and unexpected. The inventors of the present application have also surprisingly and unexpectedly discovered that these crystalline forms have improved bioavailability and increased dynamic solubility.

In one aspect, the present application relates to a method for preparing the compound of formula (I), comprising the following steps:

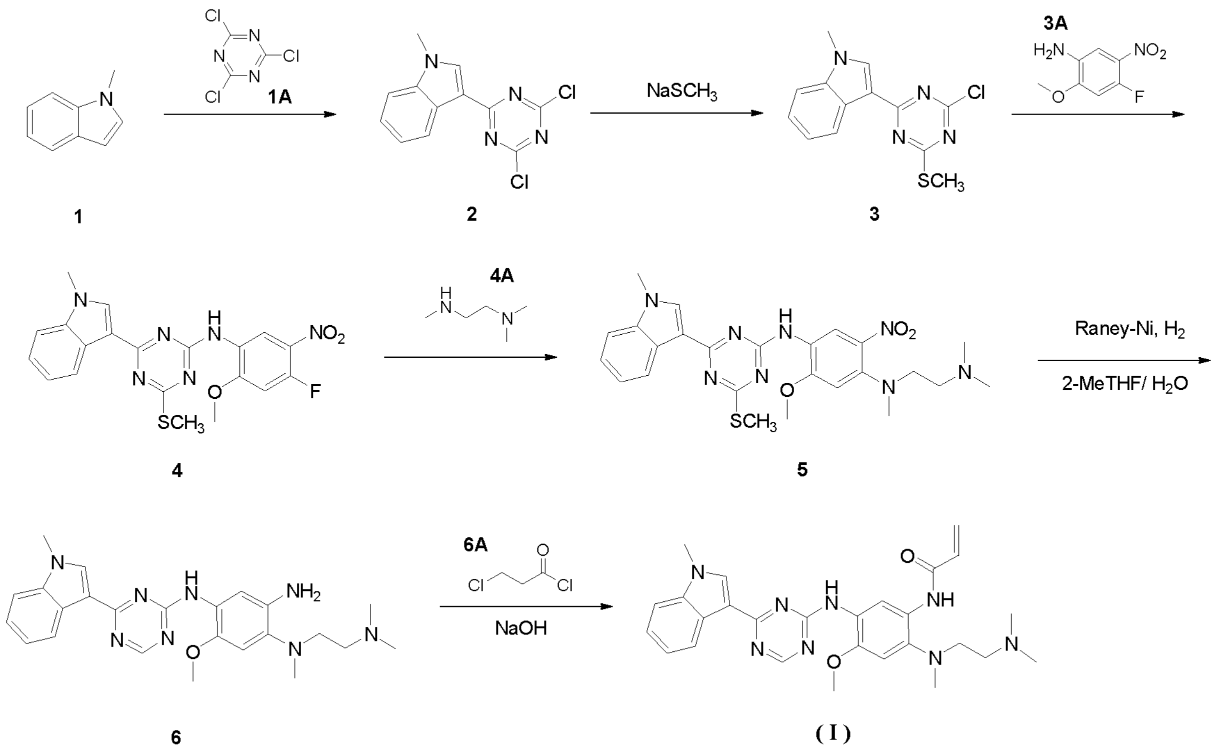


Further, the method for preparing the compound of formula (I) is as follows:

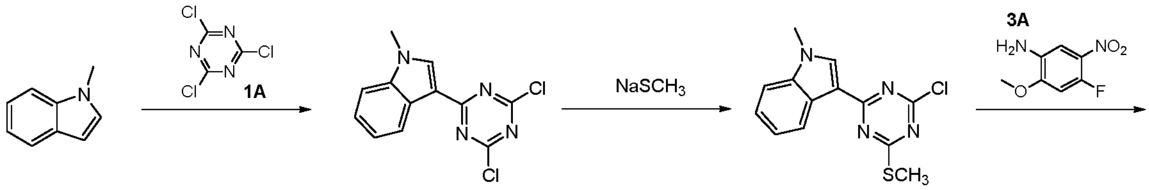


1 2 3

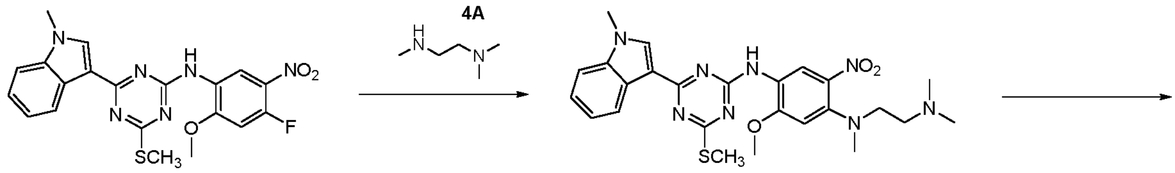


4 5

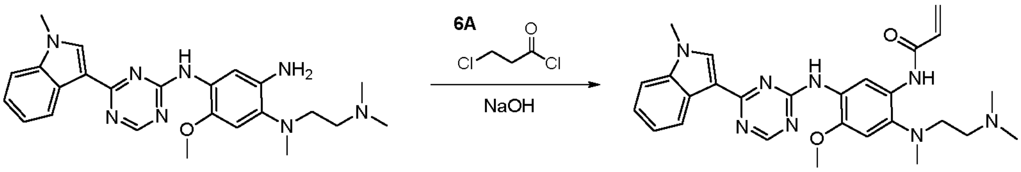


6 (I)

Compound **1A** and Compound **1** are subjected to a substitution or coupling reaction to give Compound **2.** Compound **2** is reacted with sodium thiomethoxide to give Compound **3.** Compound **3** and Compound **3A** are subjected to a substitution or coupling reaction to give Compound **4,** which is then subjected to a nucleophilic substitution reaction with Compound **4A** to give Compound **5.** The nitro group and methylthio group of Compound **5** are sequentially reduced and removed to obtain Compound **6.** Compound **6** and 3-chloropropionyl chloride **6A** are subjected to acylation and elimination under basic conditions to give the compound of formula (I). The reducing agents employed in the method for reducing the nitro group are those well known in the art, including, but not limited to, iron powder, zinc powder, sodium sulfide, palladium carbon (Pd/C)/hydrogen, Raney-Ni/hydrogen, platinum dioxide/hydrogen, etc.

In one aspect according to the invention, the present application relates to a method for preparing the mesylate salt of the compound of formula (I), comprising the following steps:

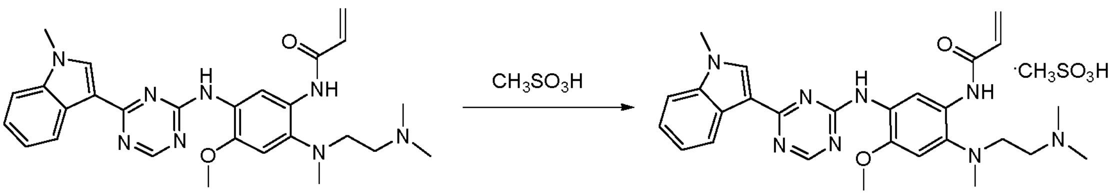


(I)

Further, the compound of formula (I) is directly reacted with methanesulfonic acid in a solvent to form a salt to obtain the mesylate salt of the compound of formula (I), and the solvent includes, but is not limited to, a mixed solvent of acetone and water.

In one aspect according to the invention, the present application relates to a method for preparing the hydrochloride salt of the compound of formula (I), comprising the following steps:

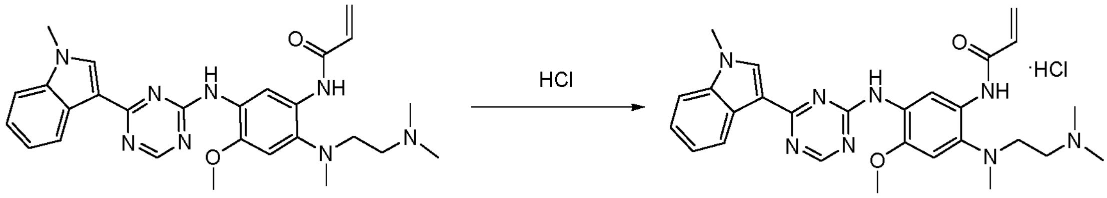


(I)

Further, the compound of formula (I) is directly reacted with hydrochloric acid in a solvent to form a salt to obtain the hydrochloride salt of the compound of formula (I), and the solvent includes, but is not limited to, a mixed solvent of ethanol and water.

In one aspect according to the invention, the present application relates to a method for preparing the maleate salt of the compound of formula (I), comprising the following steps:

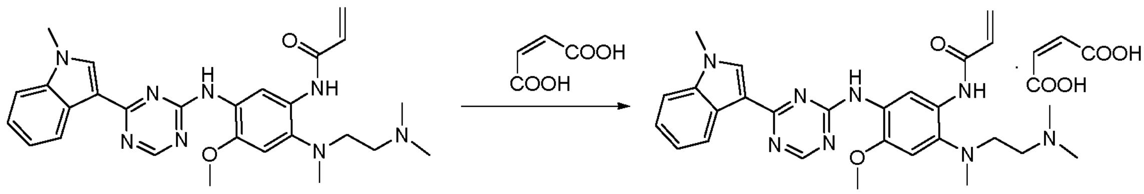

(I)

Further, the compound of formula (I) is directly reacted with maleic acid in a solvent to form a salt to obtain the maleate salt of the compound of formula (I), and the solvent includes, but is not limited to, a mixed solvent of ethanol and water.

The present invention provides a pharmaceutical composition comprising any one of the above salts or crystalline forms of the compound of formula (I) and a pharmaceutically acceptable carrier.

The present invention also provides any one of the above salts or crystalline forms of the compound of formula (I) for use as an antitumor agent.

The present invention also provides the use of any one of the above salts or crystalline forms of the compound of formula (I) in the preparation of a medicament for the treatment of diseases, especially cancer, mediated by EGFR activating, drug resistance or exon 20 insertion mutation in mammals, especially humans.

The present invention also provides the use of any one of the above salts or crystalline forms of the compound of formula (I) in the preparation of a medicament for the treatment of cancer.

The present invention also provides the use of any one of the above salts or crystalline forms of the compound of formula (I) for the treatment of diseases, especially cancer, mediated by EGFR activating, drug resistance or exon 20 insertion mutation in mammals, especially humans.

The present invention also provides a method for the treatment of diseases, especially cancer, mediated by EGFR activating, drug resistance or exon 20 insertion mutation in mammals, especially humans, comprising administering to a patient any one of the above salts or crystalline forms of the compound of formula (I) or a pharmaceutical composition comprising a therapeutically effective amount of any one of the above salts or crystalline forms of the compound of formula (I) and a pharmaceutically acceptable carrier, excipient or diluent.

The present invention also provides a method for the treatment of cancer, comprising administering to a patient any one of the above salts or crystalline forms of the compound of formula (I) or a pharmaceutical composition comprising a therapeutically effective amount of any one of the above salts or crystalline forms of the compound of formula (I) and a pharmaceutically acceptable carrier, excipient or diluent.

The cancer mentioned in the present invention includes, but is not limited to, for example ovarian cancer, non-small cell lung cancer, small cell lung cancer, cervical cancer, colorectal cancer, breast cancer, pancreatic cancer, glioma, glioblastoma, melanoma, prostatic cancer, leukemia, lymphoma, non-Hodgkin lymphoma, gastric cancer, lung cancer, hepatocellular carcinoma, gastrointestinal stromal tumor, thyroid cancer, bile duct cancer, endometrial cancer, kidney cancer, anaplastic large cell lymphoma, acute myeloid leukemia, multiple myeloma, or mesothelioma. The above salts or crystalline forms of the compound of formula (I) has better application especially for tumor types with epidermal growth factor receptor exon 20 insertion mutation. Further, the cancer is non-small cell lung cancer.

The epidermal growth factor receptor EGFR exon 20 insertion mutation mentioned in the present invention include, but are not limited to, V774insHV insertion mutation, D770insNPG insertion mutation, N771insH insertion mutation, V769insASV insertion mutation, A763insFQEA insertion mutation, D770insSVD insertion mutation and the like. For example, any one of the above salts or crystalline forms of the compound of formula (I) of the present invention can be used as an agent for the treatment of non-small cell lung cancer with epidermal growth factor receptor exon 20 insertion mutation.

Any one of the above salts or crystalline forms of the compound of formula (I) of the present invention can be administered to mammals, including humans, by oral, rectal, parenteral (intravenous, intramuscular, or subcutaneous), topical (dust, ointment, or drops), or intratumoral administration.

The dosage of any one of the above salts or crystalline forms of the compound of formula (I) of the present invention may be about 0.05-50 mg/kg body weight/day, for example 0.1-45 mg/kg body weight/day, more for example 0.5-35 mg/kg body weight/day.

Any one of the above salts or crystalline forms of the compound of formula (I) of the present invention can be formulated into solid preparations for oral administration, including, but not limited to, capsules, tablets, pills, powders and granules. In these solid dosage forms, any one of the above salts or crystalline forms of the compound of formula (I) of the present invention are mixed as an active ingredient with at least one conventional inert excipient (or carrier), such as sodium citrate or dicalcium phosphate, or mixed with the following ingredients: (1) fillers or compatibilizers, such as starch, lactose, sucrose, glucose, and mannitol; (2) binders, such as hydroxymethyl cellulose, alginate, gelatin, polyvinylpyrrolidone, sucrose, and gum arabic; (3) humectants, such as glycerin; (4) disintegrating agents, such as agar, calcium carbonate, potato starch or cassava starch, and alginic acid; (5) retarding solvents, such as paraffin; (6) absorption accelerators, such as quaternary ammonium compounds; (7) wetting agents, such as cetyl alcohol and glyceryl monostearate; (8) adsorbents, such as kaolin; and (9) lubricating agents, such as talc, calcium stearate, magnesium stearate, solid polyethylene glycol, and sodium lauryl sulfate, or mixtures thereof. Buffers may also be included in capsules, tablets and pills.

The solid dosage forms such as tablets, dragees, capsules, pills and granules can be coated or microencapsulated with coatings and shell materials such as enteric coatings and other materials well known in the art. They may contain opacifying agents, and the release of the active ingredient in such compositions may be in a certain part of the digestive tract in a delayed manner. Examples of embedding components that can be employed are polymeric substances and waxes. If desired, the active ingredient can also be in microencapsulated form with one or more of the above excipients.

Any one of the above salts or crystalline forms of the compound of formula (I) of the present invention can be formulated into liquid dosage forms for oral administration, including, but not limited to, pharmaceutically acceptable emulsions, solutions, suspensions, syrups, and tinctures. In addition to any one of the above salts or crystalline forms of the compound of formula (I) as the active ingredient, the liquid dosage forms may contain inert diluents conventionally employed in the art, such as water and other solvents, solubilizers and emulsifiers, such as ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances. In addition to these inert diluents, the liquid preparations of the present invention may also contain conventional adjuvants such as wetting agents, emulsifying agents, suspending agents, sweetening agents, flavoring agents and perfuming agents.

Such suspending agents include, for example, ethoxylated isostearyl alcohols, polyoxyethylene sorbitol and sorbitan, microcrystalline cellulose, aluminum methoxide, agar, or mixtures thereof.

Any one of the above salts or crystalline forms of the compound of formula (I) of the present invention may be formulated into dosage forms for parenteral injection, including, but not limited to, physiologically acceptable sterile aqueous or anhydrous solutions, dispersions, suspensions or emulsions, and sterile powders for reconstitution into sterile injectable solutions or dispersions. Suitable carriers, diluents, solvents or excipients include water, ethanol, polyols and suitable mixtures thereof.

Any one of the above salts or crystalline forms of the compound of formula (I) of the present invention may also be formulated into dosage forms for topical administration, including, for example, ointments, powders, suppositories, drops, sprays, and inhalants. Any one of the above salts or crystalline forms of the compound of formula (I) of the present invention as the active ingredient is mixed with a carrier acceptable under sterile and physiological conditions and optional preservatives, buffers or propellants that may be required if necessary.

The present invention also provides a pharmaceutical composition comprising any one of the above salts or crystalline forms of the compound of formula (I) of the present invention and a pharmaceutically acceptable carrier, excipient or diluent. In preparing the pharmaceutical composition, any one of the above salts or crystalline forms of the compound of formula (I) of the present invention is usually mixed with a pharmaceutically acceptable carrier, excipient or diluent.

The compositions of the present invention can be formulated into conventional pharmaceutical preparations according to conventional preparation methods. The pharmaceutical preparations are, for example, tablets, pills, capsules, powders, granules, emulsions, suspensions, dispersions, solutions, syrups, ointments, drops, suppositories, inhalants, and sprays.

Any one of the above salts or crystalline forms of the compound of formula (I) of the present invention can be administered alone or in combination with other pharmaceutically acceptable therapeutic agents, especially in combination with other antitumor drugs. Such therapeutic agents include, but are not limited to, for example, alkylating agents (e.g., cisplatin, oxaliplatin, carboplatin, cyclophosphamide, chlormethine, melphalan, chlorambucil, busulfan, temozolomide, and nitrosourea); antimetabolites (e.g., gemcitabine and antifolates such as fluoropyrimidines (e.g., 5-fluorouracil and tegafur), raltitrexed, methotrexate, cytarabine, and hydroxyurea); antitumor antibiotics (e.g., anthracyclines such as adriamycin, bleomycin, doxorubicin, daunorubicin, epirubicin, idarubicin, mitomycin C, actinomycin, and mithramycin); antimitotic agents (e.g., vinca alkaloids, such as vincristine, vinblastine, vindesine, vinorelbine; and taxanes, such as paclitaxel and taxotere); topoisomerase inhibitors (e.g., epipodophyllotoxins (e.g., etoposide and teniposide), amsacrine, topotecan, and camptothecin) and the like. The ingredients to be combined may be administered simultaneously or sequentially, in a single preparation or in separate preparations. The combination includes not only the combination of any one of the above salts or crystalline forms of the compound of formula (I) of the present invention and one other active agent, but also includes any one of the above salts or crystalline forms of the compound of formula (I) of the present invention and a combination of two or more other active agents.

The present application will be illustrated by the following examples.

## Examples

The present invention will be further described below in conjunction with the examples, and it should be understood that the examples are only used to further illustrate and elucidate the present invention, and are not intended to limit the present invention.

Unless otherwise defined, the related technical and scientific terms in this specification have the same meaning as commonly understood by a person skilled in the art. Although methods and materials similar or identical to those described herein can be used in experiments or practical applications, the materials and methods are described herein below. In case of conflict, the present specification, including definitions therein, will control, and in addition, the materials, methods and examples are not intended in a limiting sense, and are provided solely for the purpose of illustration. The present invention is further described below in conjunction with specific examples, which are not intended to limit the scope of the present invention.

The following experimental conditions were used in the examples:

| X-ray powder diffractometer (XRPD) | |
|---|---|
| Instrument | Bruker D8 Advance |
| Detector | LYNXEYE_XE_T (1D model) |
| Opening angle | 2.94° |
| Scanning mode | Continuous PSD fast mode |
| Radiation source | Cu/K-Alpha1 ($\lambda$=1.5418Å) |
| X-ray source power | 40 kV, 40 mA |
| Step size | 0.02° |
| Time per step | 0.06 seconds/step |
| Scanning range | 3° to 40° |
| Slit in primary beam path | Twin_Secondary Motorized slit 10.0 mm according to sample length; SollerMount axial Soller angle 2.5° |
| Slit in secondary beam path | Detector OpticsMount Soller slit 2.5°; Twin_Secondary Motorized slit 5.2 mm |
| Rotation speed of sample | 15 rpm |
| XRPD plate | monocrystalline silicon wafer, flat plate |
| or | |
| Instrument | Bruker D8 Advance |
| Detector | LYNXEYE XE T (1D model) |
| Opening angle | 2.94° |

(continued)

| X-ray powder diffractometer (XRPD) | |
|---|---|
| Scanning mode | Continuous PSD fast mode |
| Radiation source | Cu/K-Alpha1 ($\lambda$=1.5418Å) |
| X-ray source power | 40 kV, 40 mA |
| Step size | 0.02° |
| Time per step | 0.12 seconds/step |
| Scanning range | 3° to 40° |
| Slit in primary beam path | Twin_Secondary Motorized slit 10.0 mm according to sample length; SollerMount axial Soller angle 2.5° |
| Slit in secondary beam path | Detector OpticsMount Soller slit 2.5°; Twin_Secondary Motorized slit 5.2 mm |
| Rotation speed of sample | 15 rpm |
| XRPD plate | monocrystalline silicon wafer, flat plate |

| Differential scanning calorimetry (DSC) | |
|---|---|
| Instrument | TA Discovery 2500 or Q2000 |
| Sample pan | Tzero pan and Tzero sealing cap with pinhole |
| Temperature range | 30 to 250°C or before degradation |
| Rate of temperature rise | 10°C/min or 2°C/min |
| Flow rate of nitrogen | 50 mL/min |
| Sample amount | about 1 to 2 mg |

| Thermogravimetic analysis (TGA) | |
|---|---|
| Instrument | Discovery 5500 or Q5000 |
| Sample pan | aluminum pan, open |
| Flow rate of nitrogen | 10 mL/min for balance; 25 mL/min for sample |
| Initial temperature | Environmental condition (lower than 35°C) |
| Final temperature | 300°C or abort next segment if weight < 80%(w/w) (the weight loss of the compound is not greater than 20%(w/w)) |
| Rate of temperature rise | 10°C/min |
| Sample amount | about 2 to 10 mg |

| Polarizing microscope (PLM) | |
|---|---|
| Instrument | Nikon LV100POL |
| Method | crossed polarize, with the addition of silicone oil |

| Magnetic resonance imaging (NMR) | |
|---|---|
| Instrument | Bruker Avance-AV 400M |
| Frequency | 400 MHz |
| Probe | 5 mm PABBO BB/19F-1H/D Z-GRD Z108618/0406 |
| Number of scans | 8 |
| Temperature | 297.6K |
| Relaxation | 1 second |

| High performance liquid chromatography (HPLC) | | | |
|---|---|---|---|
| Instrument | Shimadzu LC40 | | |
| Wavelength | 210 nm | | |
| Column | Waters Xbridge C18 3.5 μm, 4.6*150 mm | | |
| Detector | DAD | | |
| Column temperature | 40°C | | |
| Flow rate | 1.0 mL/min | | |
| Mobile phase A | 20 mmol/L dipotassium hydrogen phosphate solution (adjusted with phosphoric acid to pH=8.05) | | |
| Mobile phase B | acetonitrile | | |
| Diluent | acetonitrile/water (v:v=1:1) | | |
| Injection volume | 5 μL | | |
| Gradient | Time (min) | Mobile phase A (%) | Mobile phase B (%) |
| | 0 | 70 | 30 |
| | 2 | 70 | 30 |
| | 27 | 45 | 55 |
| | 40 | 20 | 80 |
| | 45 | 20 | 80 |
| | 46 | 70 | 30 |
| | 56 | 70 | 30 |

| Ion chromatography (IC) | |
|---|---|
| Instrument | Metrohm 940 professional IC |
| Sample center | 889 IC |
| Detector | conductivity detector |
| Eluent (anion) | 3.2 mmol/L $Na_2CO_3$ + 1.0 mmol/L $NaHCO_3$ |
| Suppressor solution | 0.5%$H_2SO_4$ |
| Column | Anion A SUPP 5-150 or Cation Column C4-150 |
| Column temperature | 30°C |
| Flow rate | 0.7 mL/min (anion) |
| Injection volume | 20 μL |

The free base concentration and acid ion concentration in the same sample were determined by IC, and the base:acid stoichiometric ratio in this sample was then calculated as follows:

$$\text{free base: acid ion} = \frac{C_F}{M_F} : \frac{C_C}{M_C}$$

wherein $C_F$ is the free base concentration (mg/mL), $M_F$ is the molar mass of the free base (g/mol), Cc is the acid ion concentration (mg/mL), and $M_C$ is the molar mass of the acid ion (g/mol).

Example 1: amorphous free base (Sample No. Y11526-45-RV FWD1509-AF-SU12)

About 300 mg of the compound of formula (I) (with a purity of 99.9%) were weighed and placed in a 40 mL glass bottle, and 10 mL of methanol/acetonitrile (v:v=1:1) were added to obtain a clear solution. The clear solution was subjected to rapid volatilization (i.e., rotary evaporation) to remove the solvent to afford about 290 mg of an off-white solid in about 97% yield.

HPLC showed that the product had a purity of 99.9%. PLM showed that the product was an irregular sample (Figure 5). XRPD showed that the product was amorphous (Figure 1). DSC showed that the product had a glass-transition temperature of 68.4°C and 104.7°C (Figure 2). TGA showed that the product had a weight loss of about 5.1% at 150°C (Figure 3).

The amorphous free base (Sample No. Y11526-45-RV-FWD1509-AF-SU12) was chemically and physically stable at the end of the preparation.

Example 2: amorphous 1 eq. hydrochloride salt (Sample No. Y11526-42-SU11-methanol-dichlorom ethane)

About 300 mg of the compound of formula (I) (with a purity of 99.9%) were weighed and placed in a 40 mL glass bottle, 20 mL of methanol/dichloromethane (v:v=1:1) and 1 eq. of a diluted hydrochloric acid solution (518 μL, 44 mg/mL, in methanol/dichloromethane (v:v=1:1)) were added, and the reaction was performed at 50°C for 2 h to obtain a clear solution. The clear solution was subjected to rapid volatilization to remove the solvent to afford about 306 mg of a pale yellow solid in about 95% yield.

HPLC showed that the product had a purity of 99.9%. PLM showed that the product was an irregular sample (Figure 10). XRPD showed that the product was amorphous (Figure 6). DSC showed that the product had a glass-transition temperature of 128.5°C (Figure 7). TGA showed that the product had a weight loss of about 5.1% at 150°C (Figure 8). IC showed that the product had a free base concentration of 0.5 mg/mL and a chloride ion concentration of 33.7 mg/L, so the base:acid stoichiometric ratio in the product was about 1:1.

The amorphous 1 eq. hydrochloride salt (Sample No. Y11526-42-SU11-methanol-dichloromethane) was chemically and physically stable and reasonable in terms of the base: acid stoichiometric ratio at the end of the preparation.

Example 3: amorphous 2 eq. hydrochloride salt (Sample No. Y11526-28-SU5-methanol-dichloromethane)

About 300 mg of the compound of formula (I) (with a purity of 99.9%) were weighed and placed in a 40 mL glass bottle, 5 mL of methanol/dichloromethane (v:v=1:1) and 2 eq. of a diluted hydrochloric acid solution (1010 μL, 44 mg/mL, in methanol/dichloromethane (v:v=1:1)) were added, and the reaction was performed at 50°C for 2 h, then cooled down to 25°C and continued at this temperature for about 2 h to obtain a clear solution. The resulting clear solution was subjected to rapid volatilization to remove the solvent to afford about 310 mg of a yellow solid in about 90% yield.

HPLC showed that the product had a purity of 99.9%. PLM showed that the product was an irregular sample (Figure 15). XRPD showed that the product was amorphous (Figure 11). DSC showed that the product had a glass-transition temperature of 154.0°C (Figure 12). TGA showed that the product had a weight loss of about 4.3% at 110°C (Figure 13). IC showed that the product had a free base concentration of 0.5 mg/mL and a chloride ion concentration of 77.4 mg/L, so the base:acid stoichiometric ratio in the product was about 1:2.

The amorphous 2 eq. hydrochloride salt (Sample No. Y11526-28-SU5-methanol-dichloromethane) was chemically and physically stable and reasonable in terms of the base:acid stoichiometric ratio at the end of the preparation.

Example 4: amorphous 1 eq. mesylate salt (Sample No. Y11526-42-SU10-methanol-dichloromethane)

The weighed compound of formula (I) was added to acetone (11.70 times by weight with respect to that of the compound of formula (I) weighed) under stirring, and the temperature was increased to 45-55°C. After dissolving, the

reaction mixture was filtered while hot, the filtrate was heated to 45-55°C, water (1 time by weight with respect to that of the compound of formula (I) weighed) was added, stirring was continued at 45-55°C, methanesulfonic acid (0.188 times by weight with respect to that of the compound of formula (I) weighed) was added dropwise within 30 min, and the mixture was stirred at 45-55°C for 60±10 min. The reaction mixture was cooled down to 20-30°C within 1.0-2.0 h, and crystallization was performed at 10-30°C under stirring for 1.0-2.0 h. The reaction mixture was filtered, and the filter cake was rinsed twice with acetone (2 * 0.78 times by weight with respect to that of the compound of formula (I) weighed). The filter cake was dried to constant weight under the conditions of 40±5°C and ≤ -0.07 MPa to obtain a high-crystallinity material.

About 300 mg of the high-crystallinity material obtained were weighed, and about 50 mL of methanol/dichloromethane (v:v=1:1) were added to obtain a clear solution. The clear solution was subjected to rapid volatilization to remove the solvent to afford about 268 mg of a pale yellow solid in about 72% yield.

HPLC showed that the product had a purity of 99.9%. PLM showed that the product was an irregular sample (Figure 20). XRPD showed that the product was amorphous (Figure 16). DSC showed that the product had a glass-transition temperature of 111.1°C (Figure 17). TGA showed that the product had a weight loss of about 3.9% at 140°C (Figure 18). $^1$H-NMR showed a base:acid stoichiometric ratio of about 1:1 in the product (Figure 19).

The amorphous 1 eq. mesylate salt (Sample No. Y11526-42-SU10-methanol-dichloromethane) was chemically and physically stable and reasonable in terms of the base:acid stoichiometric ratio at the end of the preparation.

Example 5: amorphous 2 eq. mesylate salt (Sample No. Y11526-28-SU4-methanol-dichloromethane)

About 300 mg of the compound of formula (I) (with a purity of 99.9%) were weighed and placed in a 40 mL glass bottle, 2 mL of methanol and 2 eq. of a diluted methanesulfonic acid solution (798 μL, 148 mg/mL, in methanol) were added, and the clear solution became a yellow opaque system. 2 mL of dichloromethane were added, the solution became clear, and 1 mL of methanol/dichloromethane (v:v=1:1) was continued to be added. The reaction was performed at 50° C for 2 h, then cooled down to 25°C and continued at this temperature for about 3 h to obtain a clear solution. The resulting clear solution was subjected to rapid volatilization to remove the solvent to afford about 380 mg of a yellow solid in about 85% yield.

HPLC showed that the product had a purity of 99.9%. PLM showed that the product was an irregular sample (Figure 25). XRPD showed that the product was amorphous (Figure 21). DSC showed that the product had a glass-transition temperature of 131.7°C (Figure 22). TGA showed that the product had a weight loss of about 2.4% at 130°C (Figure 23). $^1$H-NMR showed a base:acid stoichiometric ratio of about 1:2 in the product (Figure 24).

The amorphous 2 eq. mesylate salt (Sample No. Y11526-28-SU4-methanol-dichloromethane) was chemically and physically stable and reasonable in terms of the base: acid stoichiometric ratio at the end of the preparation.

Example 6: amorphous 1 eq. besylate salt (Sample No. Y11526-42-SU9-methanol-dichloromethane)

About 300 mg of the compound of formula (I) (with a purity of 99.9%) were weighed and placed in a 250 mL round-bottom flask together with 1 eq. of benzenesulfonic acid, 15 mL of water were added, and the reaction was performed at 50°C for 2 h to obtain a clear solution followed by solid precipitation. The suspension was pre-frozen in a dry ice/ethanol mixture for 2 h, and then water was removed by freeze drying to afford a low-crystallinity sample. The freeze-dried sample was dissolved in 5 mL of methanol/dichloromethane (v:v=1:1) to obtain a clear solution. The clear solution was subjected to rapid volatilization to remove the solvent to afford about 320 mg of a pale yellow solid in about 83% yield.

HPLC showed that the product had a purity of 99.7%. PLM showed that the product was an irregular sample (Figure 30). XRPD showed that the product was amorphous (Figure 26). DSC showed that the product had a glass-transition temperature of 100.7°C and 114.7°C (Figure 27). TGA showed that the product had a weight loss of about 3.8% at 150°C (Figure 28). $^1$H-NMR showed a base:acid stoichiometric ratio of about 1:1 in the product (Figure 29).

The amorphous 1 eq. besylate salt (Sample No. Y11526-42-SU9-methanol-dichloromethane) was chemically and physically stable and reasonable in terms of the base: acid stoichiometric ratio at the end of the preparation.

Example 7: amorphous 2 eq. besylate salt (Sample No. Y11526-30-SU1-water)

About 300 mg of the compound of formula (I) (with a purity of 99.9%) were weighed and placed in a 250 mL round-bottom flask together with 2 eq. of benzenesulfonic acid, 15 mL of water were added, and the reaction was performed at 50°C for 2 h to obtain a clear solution. The resulting clear solution was pre-frozen in a dry ice/ethanol mixture for 2 h, and then water was removed by freeze drying to afford about 380 mg of a yellow solid in about 81% yield.

HPLC showed that the product had a purity of 99.7%. PLM showed that the product was an irregular sample (Figure 35). XRPD showed that the product was amorphous (Figure 31). DSC showed that the product had no glass-transition temperature (Figure 32). TGA showed that the product had a weight loss of about 1.9% at 100°C (Figure 33). $^1$H-NMR showed a base:acid stoichiometric ratio of about 1:2 in the product (Figure 34).

The amorphous 2 eq. besylate salt (Sample No. Y11526-30-SU1-water) was chemically and physically stable and reasonable in terms of the base:acid stoichiometric ratio at the end of the preparation.

Example 8: amorphous 1 eq. esylate salt (Sample No. Y11526-28-SU7-methanol-dichloromethane)

About 300 mg of the compound of formula (I) (with a purity of 99.9%) were weighed and placed in a 40 mL glass bottle, 5 mL of methanol/dichloromethane (v:v=1:1) and 1 eq. of a diluted ethanesulfonic acid solution (541 μL, 128 mg/mL, in methanol/dichloromethane (v:v=1:1)) were added, and the reaction was performed at 50°C for 2 h, then cooled down to 25°C and continued at this temperature for about 2 h to obtain a clear solution. The resulting clear solution was subjected to rapid volatilization to remove the solvent to afford about 340 mg of a yellow solid in about 90% yield.

HPLC showed that the product had a purity of 99.9%. PLM showed that the product was an irregular sample (Figure 40). XRPD showed that the product was amorphous (Figure 36). DSC showed that the product had a glass-transition temperature of 102.4°C (Figure 37). TGA showed that the product had a weight loss of about 1.6% at 101°C (Figure 38). $^1$H-NMR showed a base:acid stoichiometric ratio of about 1: 1 in the product (Figure 39).

The amorphous 1 eq. esylate salt (Sample No. Y11526-28-SU7-methanol-dichloromethane) was chemically and physically stable and reasonable in terms of the base:acid stoichiometric ratio at the end of the preparation.

Example 9: amorphous 1 eq. maleate salt (Sample No. Y11526-30-SU2-water)

About 300 mg of the compound of formula (I) (with a purity of 99.9%) were weighed and placed in a 250 mL round-bottom flask together with 1 eq. of maleic acid, 20 mL of water were added, and the reaction was performed at 50°C for 2 h to obtain a clear solution. The resulting clear solution was pre-frozen in a dry ice/ethanol mixture for 2 h, and then water was removed by freeze drying to afford about 330 mg of a pale yellow solid in about 89% yield.

HPLC showed that the product had a purity of 99.9%. PLM showed that the product was an irregular sample (Figure 45). XRPD showed that the product was amorphous (Figure 41). DSC showed that the product had a glass-transition temperature of 89.2°C and 125.2°C (Figure 42). TGA showed that the product had a weight loss of about 0.9% at 100°C (Figure 43). $^1$H-NMR showed a base:acid stoichiometric ratio of about 1: 1 in the product (Figure 44).

The amorphous 1 eq. maleate salt (Sample No. Y11526-30-SU2-water) was chemically and physically stable and reasonable in terms of the base:acid stoichiometric ratio at the end of the preparation.

Example 10: the crystalline 1 eq. hydrobromide salt (Sample No. Y11526-33-SU8-methanol-dichloromethane)

About 300 mg of the compound of formula (I) (with a purity of 99.9%) were weighed and placed in a 40 mL glass bottle, 20 mL of methanol/dichloromethane (v:v=1:1) and 1 eq. of a diluted hydrobromic acid solution (728 μL, 70 mg/mL, in methanol/dichloromethane (v:v=1:1)) were added, and the reaction was performed at 50°C for 2 h to obtain a nearly clear solution (trace insoluble impurities). The impurities were filtered through a 0.45 μm filter membrane to obtain a clear solution, and the resulting clear solution was subjected to rapid volatilization to remove the solvent to afford about 310 mg of a pale yellow solid in about 90% yield.

HPLC showed that the product had a purity of 99.9%. PLM showed that the product as rod-like and block-like samples (Figure 50). XRPD showed that the product had a high crystallinity (Figure 46). DSC showed that the product started to dehydrate from about 30°C (Figure 47). TGA showed that the product had a weight loss of about 1.8% at 110°C (Figure 48). KF showed that the product contained 1.9% of water. IC showed that the product had a free base concentration of 0.5 mg/mL and a bromide ion concentration of 68.1mg/L, so the base:acid stoichiometric ratio in the product was about 1:1.

The crystalline 1 eq. hydrobromide salt (Sample No. Y11526-33-SU8-methanol-dichloromethane) was chemically and physically stable and reasonable in terms of the base:acid stoichiometric ratio at the end of the preparation.

Example 11: amorphous nitrate salt (Sample No. Y11526-18-RV7-methanol-dichloromethane)

About 100 mg of the compound of formula (I) (with a purity of 99.9%) were weighed and placed in a 40 mL glass bottle, 10 mL of 1,4-dioxane were added, 2 eq. of a diluted nitric acid solution (144 μL, 180 mg/mL, in 1,4-dioxane) were added, and the reaction was performed at 50°C for 2 h. The resulting clear solution was pre-frozen in a dry ice/ethanol mixture for 2 h, and then 1,4-dioxane was removed by freeze drying to afford a nearly amorphous material.

About 80 mg of the nearly amorphous material were weighed and placed in a 40 mL glass bottle, and 10 mL of methanol/dichloromethane (v:v=1:1) were added to obtain a clear solution. The clear solution was subjected to rapid

volatilization to remove the solvent to afford the amorphous nitrate salt.

XRPD showed that the product was amorphous (Figure 51). $^1$H-NMR showed that the product was degraded (Figure 52).

The amorphous nitrate salt (Sample No. Y11526-18-RV7-methanol-dichloromethane) was at least chemically unstable at the end of the preparation.

Example 12: amorphous sulfate salt (Sample No. Y11526-15-RV3-methanol-acetonitrile)

About 100 mg of the compound of formula (I) (with a purity of 99.9%) were weighed and placed in a 40 mL glass bottle, 10 mL of 1,4-dioxane were added, 2 eq. of a diluted sulfuric acid solution (228 μL, 180.3 mg/mL, in 1,4-dioxane) were added, and the reaction was performed at 50°C for 2 h. The resulting clear solution was pre-frozen in a dry ice/ethanol mixture for 2 h, and then 1,4-dioxane was removed by freeze drying to afford a nearly amorphous material.

About 80 mg of the nearly amorphous material obtained were weighed and placed in a 40 mL glass bottle, and about 10 mL of methanol/acetonitrile (v:v=1:1) were added to obtain a clear solution. The clear solution was subjected to rapid volatilization to remove the solvent to afford the amorphous sulfate salt.

HPLC showed that the product had a purity of 99.7%. PLM showed that the product was an irregular sample (Figure 57). XRPD showed that the product was amorphous (Figure 53). DSC showed that the product had a glass-transition temperature of 103.3°C and 152.8°C (Figure 54). TGA showed that the product had a weight loss of about 3.2% at 100°C (Figure 55). IC showed that the product had a free base concentration of 0.5 mg/mL and a sulfate concentration of 162.5 mg/L, so the base:acid stoichiometric ratio in the product was about 1:1.7, which was unreasonable.

The amorphous sulfate salt (Sample No. Y11526-15-RV3-methanol-acetonitrile) was chemically stable at the end of the preparation, but was physically unstable due to a certain hygroscopicity and unreasonable in terms of the alkali: acid stoichiometric ratio.

Example 13: amorphous 2 eq. p-tosylate salt (Sample No. Y11526-23-FD11-water)

About 100 mg of the compound of formula (I) (with a purity of 99.9%) were weighed and placed in a 40 mL glass bottle together with 2 eq. of p-toluenesulfonic acid, 10 mL of 1,4-dioxane were added, and the reaction was performed at 50°C for 2 h. The resulting clear solution was pre-frozen in a dry ice/ethanol mixture for 2 h, and then 1,4-dioxane was removed by freeze drying to afford a nearly amorphous material. The resulting nearly amorphous material was fully dissolved in 10 mL of water, and the resulting clear solution was pre-frozen in a dry ice/ethanol mixture for 2 h and then water was removed by freeze drying to afford the amorphous 2 eq. p-tosylate salt.

HPLC showed that the product had a purity of 99.6%. PLM showed that the product as a block-like sample (Figure 62). XRPD showed that the product was amorphous (Figure 58). DSC showed that the product had a glass-transition temperature of 98.4°C (Figure 59). TGA showed that the product had a weight loss of about 3.8% at 140°C (Figure 60). $^1$H-NMR showed a base:acid stoichiometric ratio of about 1:2 in the product (Figure 61).

The amorphous 2 eq. p-tosylate salt (Sample No. Y11526-23-FD11-water) was chemically stable and reasonable in terms of the base:acid stoichiometric ratio at the end of the preparation, but was physically unstable due to caking and fusion to a glassy state upon short exposure to environmental conditions (Figure 63).

Example 14: amorphous sulfosalicylate salt (Sample No. Y11526-25-FD13-1,4-dioxane)

About 100 mg of the compound of formula (I) (with a purity of 99.9%) were weighed and placed in a 40 mL glass bottle together with 1 eq. of sulfosalicylic acid, 10 mL of 1,4-dioxane were added, and the reaction was performed at 50°C for 2 h. The resulting clear solution was pre-frozen in a dry ice/ethanol mixture for 2 h, and then 1,4-dioxane was removed by freeze drying to afford the amorphous sulfosalicylate salt.

HPLC showed that the product had a purity of 99.7%. PLM showed that the product was an irregular sample (Figure 68). XRPD showed that the product was amorphous (Figure 64). DSC showed that the product had a glass-transition temperature of 72.1°C (Figure 65). TGA showed that the product had a weight loss of about 1.2% at 80°C (Figure 66). $^1$H-NMR showed a base:acid stoichiometric ratio of about 1:0.8 in the product (Figure 67), which was unreasonable.

The amorphous sulfosalicylate salt (Sample No. Y11526-25-FD13-1,4-dioxane) was chemically and physically stable, but unreasonable in terms of the base: acid stoichiometric ratio at the end of the preparation.

Example 15: amorphous sulfosalicylate salt (Sample No. Y11526-25-FD12-1,4-dioxane)

About 100 mg of the compound of formula (I) (with a purity of 99.9%) were weighed and placed in a 40 mL glass bottle together with 2 eq. of sulfosalicylic acid, 10 mL of 1,4-dioxane were added, and the reaction was performed

at 50°C for 2 h. The resulting clear solution was pre-frozen in a dry ice/ethanol mixture for 2 h, and then 1,4-dioxane was removed by freeze drying to afford the amorphous sulfosalicylate salt.

HPLC showed that the product had a purity of 99.8%. PLM showed that the product was an irregular sample (Figure 73). XRPD showed that the product was amorphous (Figure 69). DSC showed that the product had a glass-transition temperature of 86.3°C (Figure 70). TGA showed that the product had a weight loss of about 2.7% at 110°C (Figure 71). $^1$H-NMR showed a base:acid stoichiometric ratio of about 1:1.5 in the product (Figure 72), which was unreasonable.

The amorphous sulfosalicylate salt (Sample No. Y11526-25-FD12-1,4-dioxane) was chemically and physically stable, but unreasonable in terms of the base: acid stoichiometric ratio at the end of the preparation.

Example 16: amorphous L-malate salt (Sample No. Y11526-17-RV6-methanol-dichloromethane)

About 100 mg of the compound of formula (I) (with a purity of 99.9%) were weighed and placed in a 40 mL glass bottle together with 1 eq. of L-malic acid, 10 mL of 1,4-dioxane were added, and the reaction was performed at 50°C for 2 h. The resulting clear solution was pre-frozen in a dry ice/ethanol mixture for 2 h, and then 1,4-dioxane was removed by freeze drying to afford a high-crystallinity material.

About 80 mg of the high-crystallinity material obtained were weighed and placed in a 40 mL glass bottle, and about 10 mL of methanol/dichloromethane (v:v=1:1) were added to obtain a clear solution. The clear solution was subjected to rapid volatilization to remove the solvent to afford the amorphous L-malate salt.

HPLC showed that the product had a purity of 91.1%, that is, the purity of the salt obtained at the end of the preparation was significantly reduced compared with the purity of the free base used to prepare the salt. PLM showed that the product was an irregular sample (Figure 78). XRPD showed that the product was amorphous (Figure 74). DSC showed that the product had a glass-transition temperature of 80.8°C (Figure 75). TGA showed that the product had a weight loss of about 3.3% at 120°C (Figure 76). $^1$H-NMR showed a base:acid stoichiometric ratio of about 1:0.6 in the product (Figure 77), which was unreasonable.

The amorphous L-malate salt (Sample No. Y11526-17-RV6-methanol-dichloromethane) was at least chemically unstable and unreasonable in terms of the base: acid stoichiometric ratio at the end of the preparation.

Example 17: amorphous 1 eq. citrate salt (Sample No. Y11526-10-FD6-1,4-dioxane)

About 100 mg of the compound of formula (I) (with a purity of 99.9%) were weighed and placed in a 40 mL glass bottle together with 1 eq. of citric acid, 10 mL of 1,4-dioxane were added, and the reaction was performed at 50°C for 2 h. The resulting clear solution was pre-frozen in a dry ice/ethanol mixture for 2 h, and then 1,4-dioxane was removed by freeze drying to afford the amorphous 1 eq. citrate salt.

HPLC showed that the product had a purity of 94.4%, that is, the purity of the salt obtained at the end of the preparation was significantly reduced compared with the purity of the free base used to prepare the salt. PLM showed that the product was an irregular sample (Figure 83). XRPD showed that the product was amorphous (Figure 79). DSC showed that the product had no glass-transition temperature (Figure 80). TGA showed that the product had a weight loss of about 9.6% at 120°C (Figure 81). $^1$H-NMR showed a base:acid stoichiometric ratio of about 1:1 in the product (Figure 82).

The amorphous 1 eq. citrate salt (Sample No. Y11526-10-FD6-1,4-dioxane) was reasonable in terms of the base:acid stoichiometric ratio, but at least chemically unstable at the end of the preparation.

Example 18: amorphous 1 eq. L-tartrate salt (Sample No. Y11526-10-FD7-1,4-dioxane)

About 100 mg of the compound of formula (I) (with a purity of 99.9%) were weighed and placed in a 40 mL glass bottle together with 1 eq. of L-tartaric acid, 10 mL of 1,4-dioxane were added, and the reaction was performed at 50°C for 2 h. The resulting clear solution was pre-frozen in a dry ice/ethanol mixture for 2 h, and then 1,4-dioxane was removed by freeze drying to afford the amorphous 1 eq. L-tartrate.

HPLC showed that the product was degraded (Figure 88). XRPD showed that the product was amorphous (Figure 84). DSC showed that the product had no glass-transition temperature (Figure 85). TGA showed that the product had a weight loss of about 8.7% at 120°C (Figure 86). $^1$H-NMR showed a base:acid stoichiometric ratio of about 1:1 in the product (Figure 87).

The amorphous 1 eq. L-tartrate salt (Sample No. Y11526-10-FD7-1,4-dioxane) was reasonable in terms of the base: acid stoichiometric ratio, but at least chemically unstable at the end of the preparation.

It can be seen from Examples 1-18 that:

when the base:acid molar charge ratio was 1:1, sulfosalicylic acid and L-malic acid cannot enable the resulting salts

to have a stoichiometric ratio of compound of formula (I):acid of 1:1;
when the base:acid molar charge ratio was 1:1, hydrochloric acid, methanesulfonic acid, benzenesulfonic acid, ethanesulfonic acid, maleic acid, hydrobromic acid, citric acid, and L-tartaric acid enable the resulting salts to have a stoichiometric ratio of compound of formula (I):acid of 1:1;
when the base:acid molar charge ratio was 1:2, nitric acid, sulfuric acid, and sulfosalicylic acid cannot enable the resulting salts to have a stoichiometric ratio of compound of formula (I):acid of 1:2; and
when the base:acid molar charge ratio was 1:2, hydrochloric acid, methanesulfonic acid, benzenesulfonic acid, and p-toluenesulfonic acid enable the resulting salts to have a stoichiometric ratio of compound of formula (I):acid of 1:2.

It can also be seen from Examples 1-18 that:

amorphous 1 eq. citrate salt, amorphous 1 eq. L-tartrate, amorphous nitrate salt and amorphous L-malate salt were chemically unstable at the end of the preparation;
amorphous 2 eq. p-tosylate salt and amorphous sulfate salt were physically unstable at the end of the preparation; and
amorphous 1 eq. hydrochloride salt, amorphous 2 eq. hydrochloride salt, amorphous 1 eq. mesylate salt, amorphous 2 eq. mesylate salt, amorphous 1 eq. besylate salt, amorphous 2 eq. besylate salt, amorphous 1 eq. esylate salt, amorphous 1 eq. maleate salt, crystalline 1 eq. hydrobromide salt, and amorphous sulfosalicylate salt were chemically and physically stable at the end of the preparation.

Example 19: solid storage stability test

Each amorphous salt solid was weighed (two 2 mg samples were weighed for purity testing after solid storage stability study, and one 10 mg sample was weighed for XRPD testing after solid storage stability study) and placed in a glass vial. In a 25°C/60%RH solid storage stability test, the amorphous salt solid was placed in a 25°C/60%RH constant temperature and humidity chamber, and placed in the dark for 1 week (i.e. "solid/25°C/60% RH/open/1 week"), and in a 60°C solid storage stability test, the amorphous salt solid was sealed in an oven at 60°C and heated for 1 week in the dark (i.e. "solid/60°C/closed container/1 week"). Then the sample was taken out for purity testing, crystal form detection and appearance observation, respectively.
Examples 1-10 were subjected to the above solid storage stability tests, and the results are shown in Table 1.

Table 1: Results of the solid storage stability test

| Examples | 1 | 2 | 3 | 4 | 5 | 6 | 7 | 8 | 9 | 10 |
|---|---|---|---|---|---|---|---|---|---|---|
| Sample | amorphous free base | amorphous 1 eq. hydrochloride salt | amorphous 2 eq. hydrochloride salt | amorphous 1 eq. mesylate salt | amorphous 2 eq. mesylate salt | amorphous 1 eq. besylate salt | amorphous 2 eq. besylate salt | amorphous 1 eq. esylate salt | amorphous 1 eq. maleate salt | crystalline 1 eq. hydrobromide salt |
| Original purity | 99.9% | 99.9% | 999% | 999% | 999% | 99.7% | 99.6% | 99.8% | 99.8% | 99.9% |
| Original morphology | amorphous | amorphous | amorphous | amorphous | amorphous | amorphous | amorphous | amorphous | amorphous | crystalline |
| | | | | | | | | | | |
| Test BS1: solid/25°C/60% RH/open/1 week | | | | | | | | | | |
| Purity | 99.6% | 999% | 99.6% | 99.8% | 99.9% | 98.2% | 99.6% | 999% | 997% | 99.9% |
| Color | no color change | no color change | slight color change | no color change | no color change | no color change | slight color change | no color change | slight color change | no color change |
| Morphology | amorphous (Figure 89), no agglomeration | amorphous (Figure 90), no agglomeration | amorphous (Figure 91), partial agglomeration (Figure 92) | amorphous (Figure 93), no agglomeration | low crystallinity (Figure 94), agglomeration (Figure 95) | amorphous (Figure 96), no agglomeration | caking and fusion to a glassy state (Figure 97) | caking and fusion to a glassy state (Figure 99) | amorphous (Figure 101), agglomeration (Figure 102) | no crystal form change (Figure 103) |
| | | | | | | | | | | |
| Test BS2: solid/60°C/closed container/1 week | | | | | | | | | | |
| Purity | 99.9% | 98.2% | 99.6% | 99.9% | 99.8% | 99.6% | 99.4% | 99.8% | 98.2% | 99.9% |
| Color | no color change | no color change | no color change | no color change | no color change | no color change | no color change | no color change | no color change | no color change |
| Morphology | medium crystallinity (Figure 89) | amorphous (Figure 90), no agglomeration | amorphous (Figure 91), no agglomeration | amorphous (Figure 93), no agglomeration | amorphous (Figure 94), partial agglomeration (Figure 95) | amorphous (Figure 96), no agglomeration | amorphous (Figure 98), no agglomeration | amorphous (Figure 100), no agglomeration | low crystallinity (Figure 101) | no crystal form change (Figure 103) |

The amorphous free base showed good physical and chemical stability under the storage stability test conditions of "solid/25°C/60%RH/open/1 week", and transformed from amorphous to a moderate crystallinity under the storage stability test conditions of "solid/60°C/closed container/1 week".

The amorphous 1 eq. hydrochloride salt showed good physical and chemical stability under the storage stability test conditions of "solid/25°C/60%RH/open/1 week", and had a decrease in purity (about 2%) under the storage stability test conditions of "solid/60°C/closed container/1 week".

The amorphous 2 eq. hydrochloride salt had slight color change and showed a certain hygroscopicity (partial agglomeration) under the storage stability test conditions of "solid/25°C/60%RH/open/1 week", and showed good physical and chemical stability under the storage stability test conditions of "solid/60°C/closed container/1 week".

The amorphous 1 eq. mesylate salt showed good physical and chemical stability under the storage stability test conditions of both "solid/25°C/60%RH/open/1 week" and "solid/60°C/closed container/1 week".

The amorphous 2 eq. mesylate salt transformed from amorphous to a low crystallinity and showed a certain hygroscopicity (agglomeration) under the storage stability test conditions of "solid/25°C/60%RH/open/1 week", and showed a certain hygroscopicity (partial agglomeration) under the storage stability test conditions of "solid/60°C/closed container/1 week".

The amorphous 1 eq. besylate salt had a decrease in purity (about 2%) under the storage stability test conditions of "solid/25°C/60%RH/open/1 week", and showed good physical and chemical stability under the storage stability test conditions of "solid/60°C/closed container/1 week".

The amorphous 2 eq. besylate salt had slight color change and showed significant hygroscopicity (caking and fusion to a glassy state) under the storage stability test conditions of "solid/25°C/60%RH/open/1 week", and showed good physical and chemical stability under the storage stability test conditions of "solid/60°C/closed container/1 week".

The amorphous 1 eq. esylate salt showed significant hygroscopicity (caking and fusion to a glassy state) under the storage stability test conditions of "solid/25°C/60%RH/open/1 week", and showed good physical and chemical stability under the storage stability test conditions of "solid/60°C/closed container/1 week".

The amorphous 1 eq. maleate salt had slight color change and showed a certain hygroscopicity (agglomeration) under the storage stability test conditions of "solid/25°C/60%RH/open/1 week", and had a decrease in purity (about 2%) and transformed from amorphous to a low crystallinity under the storage stability test conditions of "solid/ 60°C/closed container/1 week".

The crystalline 1 eq. hydrobromide salt showed good physical and chemical stability under the storage stability test conditions of both "solid/25°C/60%RH/open/1 week" and "solid/60°C/closed container/1 week".

It can be seen from Example 19 that:

The amorphous 1 eq. besylate salt, amorphous 1 eq. hydrochloride salt and amorphous 1 eq. maleate salt were chemically unstable under the storage conditions of "solid/25°C/60%RH/open/1 week" and/or "solid/60°C/closed container/1 week";

the amorphous 2 eq. hydrochloride salt, amorphous 2 eq. mesylate salt, amorphous 2 eq. besylate salt, amorphous 1 eq. esylate salt, and amorphous 1 eq. maleate salt were physically unstable under the storage conditions of "solid/25°C/60%RH/open/1 week" and/or "solid/60°C/closed container/1 week"; and

the amorphous 1 eq. mesylate salt and crystalline 1 eq. hydrobromide salt were chemically and physically stable under the storage conditions of "solid/25°C/60%RH/open/1 week" and "solid/60°C/closed container/1 week".

Example 20: solid solubility test

5 samples were weighed for each amorphous salt solid (equivalent to 20 mg of free base), and 10 mL of the following solvents were respectively added: 0.1N HCl solution (pH 1.0), 50 mM phosphate buffer (pH 4.5), FeSSIF-V1 (pH 5.0), FaSSIF-V1 (pH 6.5) and SGF (pH 2.0), and stirred at 37°C for 2 h. The suspension was then centrifuged at 37°C, the supernatant was measured for solubility by HPLC, and the solid fraction was measured for XRPD. The target solubility was at least 2 mg (based on free base)/mL.

Examples 1-10 were subjected to the above 2 h solid solubility test, and the results are shown in Table 2. Similarly, Examples 1 and 4 were subjected to a 24 h solid solubility test, and the results are shown in Table 3.

Table 2: Results of the 2 h solid solubility test

| Examples | 1 | | 2 | | 3 | | 4 | | 5 | |
|---|---|---|---|---|---|---|---|---|---|---|
| Sample | amorphous free base | | amorphous 1 eq. hydrochloride salt | | amorphous 2 eq. hydrochloride salt | | amorphous 1 eq. mesylate salt | | amorphous 2 eq. mesylate salt | |
| | Solubility measured at 2 h (pH) | XRPD measured at 2 h | Solubility measured at 2 h (pH) | XRPD measured at 2 h | Solubility measured at 2 h (pH) | XRPD measured at 2 h | Solubility measured at 2 h (pH) | XRPD measured at 2 h | Solubility measured at 2 h (pH) | XRPD measured at 2 h |
| Test ES1: 0.1N HCl solution (pH 1.0) | | | | | | | | | | |
| | > 2 mg/mL (0.8) | clear solution not performed | > 2 mg/mL (0.7) | clear solution not performed | > 2 mg/mL (0.7) | clear solution not performed | > 2 mg/mL (0.7) | clear solution not performed | > 2 mg/mL (0.7) | clear solution not performed |
| Test ES2: 50 mM phosphate buffer (pH 4.5) | | | | | | | | | | |
| | > 2 mg/mL (4.6) | clear solution not performed | > 2 mg/mL (4.5) | clear solution not performed | > 2 mg/mL (4.2) | clear solution not performed | > 2 mg/mL (4.5) | clear solution not performed | > 2 mg/mL (4.2) | clear solution not performed |
| Test ES3: FeSSIF-V1 (pH 5.0) | | | | | | | | | | |
| | > 2 mg/mL (4.9) | clear solution not performed | > 2 mg/mL (4.8) | clear solution not performed | > 2 mg/mL (4.8) | clear solution not performed | > 2 mg/mL (4.8) | clear solution not performed | > 2 mg/mL (4.8) | clear solution not performed |
| Test ES4: FaSSIF-V1 (pH 6.5) | | | | | | | | | | |
| | > 2 mg/mL (6.8) | clear solution not performed | > 2 mg/mL (6.5) | clear solution not performed | > 2 mg/mL (6.1) | clear solution not performed | > 2 mg/mL (6.5) | clear solution not performed | > 2 mg/mL (6.2) | clear solution not performed |
| Test ES5: SGF (pH 2.0) | | | | | | | | | | |
| | > 2 mg/mL (2.4) | clear solution not performed | > 2 mg/mL (2.0) | clear solution not performed | > 2 mg/mL (1.8) | clear solution not performed | > 2 mg/mL (2.0) | clear solution not performed | > 2 mg/mL (1.8) | clear solution not performed |

Table 2: Results of the 2 h solid solubility test (continued)

| Examples | 6 | | 7 | | 8 | | 9 | | 10 | |
|---|---|---|---|---|---|---|---|---|---|---|
| Sample | amorphous 1 eq. besylate salt | | amorphous 2 eq. besylate salt | | amorphous 1 eq. esylate salt | | amorphous 1 eq. maleate salt | | crystalline 1 eq. hydrobromide salt | |
| | Solubility measured at 2 h (pH) | XRPD measured at 2 h | Solubility measured at 2 h (pH) | XRPD measured at 2 h | Solubility measured at 2 h (pH) | XRPD measured at 2 h | Solubility measured at 2 h (pH) | XRPD measured at 2 h | Solubility measured at 2 h (pH) | XRPD measured at 2 h |
| Test ES1: 0.1N HCl solution (pH 1.0) | | | | | | | | | | |
| | > 2 mg/mL (0.7) | clear solution not performed | 1.8 mg/mL (0.8) | nearly clear solution a small amount of floc not performed | > 2 mg/mL (0.8) | clear solution not performed | > 2 mg/mL (0.8) | clear solution not performed | > 2 mg/mL (0.8) | clear solution not performed |
| Test ES2: 50 mM phosphate buffer (pH 4.5) | | | | | | | | | | |
| | > 2 mg/mL (4.5) | clear solution not performed | > 2 mg/mL (4.2) | clear solution not performed | > 2 mg/mL (4.4) | clear solution not performed | > 2 mg/mL (4.3) | clear solution not performed | > 2 mg/mL (4.4) | clear solution not performed |
| Test ES3: FeSSIF-V1 (pH 5.0) | | | | | | | | | | |
| | > 2 mg/mL (4.8) | clear solution not performed | > 2 mg/mL L(4.8) | clear solution not performed | > 2 mg/mL (4.8) | clear solution not performed | > 2 mg/mL (4.8) | clear solution not performed | > 2 mg/mL (4.8) | clear solution not performed |
| Test ES4: FaSSIF-V1 (pH 6.5) | | | | | | | | | | |
| | > 2 mg/mL (6.5) | clear solution not performed | > 2 mg/mL (6.1) | clear solution not performed | > 2 mg/mL (6.4) | clear solution not performed | > 2 mg/mL (6.1) | clear solution not performed | 0.7 mg/mL (6.4) | hydrobromide salt Figure 104 |
| Test ES5: SGF (pH 2.0) | | | | | | | | | | |
| | > 2 mg/mL (2.1) | clear solution not performed | > 2 mg/mL (1.7) | clear solution not performed | > 2 mg/mL (1.9) | clear solution not performed | > 2 mg/mL (2.0) | clear solution not performed | > 2 mg/mL (1.9) | clear solution not performed |

Table 3: Results of the 24 h solid solubility test

| Examples | 1 | | 4 | |
|---|---|---|---|---|
| Sample | amorphous free base | | amorphous 1 eq. mesylate salt | |
| | Solubility measured at 24 h (pH) | XRPD measured at 24 h | Solubility measured at 24 h (pH) | XRPD measured at 24 h |
| Test ES3: FeSSIF-V1 (pH 5.0) | | | | |
| | > 2 mg/mL (5.0) | clear solution not performed | > 2 mg/mL (5.0) | clear solution not performed |
| Test ES4: FaSSIF-V1 (pH 6.5) | | | | |
| | > 2 mg/mL (6.8) | clear solution not performed | > 2 mg/mL (6.6) | clear solution not performed |

In the 2 h solid solubility test, the amorphous 2 eq. besylate salt did not achieve a solubility of 2 mg/mL in the 0.1N HCl solution (pH 1.0), the crystalline 1 eq. hydrobromide salt did not achieve a solubility of 2 mg/mL in FaSSIF-V1 (pH 6.5), and the amorphous 1 eq. hydrochloride salt, amorphous 2 eq. hydrochloride salt, amorphous 1 eq. mesylate salt, amorphous 2 eq. mesylate salt, amorphous 1 eq. besylate salt, amorphous 1 eq. esylate salt, and amorphous 1 eq. maleate salt had a solubility greater than 2 mg/mL in all solvents tested. Additionally, the amorphous free base and the amorphous 1 eq. mesylate salt had a solubility greater than 2 mg/mL in FeSSIF-V1 (pH 5.0) and FaSSIF-V1 (pH 6.5) in the 24 h solid solubility test.

It can be seen from Examples 1-20 that the amorphous 1 eq. mesylate salt had a reasonable salt-forming equivalent ratio, better chemical stability, better physical stability and better solubility simultaneously.

Example 21: Synthesis of N-(2-((2-(dimethylamino)ethyl(methyl)amino)-4-methoxy-5-((4-(1-methyl-1H-indole-3-yl)-1,3,5-triazin-2-yl)amino)phenyl)acrylamide

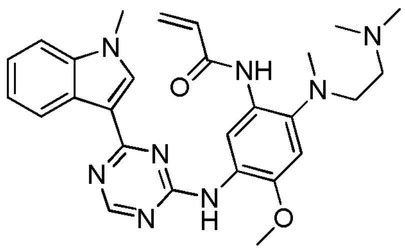

A. Synthesis of key intermediate 6:

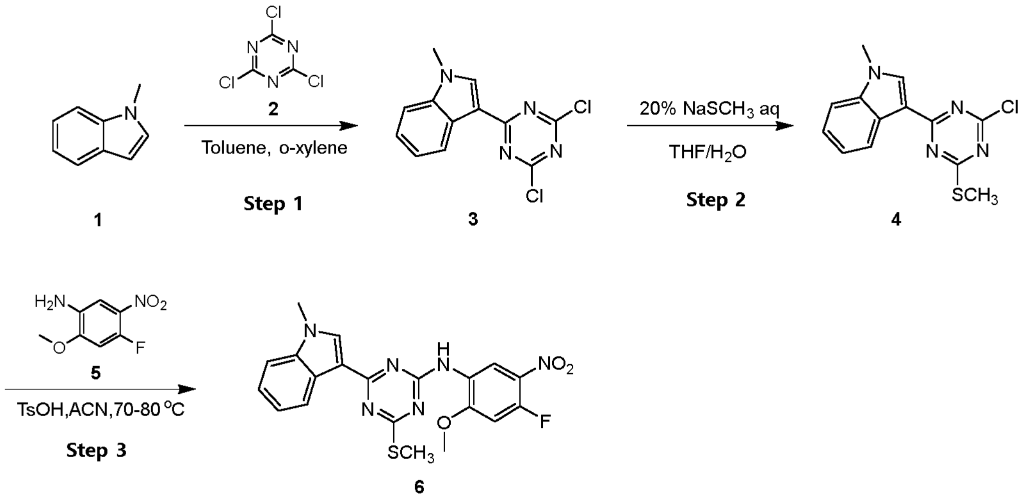


Step 1: Toluene (6V) and o-xylene (6V) were added to a reaction flask, stirred, and heated up to 60-70°C, and Starting material 1 (1.0 eq.) and Compound 2 (1.2 eq.) were added successively to the reaction flask, heated up to 110-120°C, reacted under stirring for 16-19 h, cooled down to 20-30°C and stirred for crystallization for 3 h, and filtered. The filter cake was rinsed twice with acetonitrile (1V*2), and dried to constant weight to obtain Intermediate 3 with a purity of 96% in a yield of 75%.

$^1H$ NMR ($CDCl_3$, 400 MHz): δ 3.91 (s, 3 H), 7.35 - 7.44 (m, 3 H), 8.30 (s, 1 H), 8.50 - 8.55 (m, 1 H) ppm.

Step 2: The Intermediate 3 (1.0 eq.) and tetrahydrofuran (20V) were added to the reaction flask and stirred at 0-20°C. 10% $NaSCH_3$ (1.1 eq.) was slowly added dropwise to the reaction flask with the temperature controlled at 0-20°C, and reacted under stirring for 1-2 h, and water (40V) was added to the reaction mixture, stirred with the temperature controlled at 0-20°C, and filtered. The filter cake was rinsed twice with water (1V*2), and dried to constant weight to obtain the crude product. The crude product and DMA (8V) were added to the reaction flask successively and heated up to 70-80°C for dissolved clarification. ACN (12V) was slowly added to the reaction flask with the temperature controlled at 70-80°C, stirred for 30 min, cooled down to 20-30°C, stirred for 1-2 h, and filtered. The filter cake was washed with acetonitrile (1V*2), and dried to constant weight to obtain Intermediate 4 with a purity of 98% in a yield of 68%.

$^1H$ NMR (DMSO-$d_6$, 400 MHz): δ 2.66 (s, 3 H), 3.93 (s, 3 H), 7.31-7.34 (m, 2 H), 7.60 - 7.62 (m, 1 H), 8.34 - 8.38 (m, 2 H), 8.60 (s, 1 H) ppm.

Step 3: ACN (15V) and Starting material 5 (1.1 eq.) were added to the reaction flask, and then TsOH (0.2 eq.) and Intermediate 4 (1.0 eq.) were added to the reaction flask, heated up to 70-80°C, reacted under stirring for 4-6 h, and filtered while hot. ACN (5V) was added to the filter cake, heated up to 60-70°C and stirred for 30 min, and filtered while hot, and the filter cake was washed twice with ACN (2V*2). The filter cake was dried to constant weight to obtain Intermediate 6 with a purity of 97% in a yield of 86%.

$^1H$ NMR (DMSO-$d_6$, 400 MHz): δ 2.58 (s, 1 H), 3.90 (s, 1 H), 3.99 (s, 1 H), 7.17 (s, 1 H), 7.27 (t, J=7.50 Hz, 1 H), 7.40 (d, J=13.38 Hz, 1 H), 7.54 (d, J=8.25 Hz, 1 H), 8.36 (s, 1 H), 8.86 (d, J=8.50 Hz, 1 H), 9.16 (s, 1 H) ppm.

B. Synthesis of the target compound N-(2-((2-(dimethylamino)ethyl(methyl)amino)-4-methoxy-5-((4-(1-methyl-1H-indole-3-yl)-1,3,5-triazin-2-yl)amino)phenyl)acrylamide

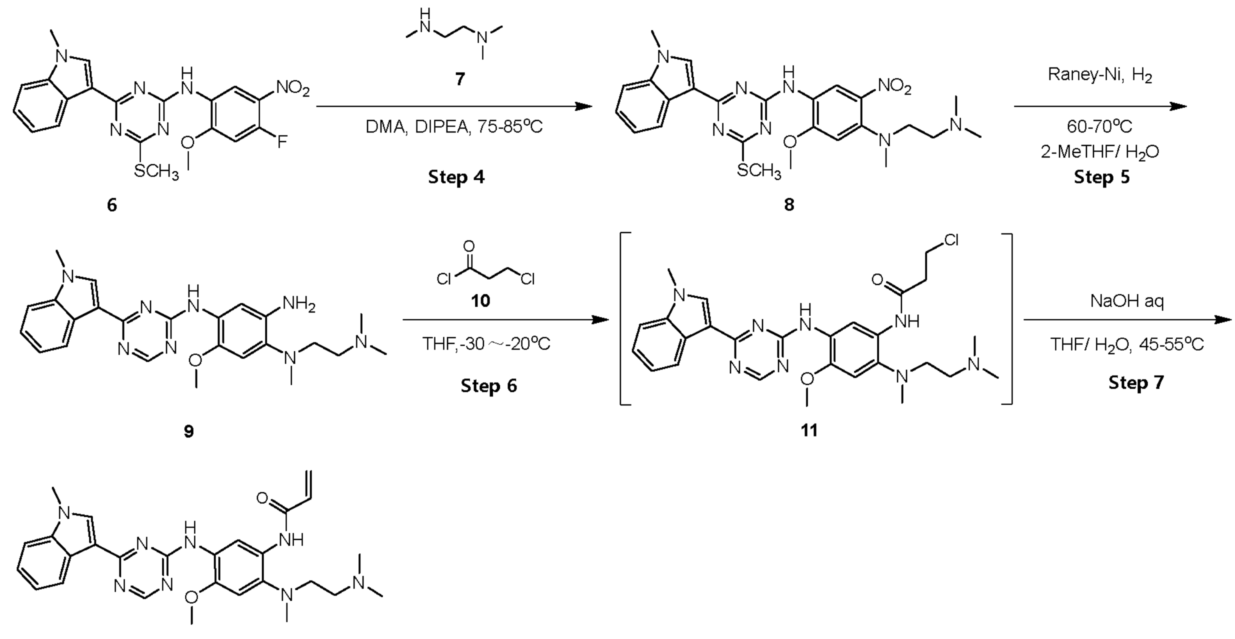


6 8 9 11

Target Compound (I)

Step 4: DMA (4.68 g) was weighed into the reaction flask, stirred and heated up to 50-60°C. DIPEA (0.44 g) and Starting material 7 (0.30 g) were added to the reaction and stirred at 50-60°C. Intermediate 6 (1 g) was weighed, added to the reaction system at 50-60°C and stirred. The reaction system was heated up to 75-85°C, the reaction mixture was stirred at 75-85°C for 4-5 h, and a sample was taken and sent to HPLC for in-process control. If the Intermediate 6 was <_ 0.5%, the reaction was stopped (if the Intermediate 6 was > 0.5%, the reaction time was prolonged, and the sample was taken every 1-2 h, until the Intermediate 6 was <_ 0.5%, and the reaction was stopped). The reaction mixture was subjected to silica gel column chromatography to obtain Intermediate 8 with a purity of 99% in a yield of 72%.

$^1$H NMR (DMSO-$d_6$, 400 MHz): δ 2.16 (s, 6 H), 2.46 - 2.49 (m, 2 H), 2.55 (s, 3 H), 2.88 (s, 3 H), 3.30 (t, 2 H), 3.88 (s, 3 H), 3.92 (s, 3 H), 6.82 (s, 1 H), 7.24 (m, 2 H), 7.52 (d, J=8.00 Hz, 1 H), 8.32- 8.40 (m, 3 H), 8.90 (s, 1 H) ppm.

Step 5: Raney-Ni (4.00 g) and water (4.50 g) were weighed into an autoclave, Intermediate 8 (1 g) was weighed, added to 2-MeTHF (12.90 g) and stirred for use. The mixture of Intermediate 8 and 2-MeTHF was poured into a 10 L autoclave, and the reaction system in the autoclave was purged with argon or nitrogen 3 times, then purged with hydrogen 3 times, with the hydrogen pressure controlled at 0.2-0.6 Mpa, and stirred. The temperature was increased to 60-70°C, the hydrogen pressure was 0.2-0.6 Mpa, and the reaction was stirred for 10-12 h. The reaction mixture was subjected to silica gel column chromatography to obtain Intermediate 9 with a purity of 98% in a yield of 63%.

$^1$H NMR (DMSO-$d_6$, 400 MHz): δ 2.18 (s, 6 H), 2.38 (t, J=6.57 Hz, 2 H), 2.65 (s, 3 H), 2.91 (t, J=6.38 Hz, 2 H), 3.35 (s, 3 H), 3.88 (s, 3 H), 4.67 (br s, 2 H), 6.77 (s, 1 H), 7.12 (s, 2 H), 7.24 (t, J=7.44 Hz, 1 H), 7.50 (d, J=8.25 Hz, 1 H), 8.32 (br s, 2 H), 8.53 (s, 1 H), 8.67 (s, 1 H) ppm.

Step 6: Tetrahydrofuran (8.90 g) was weighed, added to a glass reaction flask and stirred, and Intermediate 9 (1 g) was weighed and added to the reaction flask and stirred until dissolved. The mixture was cooled down to -30°C to -20°C, and 3-chloropropionyl chloride (0.34 g) was weighed, slowly added dropwise to the reaction flask at -30°C to -20°C, and stirred for reaction at -30°C to -10°C for 0.5-1.0 h.

Step 7: A sodium hydroxide solution (sodium hydroxide (0.45 g) was dissolved in water (2.00 g) and stirred until dissolved completely) was added to the reaction flask and heated up to 45-55°C, at which temperature the reaction was kept under stirring for 12-14 h. The reaction mixture was extracted and subjected to silica gel column chromatography to obtain the target compound of formula (I) with a purity of 99% in a yield of 72%.

$^1$H NMR ($CDCl_3$, 400 MHz):δ 10.15 (s, 1H), 9.92 (s, 1H), 9.38 (s, 1H), 8.74 (s, 1H), 8.65 (dd, J = 6.1, 2.9 Hz, 1H), 7.80 (s, 1H), 7.41 (dd, J = 6.3, 2.8 Hz, 1H), 7.37 - 7.22 (m, 2H), 6.83 (s, 1H), 6.57 - 6.46 (m, 1H), 6.41 (dd, J = 16.8, 9.8 Hz, 1H), 5.77 (dd, J = 9.8, 1.9 Hz, 1H), 4.02 (s, 3H), 3.93 (s, 3H), 2.37 (s, 2H), 2.36 (d, J = 8.7 Hz, 6H) ppm.

Example 22: Preparation of the crystalline form II-A of the mesylate salt of N-(2-((2-(dimethylamino)ethyl(methyl)amino)-4-methoxy-5-((4-(1-methyl-1H-indole-3-yl)-1,3,5-triazin-2-yl)amino)phenyl)acrylamide

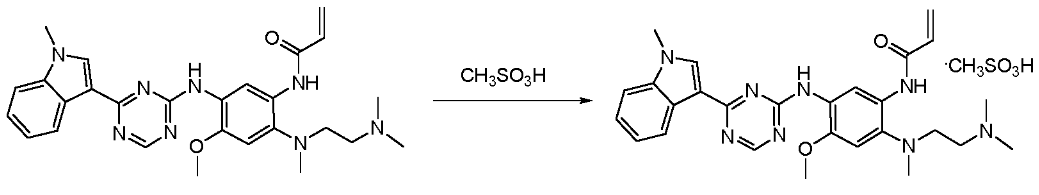


( I )

800 mg of N-(2-((2-(dimethylamino)ethyl(methyl)amino)-4-methoxy-5-((4-(1-methyl-1H-indole-3-yl)-1,3,5-triazin-2-yl)amino)phenyl)acrylamide were added to a round-bottom flask, 0.4 mL of water was added to the round-bottom flask, 152 mg of methanesulfonic acid dissolved in 5 mL of acetone were added to the round-bottom flask, and after suspending and stirring at room temperature for about 3 days, the solid was separated by centrifugation and dried under vacuum at 50°C for 1 h to obtain the target product. The yield was 85.0%.

$^1$H NMR ($CDCl_3$, 400 MHz):δ 10.15 (s, 1H), 9.92 (s, 1H), 9.38 (s, 1H), 8.74 (s, 1H), 8.65 (dd, J = 6.1, 2.9 Hz, 1H), 7.80 (s, 1H), 7.41 (dd, J = 6.3, 2.8 Hz, 1H), 7.37 - 7.22 (m, 2H), 6.83 (s, 1H), 6.57 - 6.46 (m, 1H), 6.41 (dd, J = 16.8, 9.8 Hz, 1H), 5.77 (dd, J = 9.8, 1.9 Hz, 1H), 4.02 (s, 3H), 3.93 (s, 3H), 2.30 (s, 3H), 2.37 (s, 2H), 2.36 (d, J = 8.7 Hz, 6H) ppm.

After testing, the powder X-ray diffraction of the crystalline form II-A obtained in this example had characteristic peaks at diffraction angle 2θ° of 7.22°±0.2°, 8.62°±0.2°, 9.48°±0.2°, 10.36°±0.2°, 15.12°±0.2°, 15.74°±0.2°, 16.46°±0.2°, 16.92°±0.2°, 17.70°±0.2°, 18.94°±0.2°, 19.30°±0.2°, 19.62°±0.2°, 20.36°±0.2°, 20.81°±0.2°, 22.28°±0.2°, 24.06°±0.2°, 24.78°±0.2°, 25.54°±0.2°, 25.82°±0.2°, and 26.24°±0.2°. Its X-ray powder diffraction pattern (XRPD) is shown in Figure 105.

After testing, the crystalline form II-A was obtained in this example, and had a weight loss of about 0.56% when heated to 200.0°C; and the crystalline form II-A began to show an endothermic peak when heated to 255.9°C, with its thermogravimetric analysis (TGA)/differential scanning calorimetry (DSC) profile substantially as shown in Figure 106.

Example 23: Preparation of the crystalline form III-A of the hydrochloride salt of N-(2-((2-(dimethylamino)ethyl(methyl)amino)-4-methoxy-5-((4-(1-methyl-1H-indole-3-yl)-1,3,5-triazin-2-yl)amino)phenyl)acrylamide

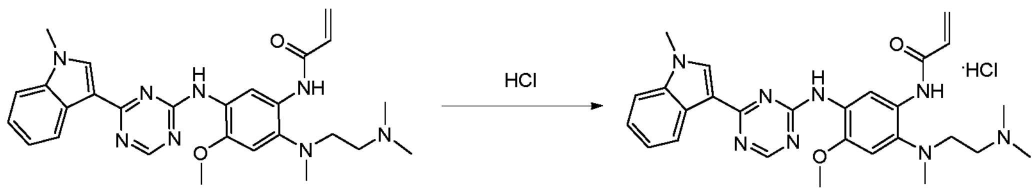


( I )

800 mg of N-(2-((2-(dimethylamino)ethyl(methyl)amino)-4-methoxy-5-((4-(1-methyl-1H-indole-3-yl)-1,3,5-triazin-2-yl)amino)phenyl)acrylamide were added to a round-bottom flask, 132 μL of concentrated hydrochloric acid (about 37%) dissolved in 5 mL of ethanol were added to the round-bottom flask, and after suspending and stirring at room temperature for about 3 days, the solid was separated by centrifugation and dried under vacuum at 50°C for 1 h to obtain the target product. The yield was 79.4%.

$^1$H NMR ($CDCl_3$, 400 MHz):δ 10.15 (s, 1H), 9.92 (s, 1H), 9.38 (s, 1H), 8.74 (s, 1H), 8.65 (dd, J = 6.1, 2.9 Hz, 1H), 7.80 (s, 1H), 7.41 (dd, J = 6.3, 2.8 Hz, 1H), 7.37 - 7.22 (m, 2H), 6.83 (s, 1H), 6.57 - 6.46 (m, 1H), 6.41 (dd, J = 16.8, 9.8 Hz, 1H), 5.77 (dd, J = 9.8, 1.9 Hz, 1H), 4.02 (s, 3H), 3.93 (s, 3H), 2.37 (s, 2H), 2.36 (d, J = 8.7 Hz, 6H) ppm.

After testing, the powder X-ray diffraction of the crystalline form III-A obtained in this example had characteristic peaks at 2θ° of 6.55°±0.2°, 10.36°±0.2°, 12.28°±0.2°, 13.10°±0.2°, 14.08°±0.2°, 14.45°±0.2°, 15.98°±0.2°, 17.33°±0.2°, 19.07°±0.2°, 19.57°±0.2°, 21.15°±0.2°, 21.72°±0.2°, 24.30°±0.2°, 24.85°±0.2°, 25.87°±0.2°, and 33.29°±0.2°. Its X-ray powder diffraction pattern (XRPD) is shown in Figure 107.

After testing, the crystalline form III-A was obtained in this example, and had a weight loss of about 1.05% when heated to 150.0°C; and the crystalline form III-A began to show an endothermic peak when heated to 193.3°C, with its thermogravimetric analysis (TGA)/differential scanning calorimetry (DSC) profile substantially as shown in Figure 108.

Example 24: Preparation of the crystalline form IV-C of the maleate salt of N-(2-((2-(dimethylamino)ethyl(methyl)amino)-4-methoxy-5-((4-(1-methyl-1H-indole-3-yl)-1,3,5-triazin-2-yl)amino)phenyl)acrylamide

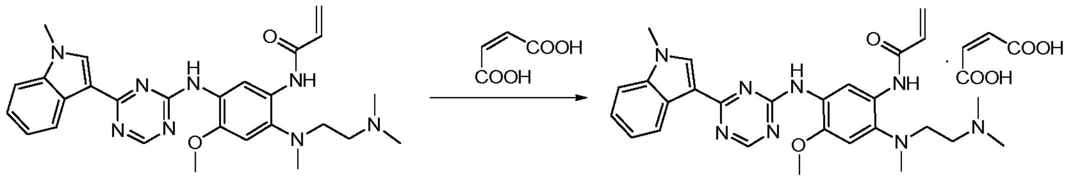

(I)

800 mg of N-(2-((2-(dimethylamino)ethyl(methyl)amino)-4-methoxy-5-((4-(1-methyl-1H-indole-3-yl)-1,3,5-triazin-2-yl)amino)phenyl)acrylamide were added to a round-bottom flask, 0.4 mL of water was added to the round-bottom flask, 184 mg of maleic acid dissolved in 5 mL of ethanol were added to the round-bottom flask, and after suspending and stirring at room temperature for about 3 days, the solid was separated by centrifugation and dried under vacuum at 50°C for 1 h to obtain the target product. The yield was 83.8%.

$^1$H NMR ($CDCl_3$, 400 MHz):δ 10.15 (s, 1H), 9.92 (s, 1H), 9.38 (s, 1H), 8.74 (s, 1H), 8.65 (dd, J = 6.1, 2.9 Hz, 1H), 7.80 (s, 1H), 7.41 (dd, J = 6.3, 2.8 Hz, 1H), 7.37 - 7.22 (m, 2H), 6.83 (s, 1H), 6.57 - 6.46 (m, 1H), 6.41 (dd, J = 16.8, 9.8 Hz, 1H), 6.02 (s, 2H), 5.77 (dd, J = 9.8, 1.9 Hz, 1H), 4.02 (s, 3H), 3.93 (s, 3H), 2.37 (s, 2H), 2.36 (d, J = 8.7 Hz, 6H) ppm.

After testing, the powder X-ray diffraction of the crystalline form IV-C obtained in this example had characteristic peaks at diffraction angle 2θ° of 6.47°±0.2°, 8.44°±0.2°, 9.15°±0.2°, 10.63°±0.2°, 12.70°±0.2°, 15.48°±0.2°, 16.42°±0.2°, 17.00°±0.2°, 18.19°±0.2°, 20.71°±0.2°, 22.43°±0.2°, 25.76°±0.2°, 28.70°±0.2°, and 31.39°±0.2°. Its X-ray powder diffraction pattern (XRPD) is shown in Figure 109.

After testing, the crystalline form IV-C was obtained in this example, and had a weight loss of about 1.96% when heated to 220.0°C; and the crystalline form IV-C began to show an endothermic peak when heated to 255.5°C, with its thermogravimetric analysis (TGA)/differential scanning calorimetry (DSC) profile substantially as shown in Figure 110.

Test Example 1: Drug absorption test in male Han-Wister rats

Intravenous administration: Three healthy male Han-Wister rats having a body weight of 250-350 g were provided by Beijing Vital River Laboratory Animal Technology Co., Ltd. The above compound of Example 1 was administered intravenously at the doses (calculated based on free base) listed in Table 1 below, respectively. Before administration and 15 min, 0.5 h, 1.0 h, 2.0 h, 4.0 h, 8.0 h, 12 h, and 24 h after administration, jugular vein puncture was performed to take 0.05 mL of blood, plasma was separated and prepared, and the drug concentration in the plasma was measured by liquid chromatography-tandem mass spectrometry (LC-MS) to obtain a drug concentration-time curve.

The main pharmacokinetic parameters are shown in Table 1 below:

Table 1

| Parameter | Example 1 |
|---|---|
| Dose (mg/kg) | 1.0 |
| $C_{max}$ (ng/mL) | 364 |
| $AUC_{0-t}$ (ng·h/mL) | 446 |
| $T_{1/2}$ (h) | 2.99 |

Intragastric administration: Twelve healthy male Han-Wister rats having a body weight of 250-350 g were provided by Beijing Vital River Laboratory Animal Technology Co., Ltd. and randomly divided into 4 groups. The above compounds of Example 1, Example 2, Example 3 and Example 4 were administered intragastrically at the doses (calculated based on free base) listed in Table 2 below, respectively. Before administration and 15 min, 0.5 h, 1.0 h, 2.0 h, 4.0 h, 8.0 h, 12 h, and 24 h after administration, jugular vein puncture was performed to take 0.05 mL of blood, plasma was separated and prepared, and the drug concentration in the plasma was measured by liquid chromatography-tandem mass spectrometry (LC-MS) to obtain a drug concentration-time curve.

The main pharmacokinetic parameters are shown in Table 2 below:

Table 2

| Parameter | Example 1 | Example 2 | Example 3 | Example 4 |
|---|---|---|---|---|
| Dose (mg/kg) | 80 | 100 | 93.2 | 81.1 |
| $C_{max}$ (ng/mL) | 583 | 1024 | 1020 | 1376 |
| $AUC_{0-t}$ (ng·h/mL) | 3968 | 13060 | 11213 | 9703 |
| $T_{1/2}$ (h) | 3.36 | 6.65 | 6.04 | 6.17 |
| F (%) | 11.1% | 29.3% | 27.0% | 26.8% |

Conclusion: The bioavailability of the crystalline form II-A of the mesylate salt of the compound of formula (I), the crystalline form III-A of the hydrochloride salt of the compound of formula (I) and the crystalline form IV-C of the maleate salt of the compound of formula (I) is significantly better than that of the compound of formula (I) of Example 1.

Test Example 2: Solubility test

The dynamic solubility of the compound of formula (I) of Example 1, the crystalline form II-A of the mesylate salt of the compound of formula (I), the crystalline form III-A of the hydrochloride salt of the compound of formula (I) and the crystalline form IV-C of the maleate salt of the compound of formula (I) in water, SGF, FaSSIF and FeSSIF (taken at the time of 0.5 h, 1 h, 2 h, 4 h, and 24 h) was investigated.

Determination method: According to a feeding concentration of 10 mg/mL, about 50 mg of the sample were weighed and added to 5 mL of the corresponding medium, and the sample was dissolved by rotating at -25 rpm at room temperature. After rotating for 0.5 h, 1 h, 2 h, 4 h, and 24 h, respectively, 1 mL of the sample was taken and centrifuged at 12,000 rpm for 2 min at room temperature, and the supernatant was used for solubility determination.

The solubility results are shown in Table 3 below, in mg/mL.

Table 3

| Solvent | Sample | 0.5 h | 1 h | 2h | 4h | 24 h |
|---|---|---|---|---|---|---|
| water | crystaline form II-A of mesylate salt | >9.2 | >9.2 | >9.4 | >9.8 | >9.1 |
| | crystaline form III-A of hydrochloride salt | 8.2 | 8.8 | 9.7 | 9.0 | 9.7 |
| | crystaline form IV-C of maleate salt | 4.0 | 4.3 | 4.4 | 4.7 | 4.4 |
| | compound of formula (I) | <1.0 | <1.0 | <1.0 | <1.0 | <1.0 |
| SGF | crystaline form II-A of mesylate salt | >10.0 | >10.0 | >10.0 | >10.0 | >10.0 |
| | crystaline form III-A of hydrochloride salt | >10.0 | >10.0 | >10.0 | >10.0 | >10.0 |
| | crystaline form IV-C of maleate salt | >10.0 | >10.0 | >10.0 | >10.0 | >10.0 |
| | compound of formula (I) | <1.0 | <1.0 | <1.0 | <1.0 | <1.0 |
| FaSSIF | crystaline form II-A of mesylate salt | 6.6 | 6.2 | 7.0 | 6.9 | 9.9 |
| | crystaline form III-A of hydrochloride salt | 5.3 | 5.2 | 5.0 | 5.0 | 8.2 |
| | crystaline form IV-C of maleate salt | 6.1 | 6.2 | 7.2 | 7.7 | >9.9 |
| | compound of formula (I) | <1.0 | <1.0 | <1.0 | <1.0 | <1.0 |
| FeSSIF | crystaline form II-A of mesylate salt | 8.1 | >9.1 | >10.0 | >10.0 | >10.0 |
| | crystaline form III-A of hydrochloride salt | >10.0 | >10.0 | >10.0 | >10.0 | >10.0 |
| | crystaline form IV-C of maleate salt | >10.0 | >10.0 | >10.0 | >10.0 | >10.0 |
| | compound of formula (I) | <1.0 | <1.0 | <1.0 | <1.0 | <1.0 |

Conclusion: The crystalline form II-A of the mesylate salt of the compound of formula (I), the crystalline form

III-A of the hydrochloride salt of the compound of formula (I) and the crystalline form IV-C of the maleate salt of the compound of formula (I) have higher dynamic solubility (> 4.0 mg/mL) in water, SGF, FaSSIF and FeSSIF, which is significantly better than the dynamic solubility of the compound of formula (I) (free base) in the corresponding medium.

Since the mesylate salt of the compound of formula (I) achieves surprising and unexpected technical effects in both salt formation and crystallization, it is more suitable for further drug development.

Although the invention has been described in detail by the detailed description and examples for clarity, these description and examples should not be construed as limiting the scope of the present invention.

## Claims

1. A crystalline form of a mesylate salt of a compound of formula (I):

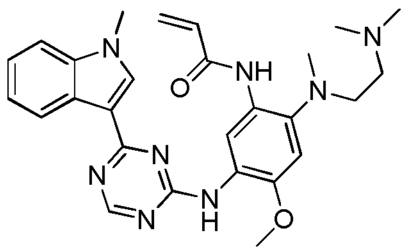

(I) .

2. The crystalline form of the mesylate salt of the compound of formula (I) according to claim 1, which is crystalline form II-A, **characterized in that** the crystalline form II-A at least has an X-ray powder diffraction pattern having characteristic peaks expressed in 2θ° of 15.12°±0.2°, 22.28°±0.2° and 25.82°±0.2°.

3. A crystalline form of the hydrochloride salt of a compound of formula (I):

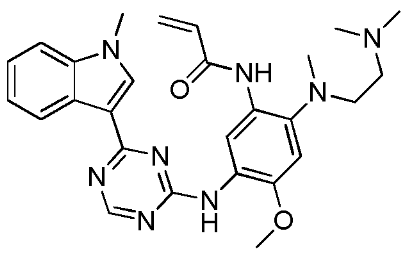

(I) .

4. The crystalline form of the hydrochloride salt of the compound of formula (I) according to claim 3, which is crystalline form III-A, **characterized in that** the crystalline form III-A at least has an X-ray powder diffraction pattern having characteristic peaks expressed in 2θ° of 24.85°±0.2°, 14.08°±0.2° and 14.45°±0.2°.

5. A crystalline form of the maleate salt of a compound of formula (I):

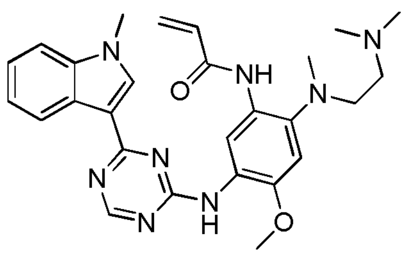

(I) .

6. The crystalline form of the maleate salt of the compound of formula (I) according to claim 5, which is crystalline form IV-C, **characterized in that** the crystalline form IV-C at least has an X-ray powder diffraction pattern having characteristic peaks expressed in 2θ° of 20.71°±0.2°, 25.76°±0.2° and 17.00°±0.2°.

7. A pharmaceutical composition comprising the crystalline form according to any one of claims 1-6 and a pharmaceutically acceptable carrier.

8. A method of treating non-small cell lung cancer (NSCLC) with EGFR exon 20 insertion mutation, comprising administering the crystalline form according to any one of claims 1-6 or the pharmaceutical composition according to claim 7 to a patient.

9. Use of the crystalline form according to any one of claims 1-6 in the preparation of a medicament for the treatment of non-small cell lung cancer (NSCLC) with EGFR exon 20 insertion mutation.

10. A method for preparing a compound of formula (I), comprising the following steps:

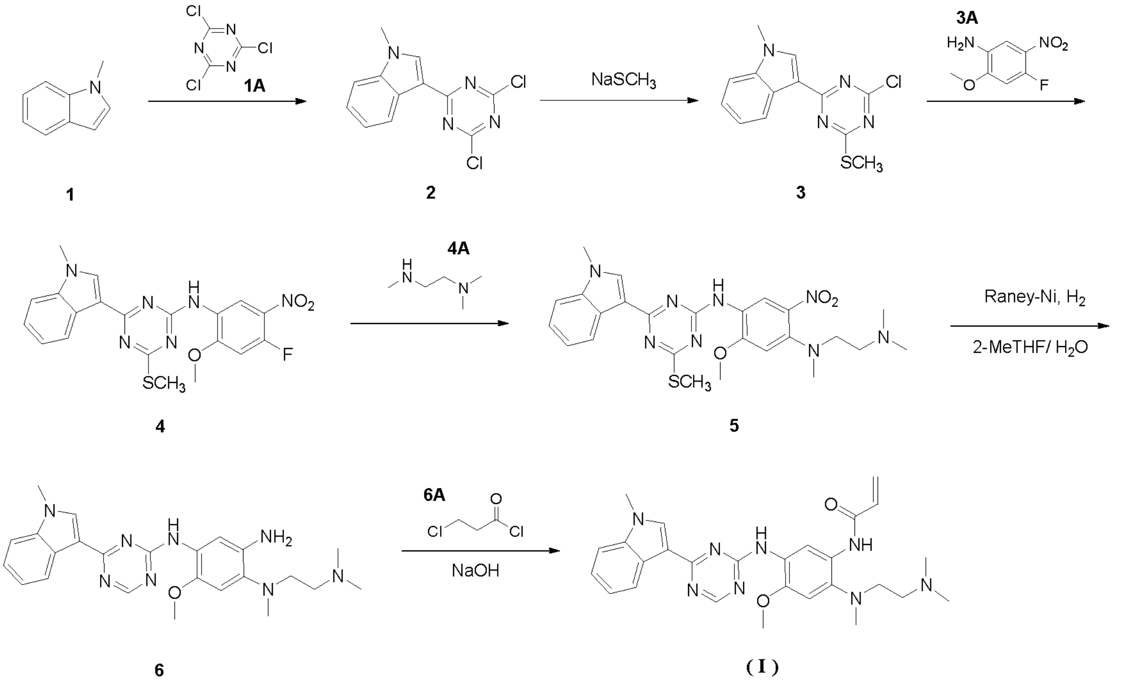

1 2 3
4 5
6 (I)

.

XRPD pattern of the amorphous free base (Sample No. Y11526-45-RV-FWD1509-AF-SU12)

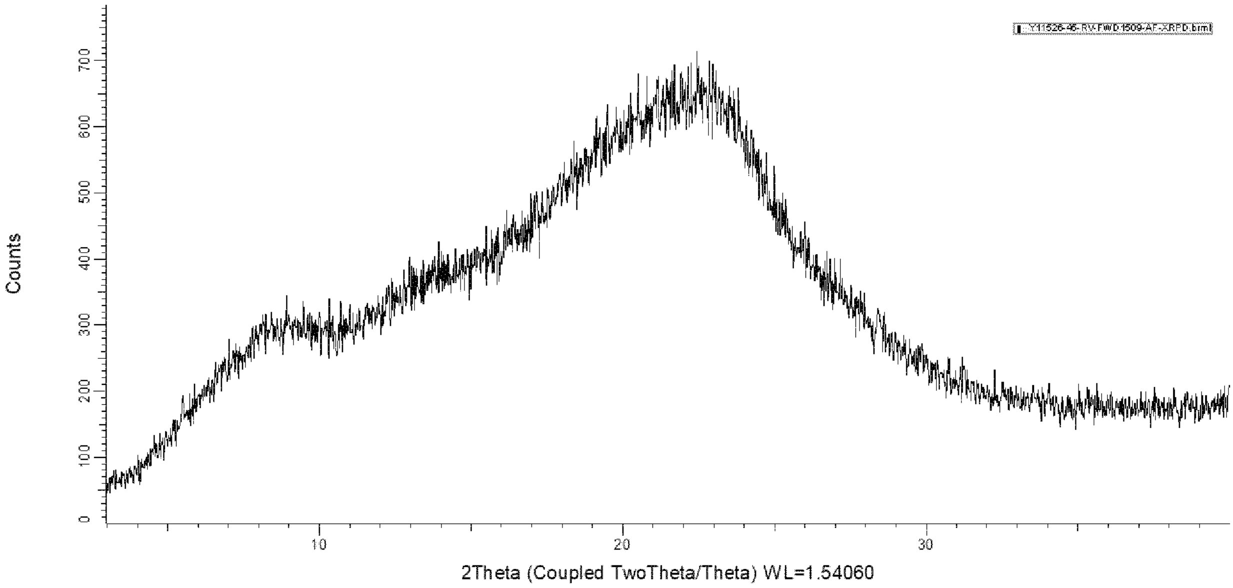


Figure 1

DSC profile of the amorphous free base (Sample No. Y11526-45-RV-FWD1509-AF-SU12)

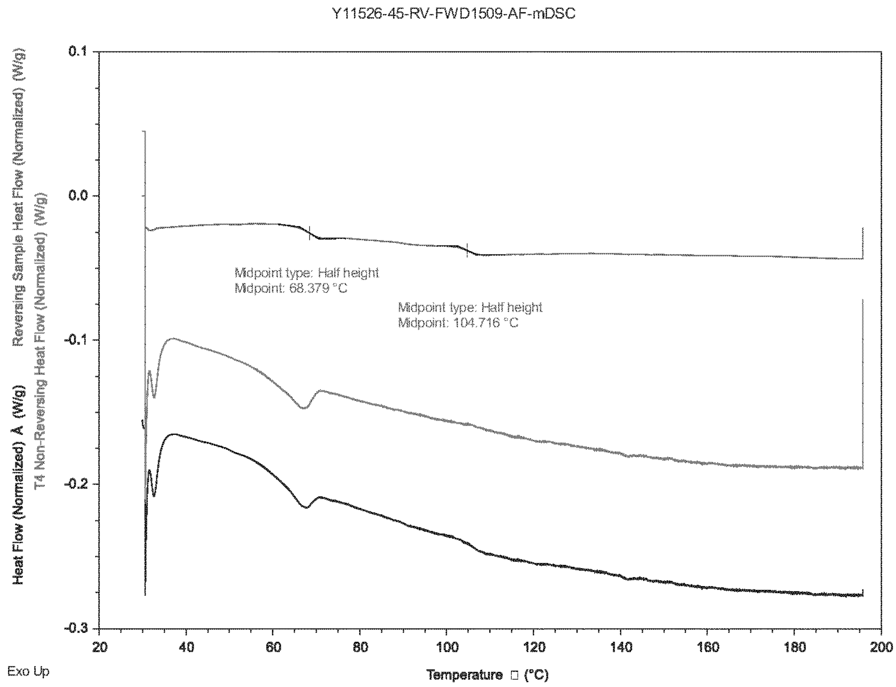


Figure 2

TGA profile of the amorphous free base (Sample No. Y11526-45-RV-FWD1509-AF-SU12)

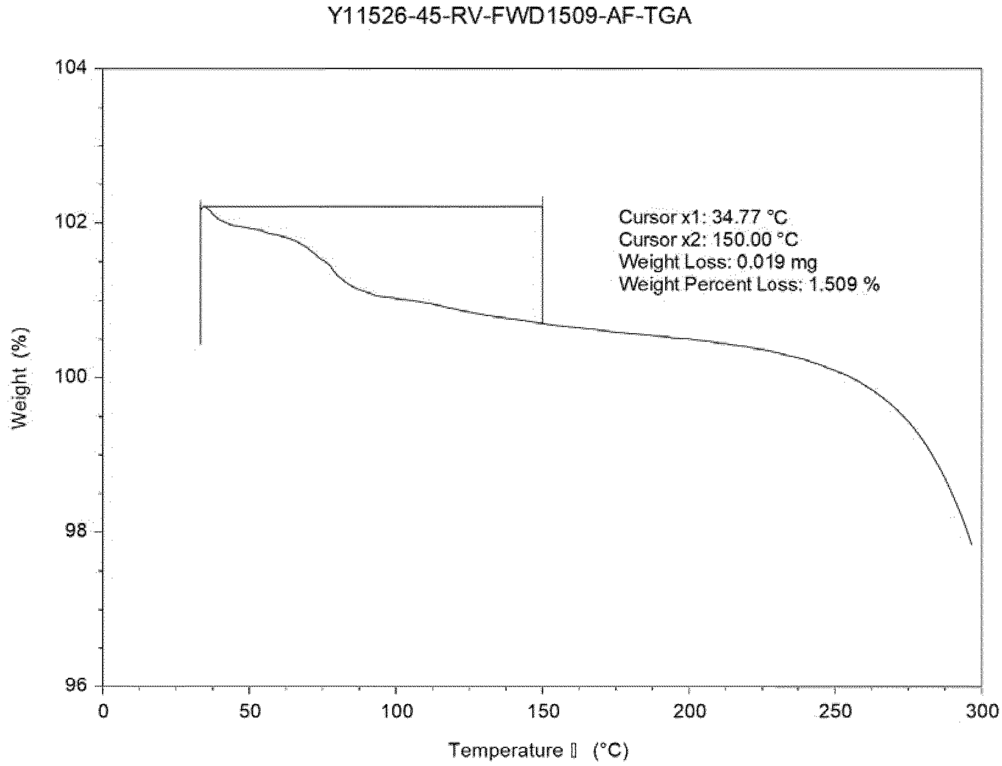


Figure 3

$^1$H-NMR spectrum of the amorphous free base (Sample No. Y11526-45-RV-FWD1509-AF-SU12)

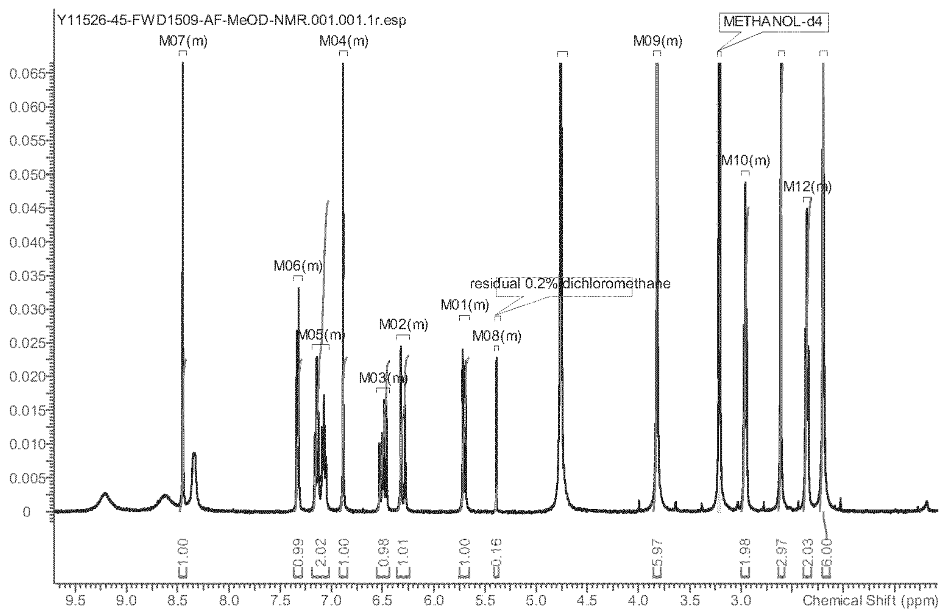


Figure 4

PLM photograph of the amorphous free base (Sample No. Y11526-45-RV-FWD1509-AF-SU12)

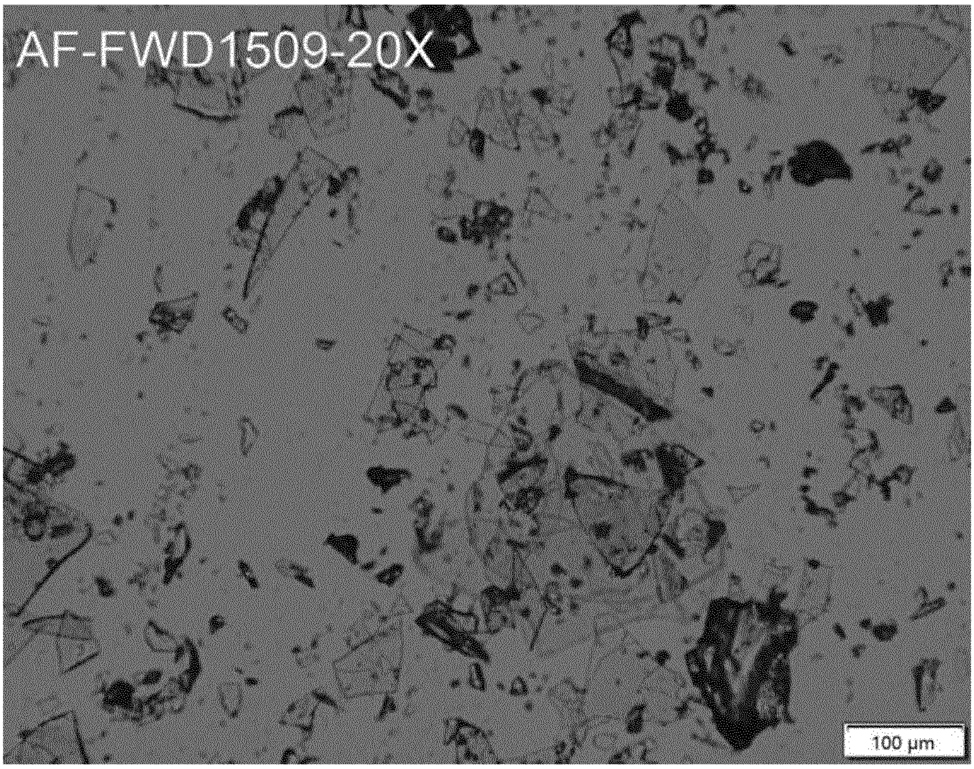


Figure 5

XRPD pattern of the amorphous 1 eq. hydrochloride salt (Sample No. Y11526-42-SU11-methanol-dichloromethane)

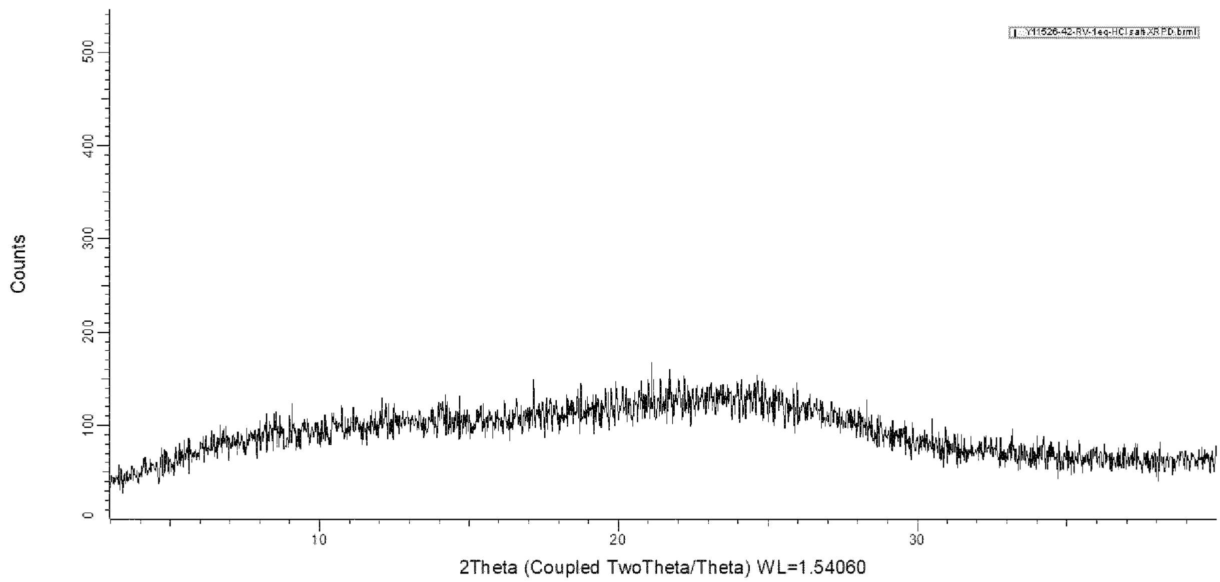


Figure 6

DSC profile of the amorphous 1 eq. hydrochloride salt (Sample No. Y11526-42-SU11-methanol-dichloromethane)

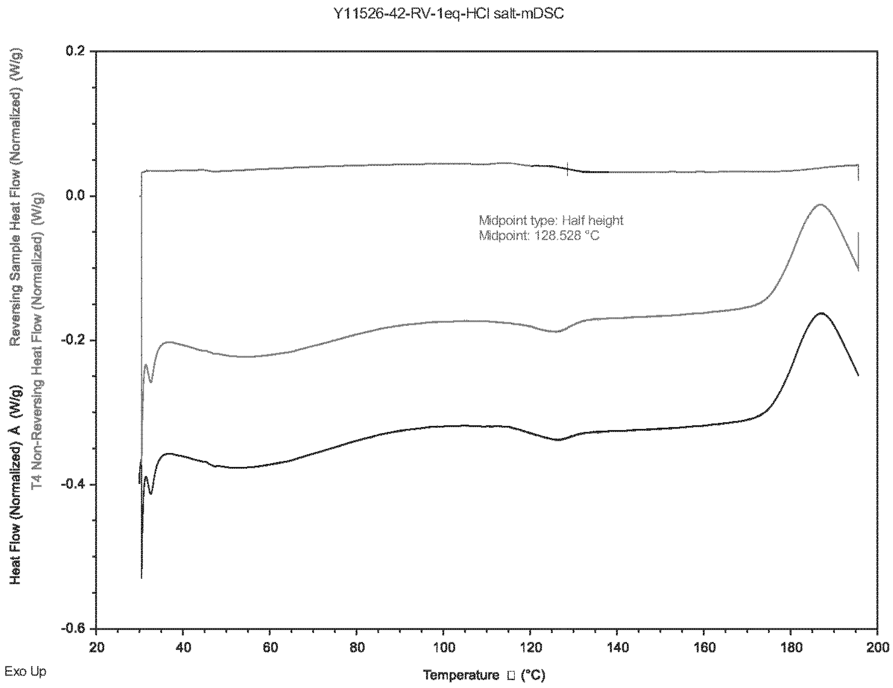


Figure 7

TGA profile of the amorphous 1 eq. hydrochloride salt (Sample No. Y11526-42-SU11-methanol-dichloromethane)

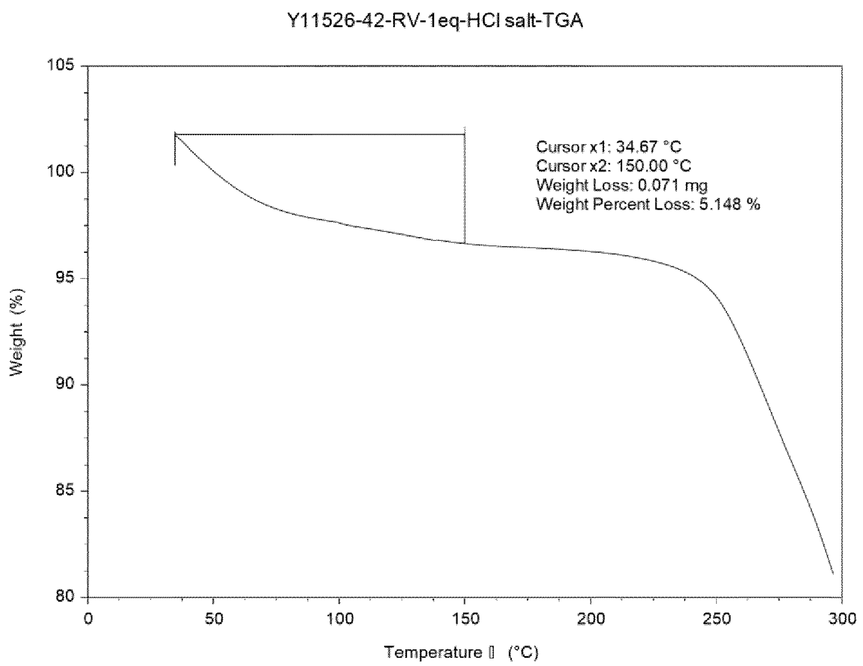


Figure 8

$^1$H-NMR spectrum of the amorphous 1 eq. hydrochloride salt (Sample No. Y11526-42-SU11-methanol-dichloromethane)

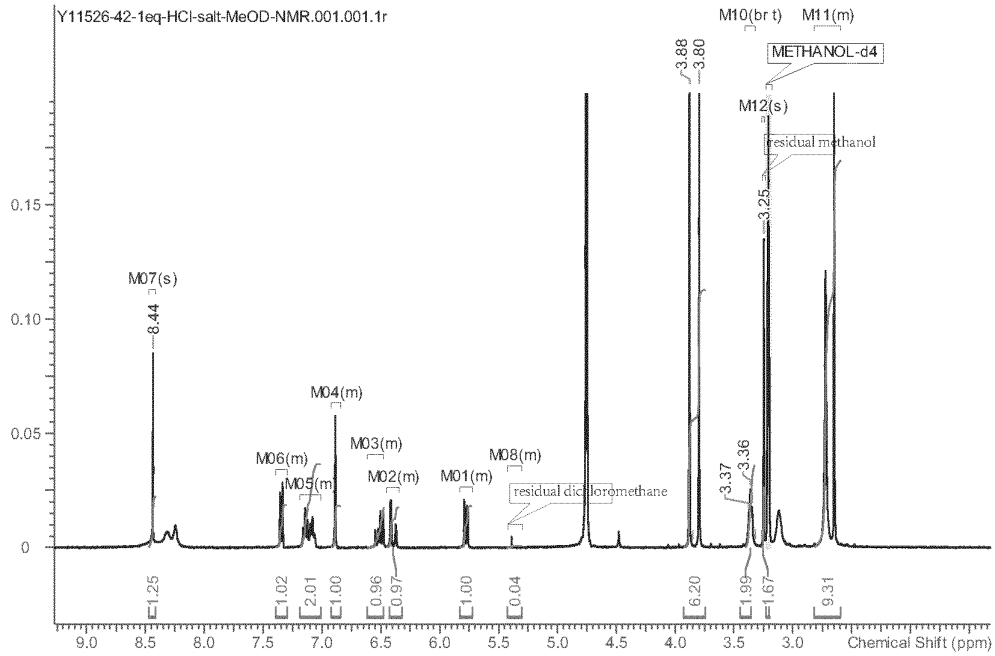


Figure 9

PLM photograph of the amorphous 1 eq. hydrochloride salt (Sample No. Y11526-42-SU11-methanol-dichloromethane)

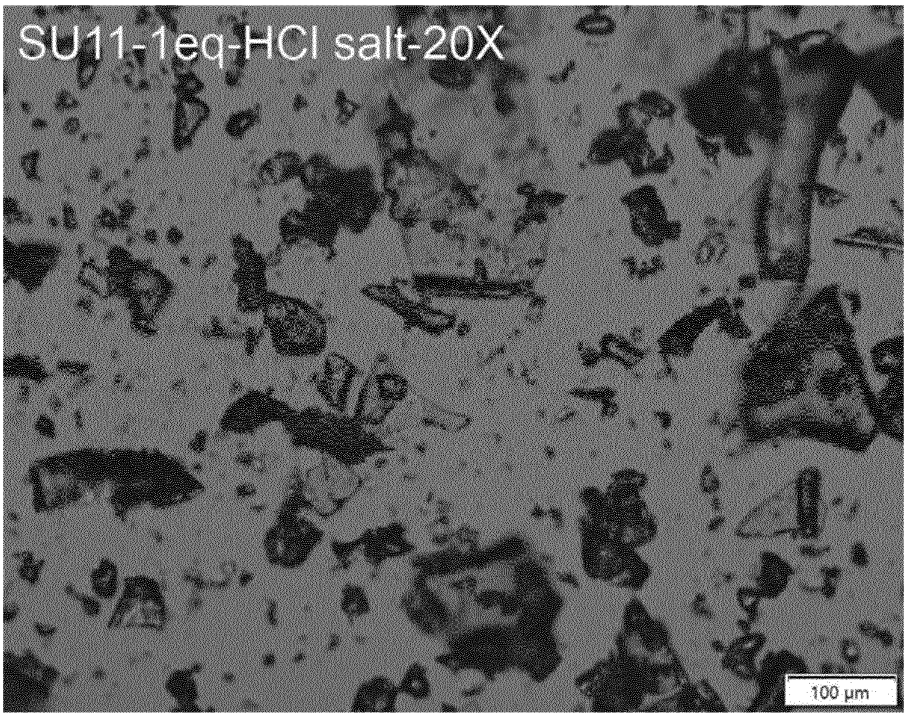


Figure 10

XRPD pattern of the amorphous 2 eq. hydrochloride salt (Sample No. Y11526-28-SU5-methanol-dichloromethane)

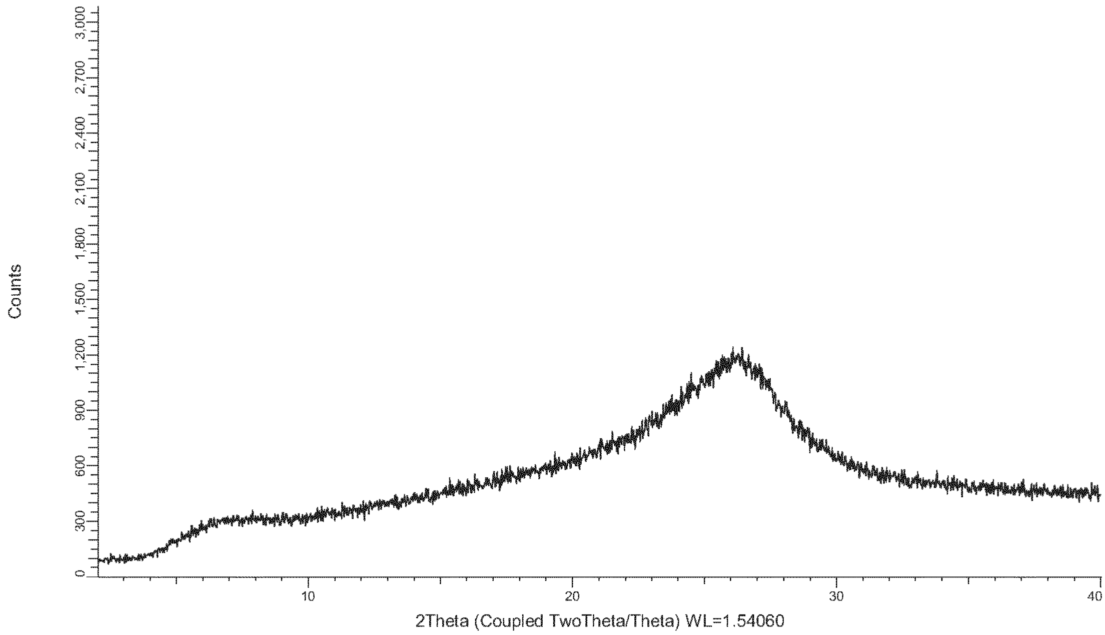


Figure 11

DSC profile of the amorphous 2 eq. hydrochloride salt (Sample No. Y11526-28-SU5-methanol-dichloromethane)

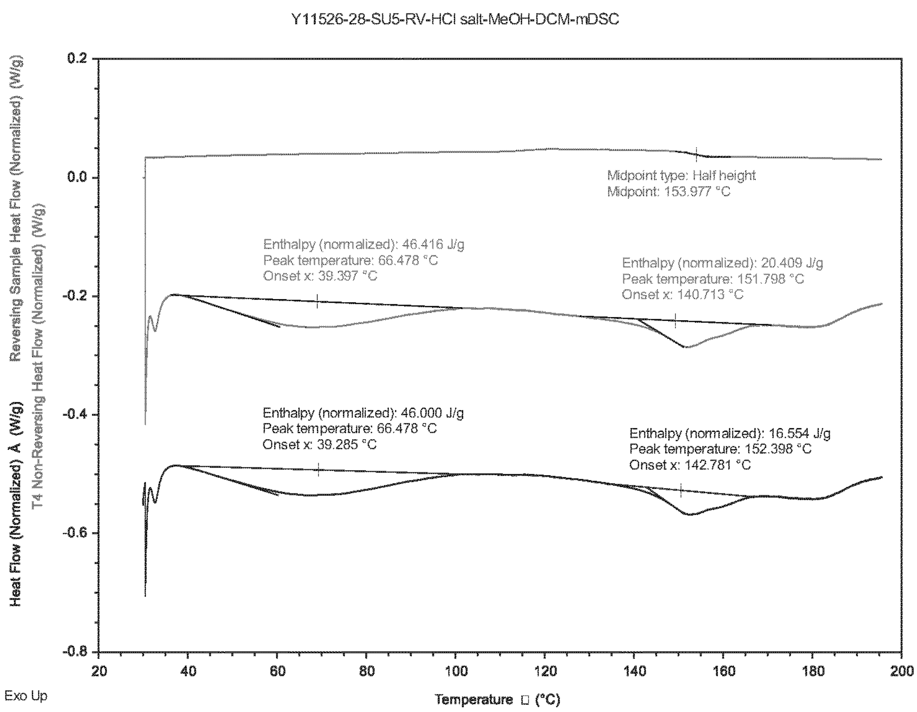


Figure 12

TGA profile of the amorphous 2 eq. hydrochloride salt (Sample No. Y11526-28-SU5-methanol-dichloromethane)

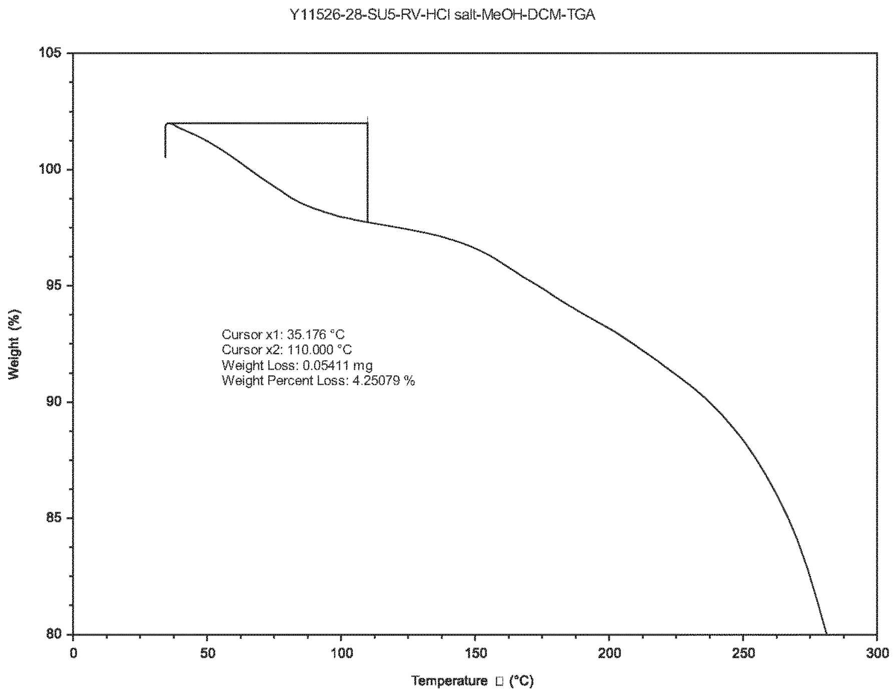


Figure 13

$^1$H-NMR spectrum of the amorphous 2 eq. hydrochloride salt (Sample No. Y11526-28-SU5-methanol-dichloromethane)

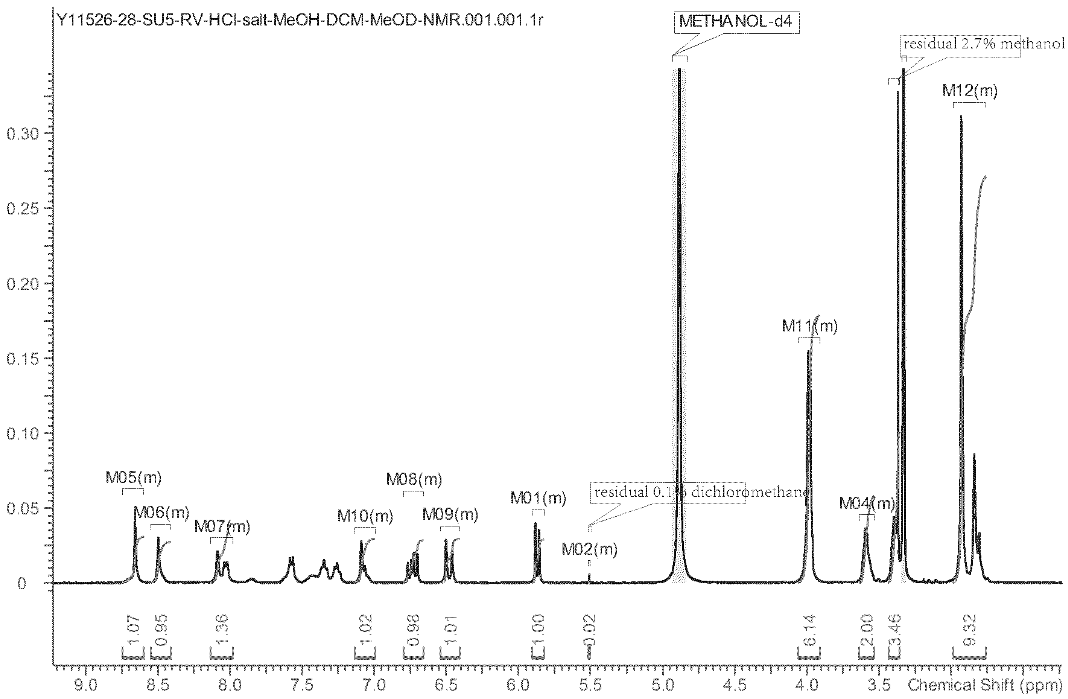


Figure 14

PLM photograph of the amorphous 2 eq. hydrochloride salt (Sample No. Y11526-28-SU5-methanol-dichloromethane)

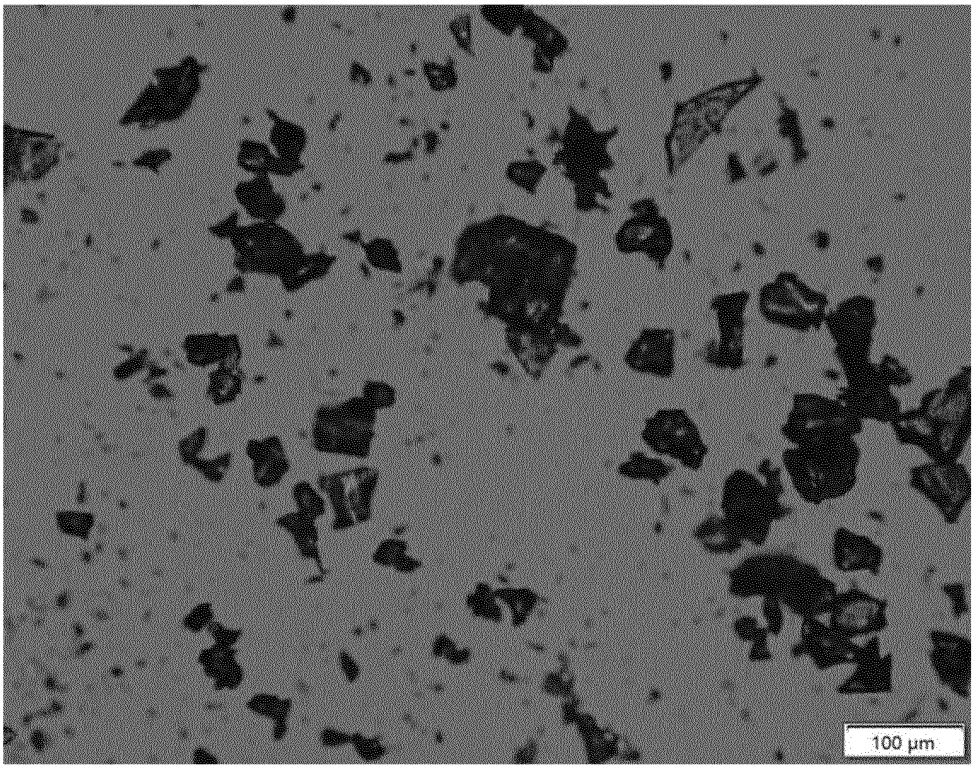


Figure 15

XRPD pattern of the amorphous 1 eq. mesylate salt (Sample No. Y11526-42-SU10-methanol-dichloromethane)

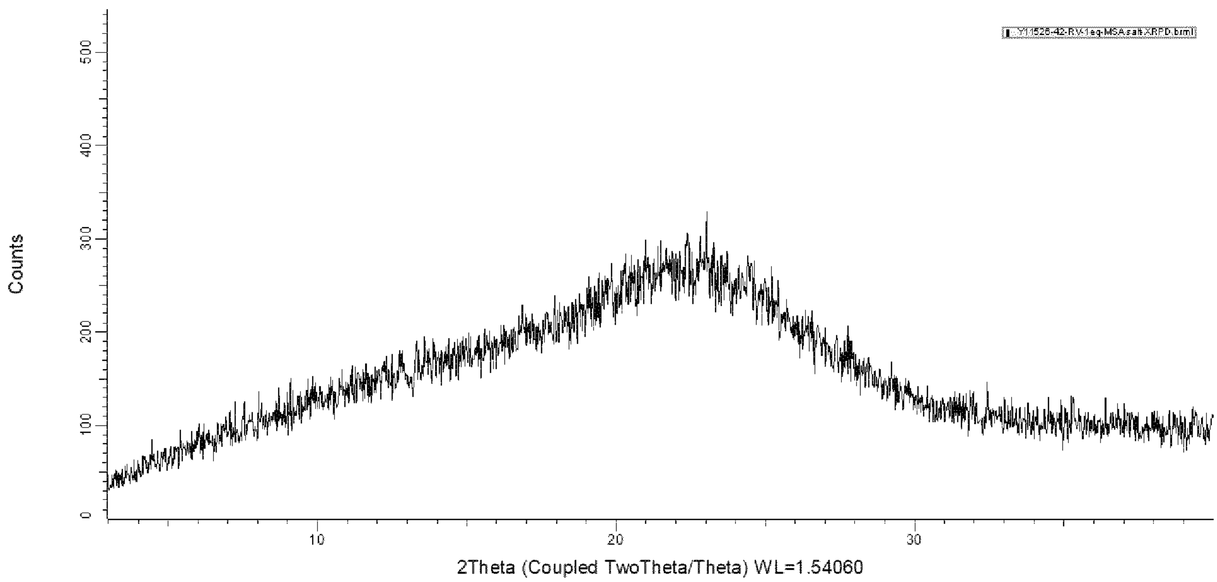


Figure 16

DSC profile of the amorphous 1 eq. mesylate salt (Sample No. Y11526-42-SU10-methanol-dichloromethane)

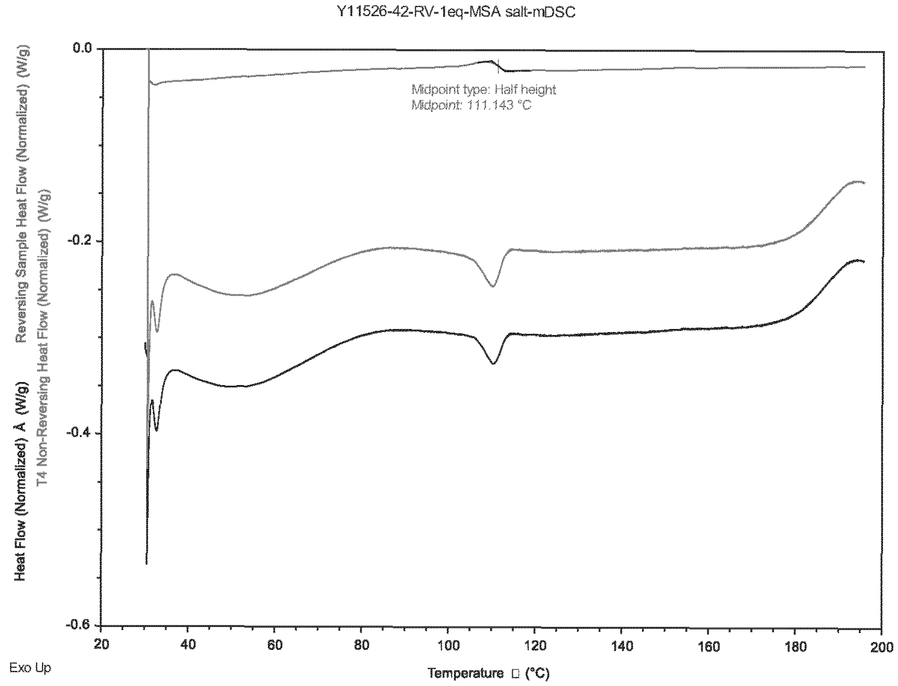


Figure 17

TGA profile of the amorphous 1 eq. mesylate salt (Sample No. Y11526-42-SU10-methanol-dichloromethane)

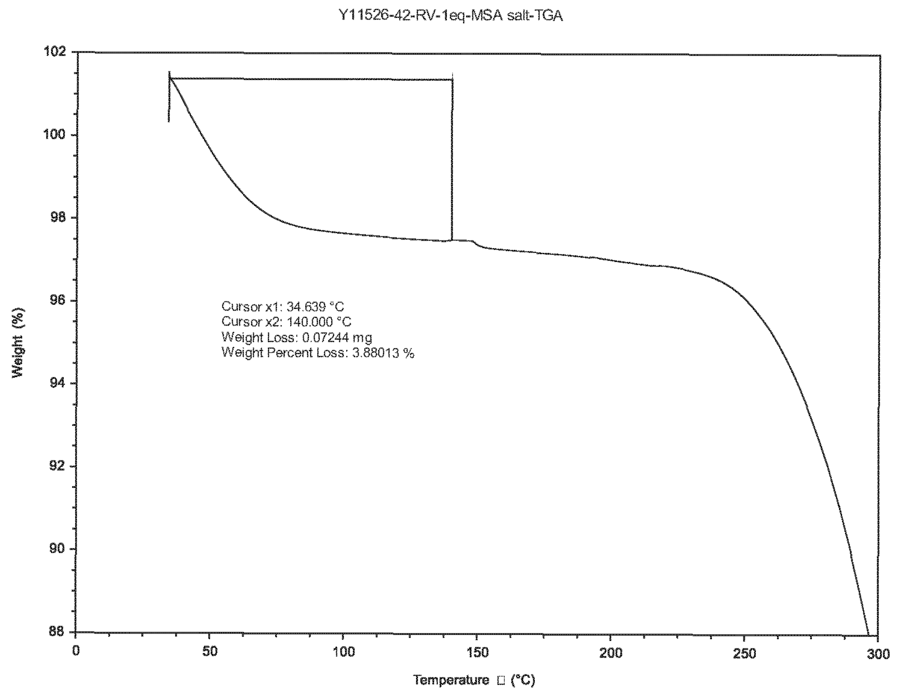


Figure 18

$^1$H-NMR spectrum of the amorphous 1 eq. mesylate salt (Sample No. Y11526-42-SU10-methanol-dichloromethane)

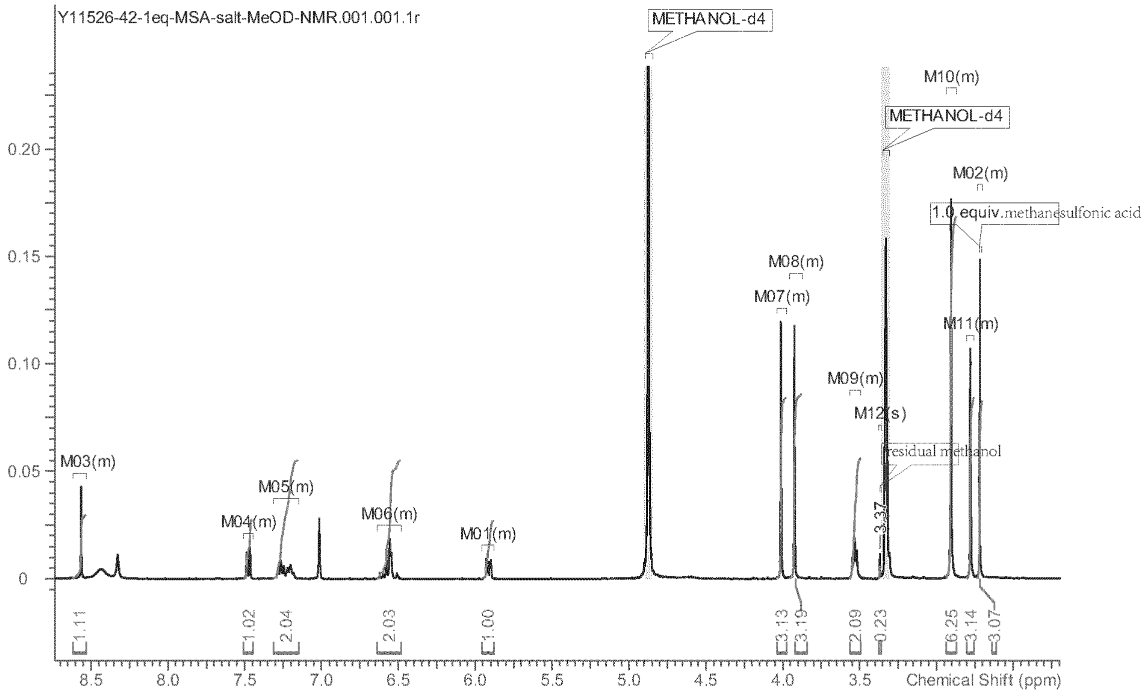


Figure 19

PLM photograph of the amorphous 1 eq. mesylate salt (Sample No. Y11526-42-SU10-methanol-dichloromethane)

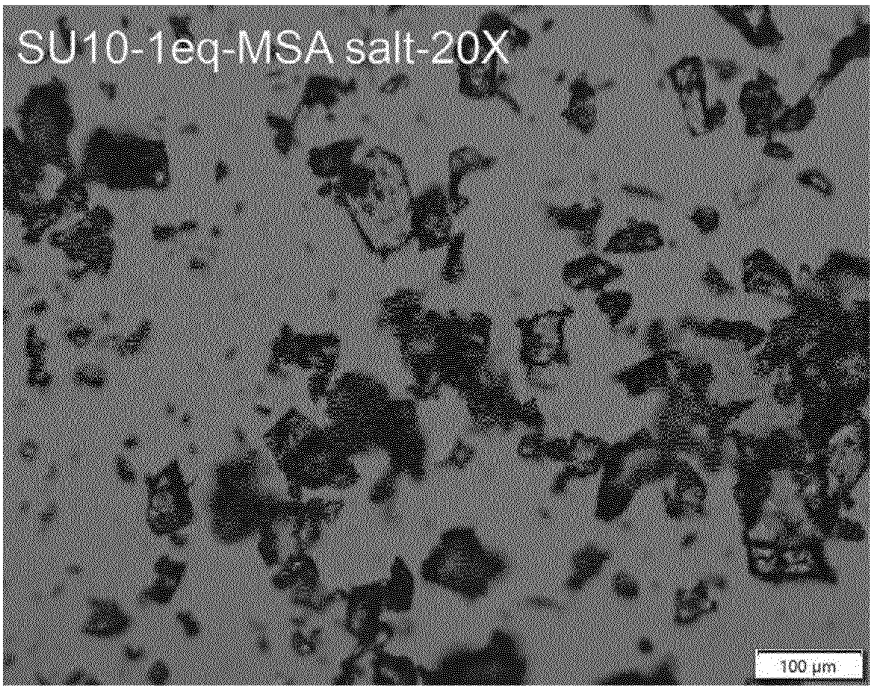


Figure 20

XRPD pattern of the amorphous 2 eq. mesylate salt (Sample No. Y11526-28-SU4-methanol-dichloromethane)

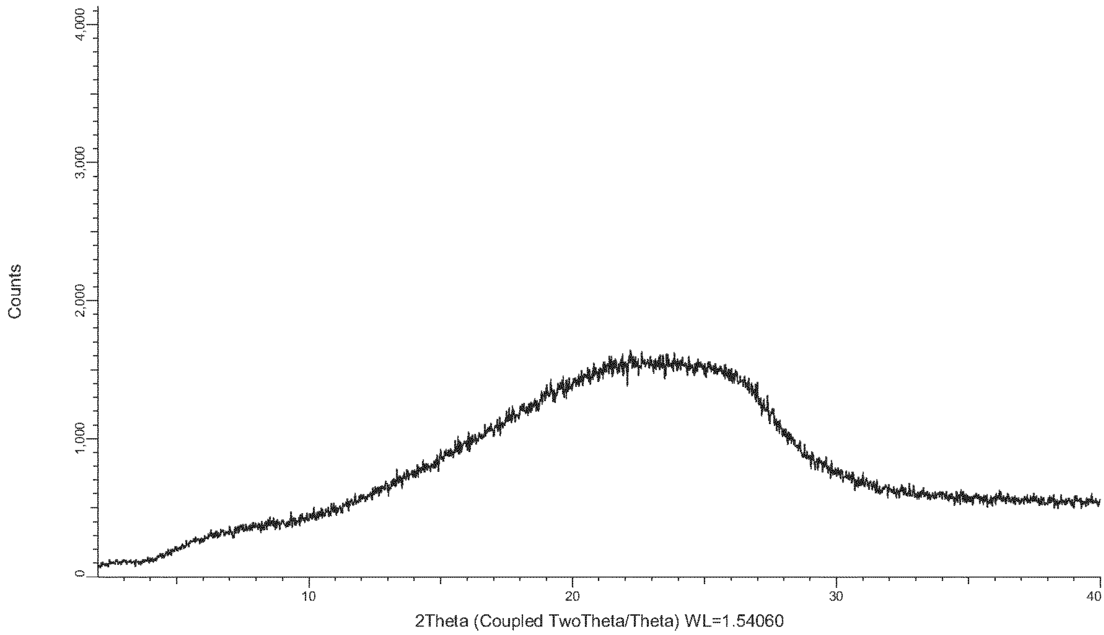


Figure 21

DSC profile of the amorphous 2 eq. mesylate salt (Sample No. Y11526-28-SU4-methanol-dichloromethane)

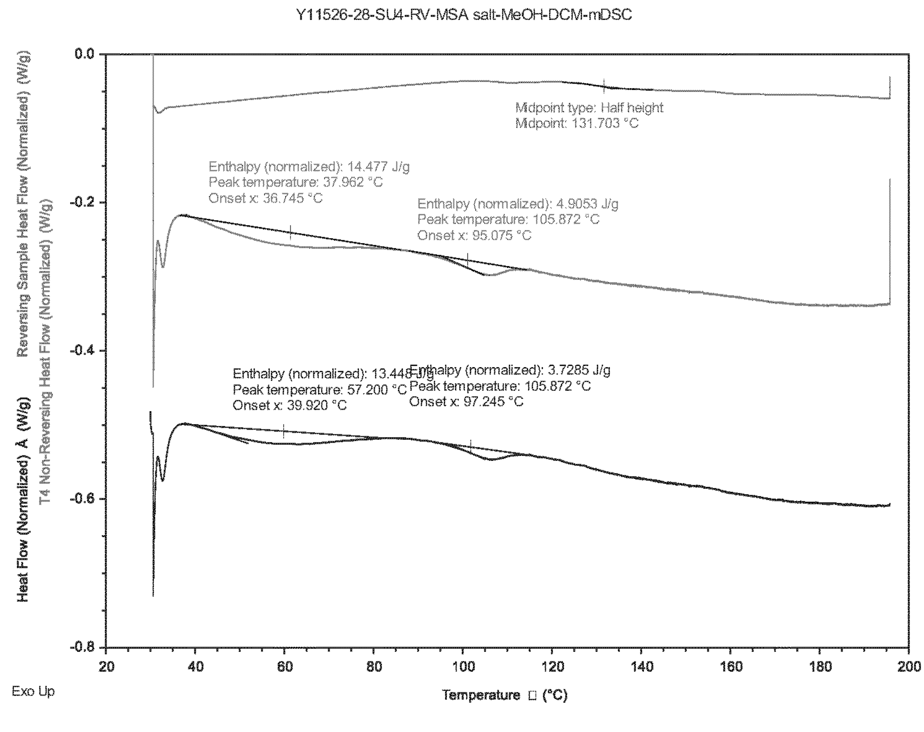


Figure 22

TGA profile of the amorphous 2 eq. mesylate salt (Sample No. Y11526-28-SU4-methanol-dichloromethane)

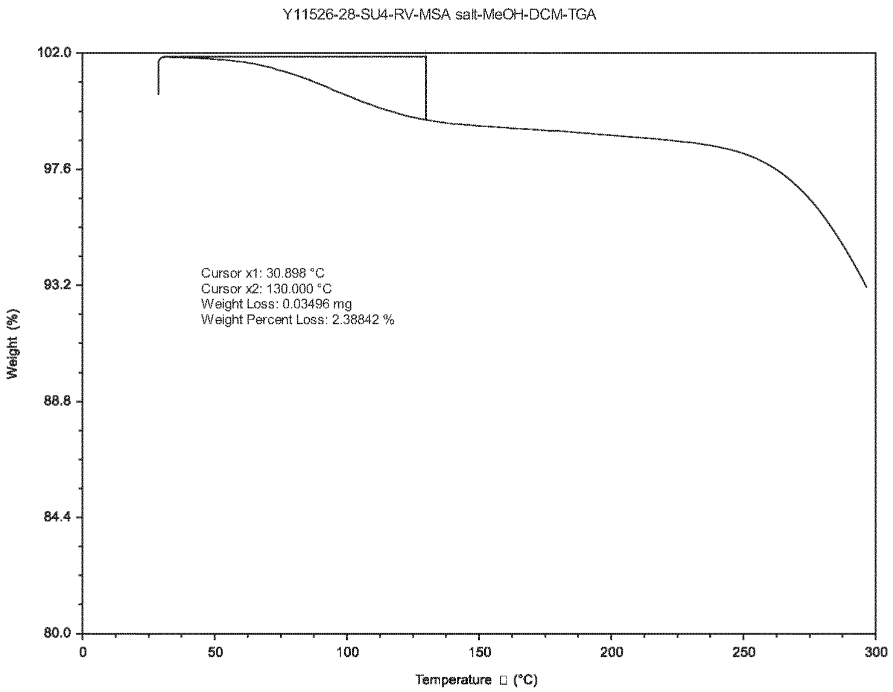


Figure 23

$^1$H-NMR spectrum of the amorphous 2 eq. mesylate salt (Sample No. Y11526-28-SU4-methanol-dichloromethane)

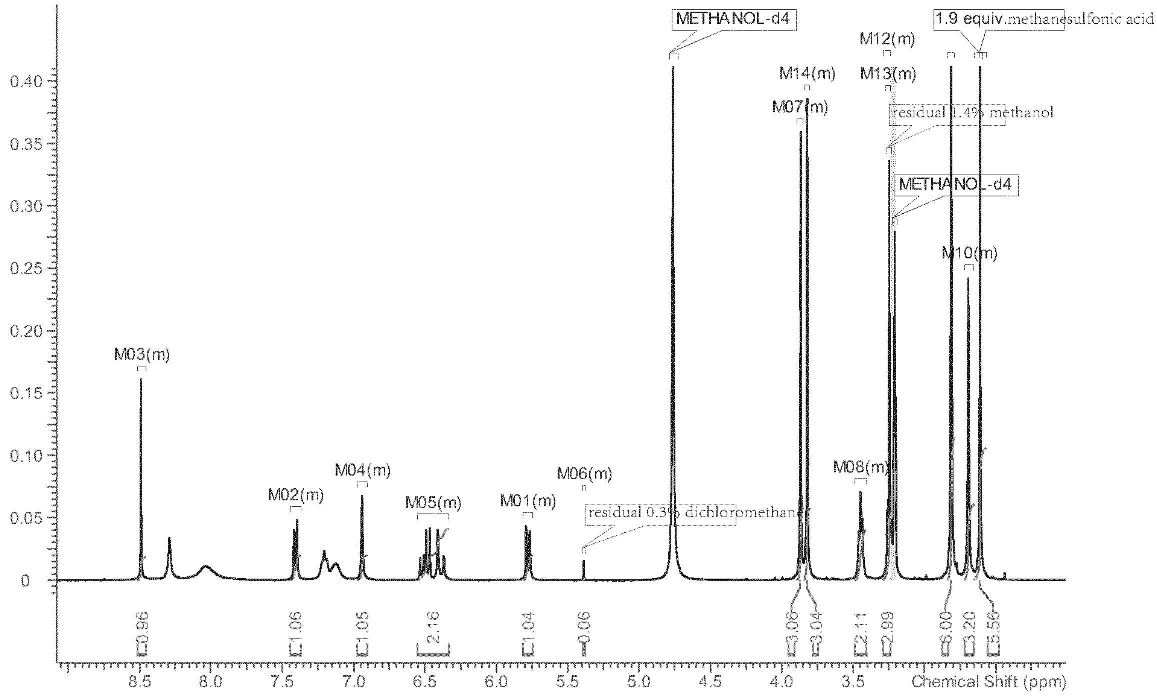


Figure 24

PLM photograph of the amorphous 2 eq. mesylate salt (Sample No. Y11526-28-SU4-methanol-dichloromethane)

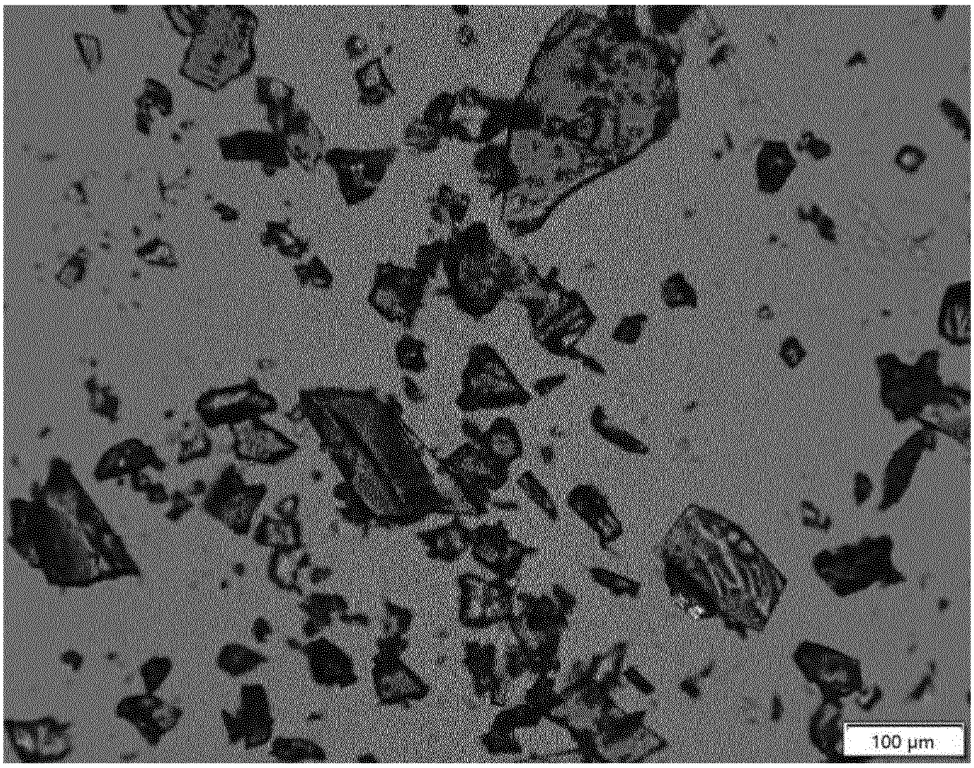


Figure 25

XRPD pattern of the amorphous 1 eq. besylate salt (Sample No. Y11526-42-SU9-methanol-dichloromethane)

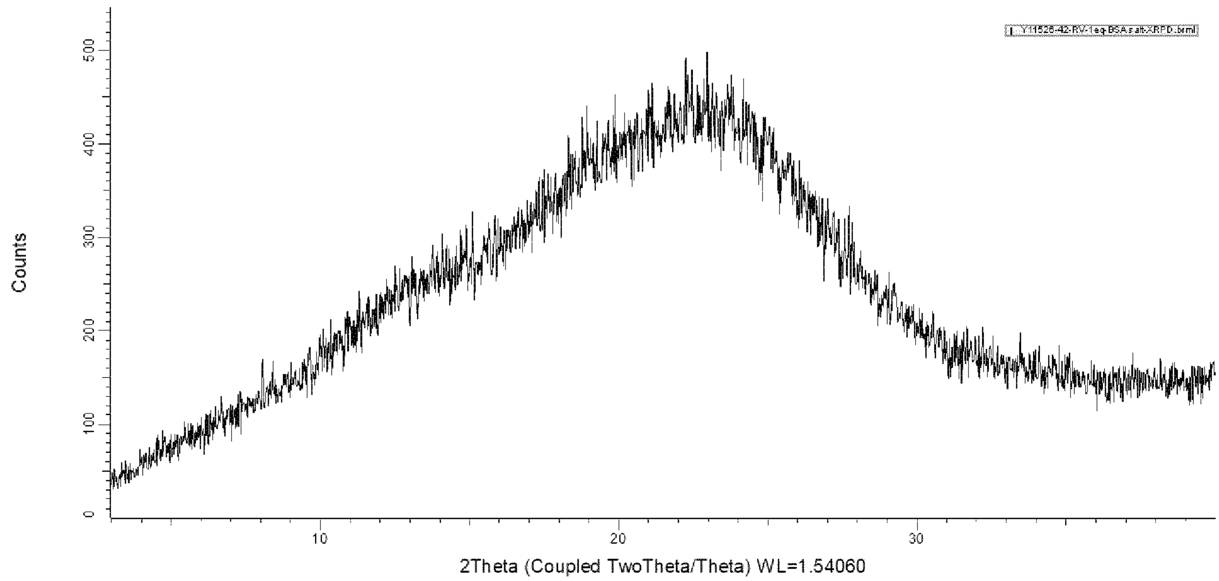


Figure 26

DSC profile of the amorphous 1 eq. besylate salt (Sample No. Y11526-42-SU9-methanol-dichloromethane)

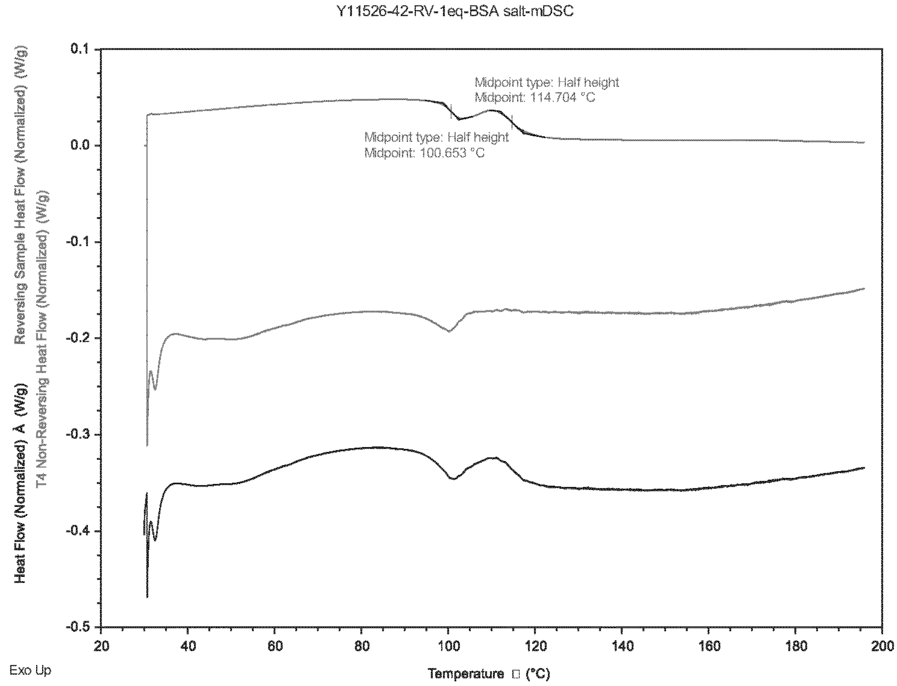


Figure 27

TGA profile of the amorphous 1 eq. besylate salt (Sample No. Y11526-42-SU9-methanol-dichloromethane)

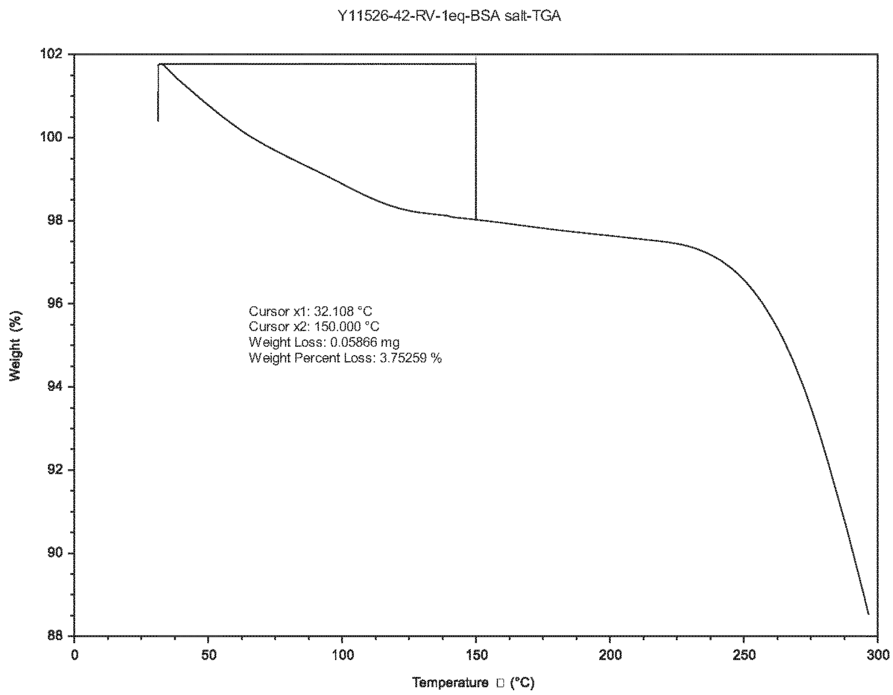


Figure 28

$^1$H-NMR spectrum of the amorphous 1 eq. besylate salt (Sample No. Y11526-42-SU9-methanol-dichloromethane)

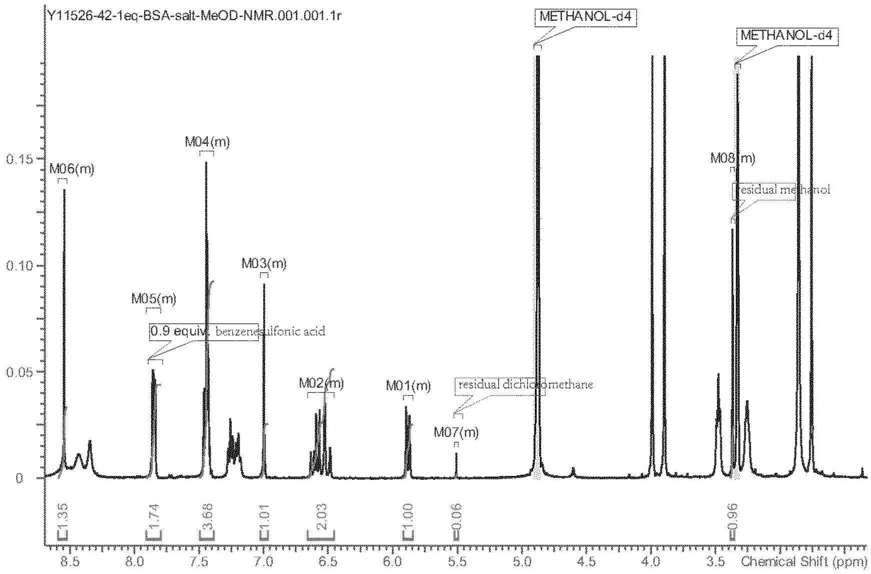


Figure 29

PLM photograph of the amorphous 1 eq. besylate salt (Sample No. Y11526-42-SU9-methanol-dichloromethane)

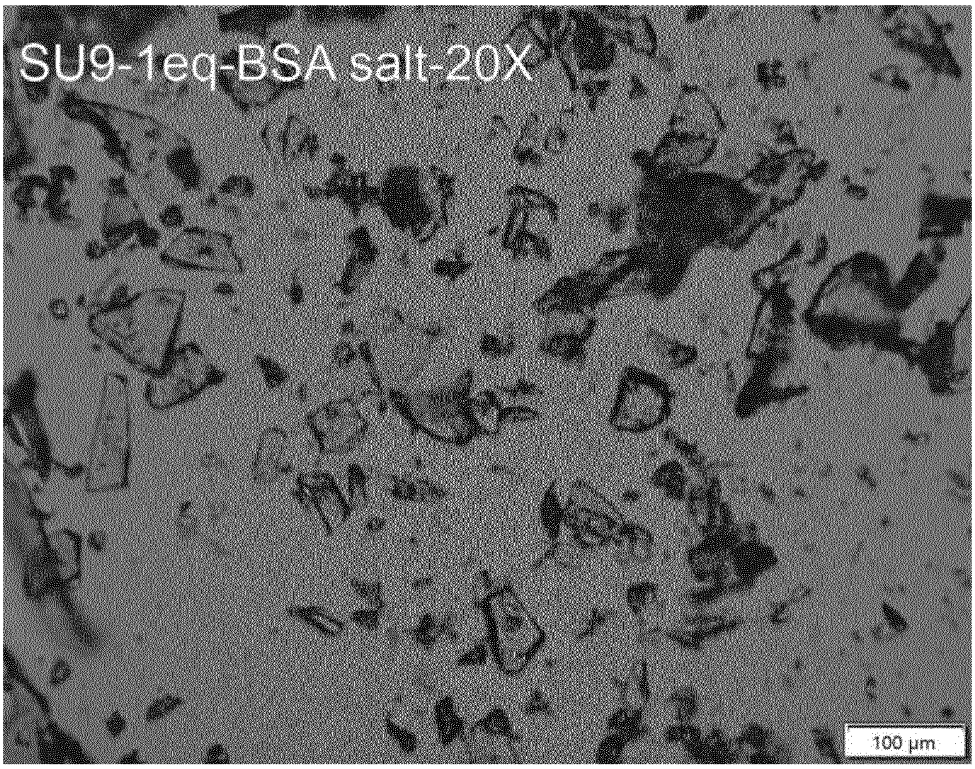


Figure 30

XRPD pattern of the amorphous 2 eq. besylate salt (Sample No. Y11526-30-SU1-water)

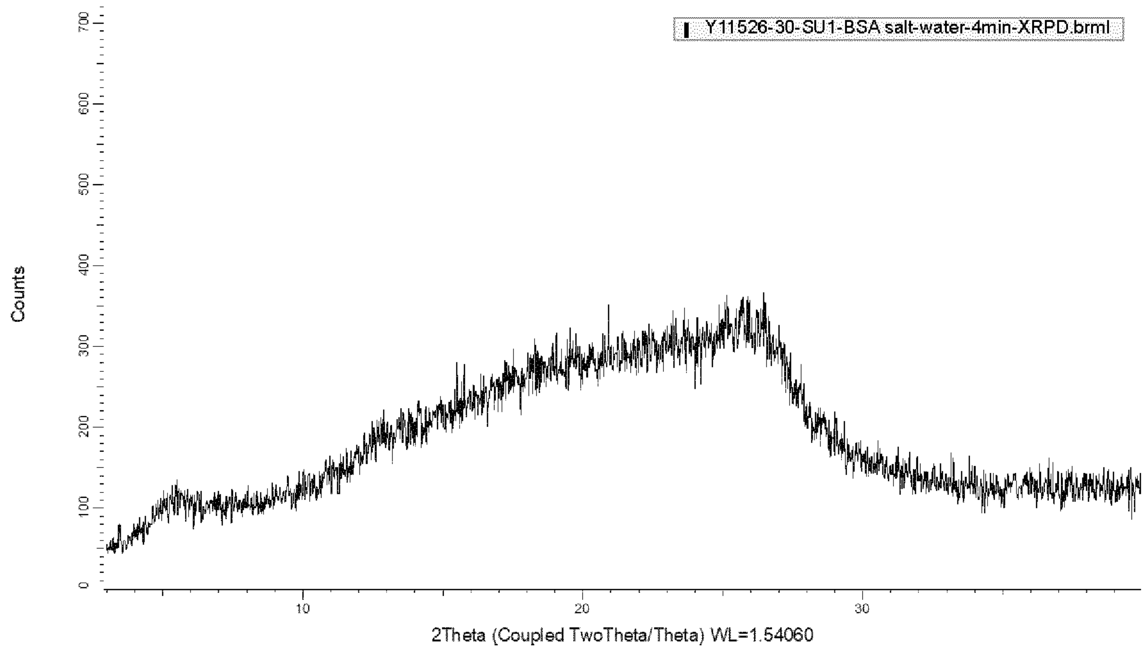


Figure 31

DSC profile of the amorphous 2 eq. besylate salt (Sample No. Y11526-30-SU1-water)

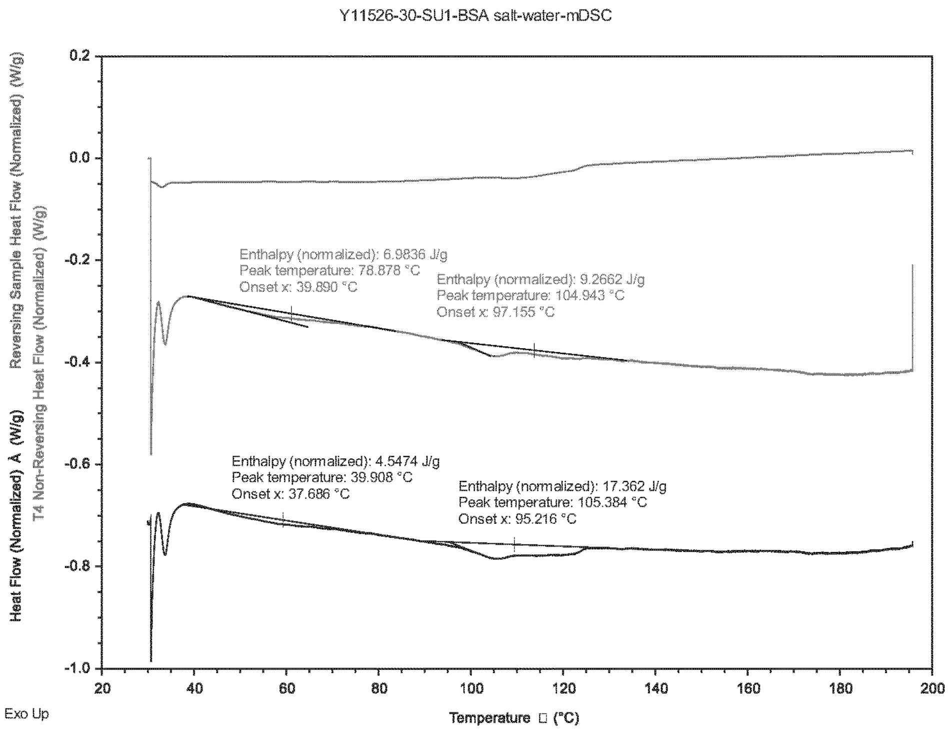


Figure 32

TGA profile of the amorphous 2 eq. besylate salt (Sample No. Y11526-30-SU1-water)

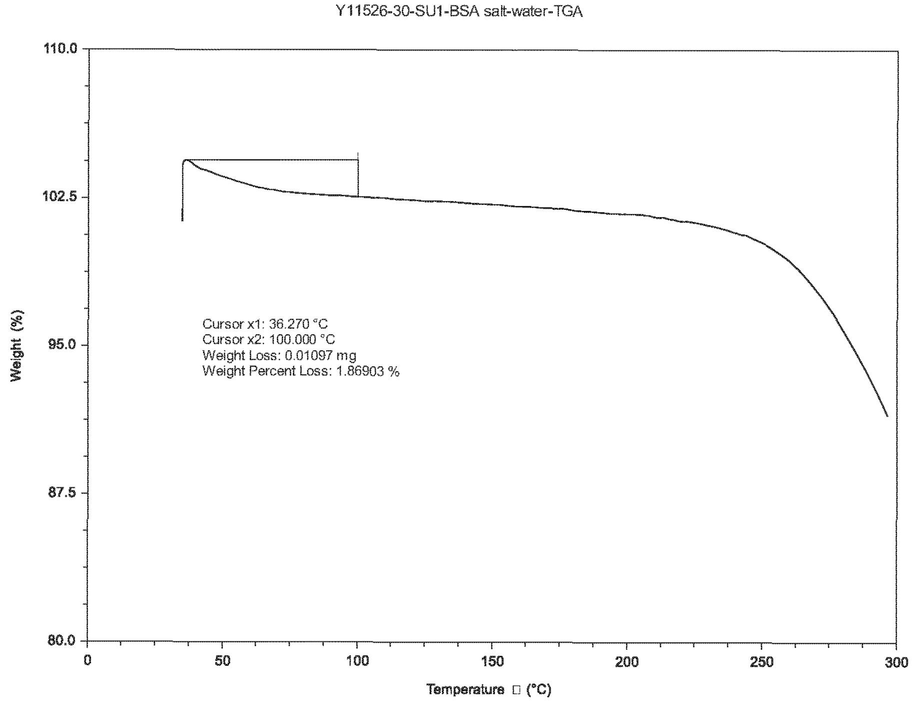


Figure 33

$^1$H-NMR spectrum of the amorphous 2 eq. besylate salt (Sample No. Y11526-30-SU1-water)

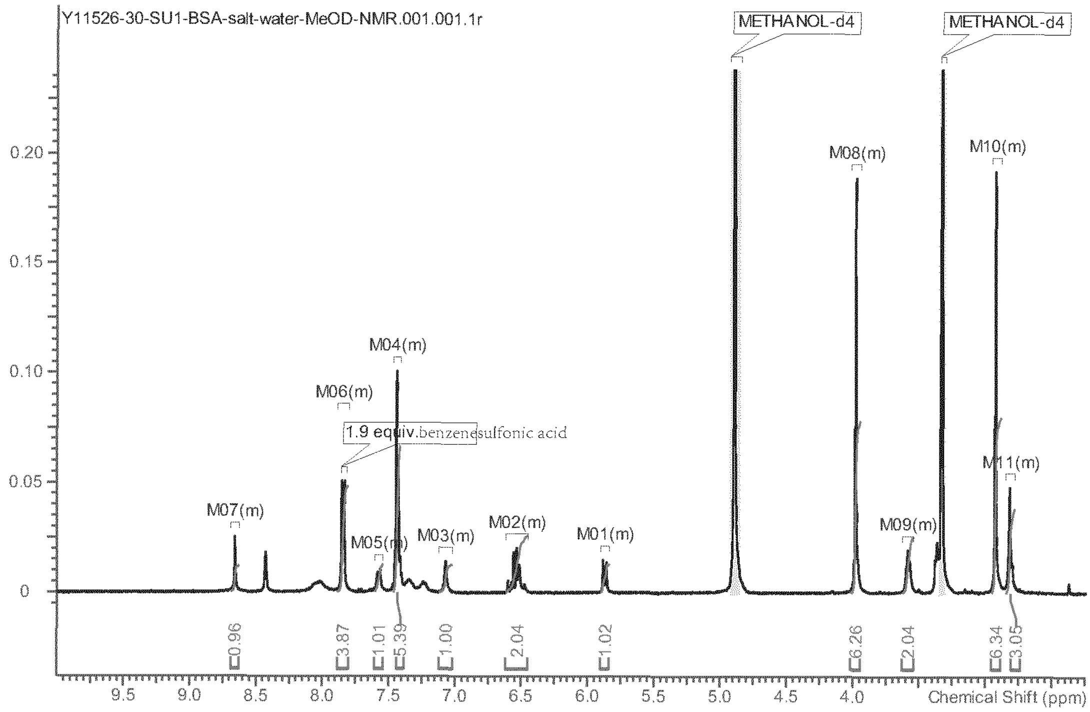


Figure 34

PLM photograph of the amorphous 2 eq. besylate salt (Sample No. Y11526-30-SU1-water)

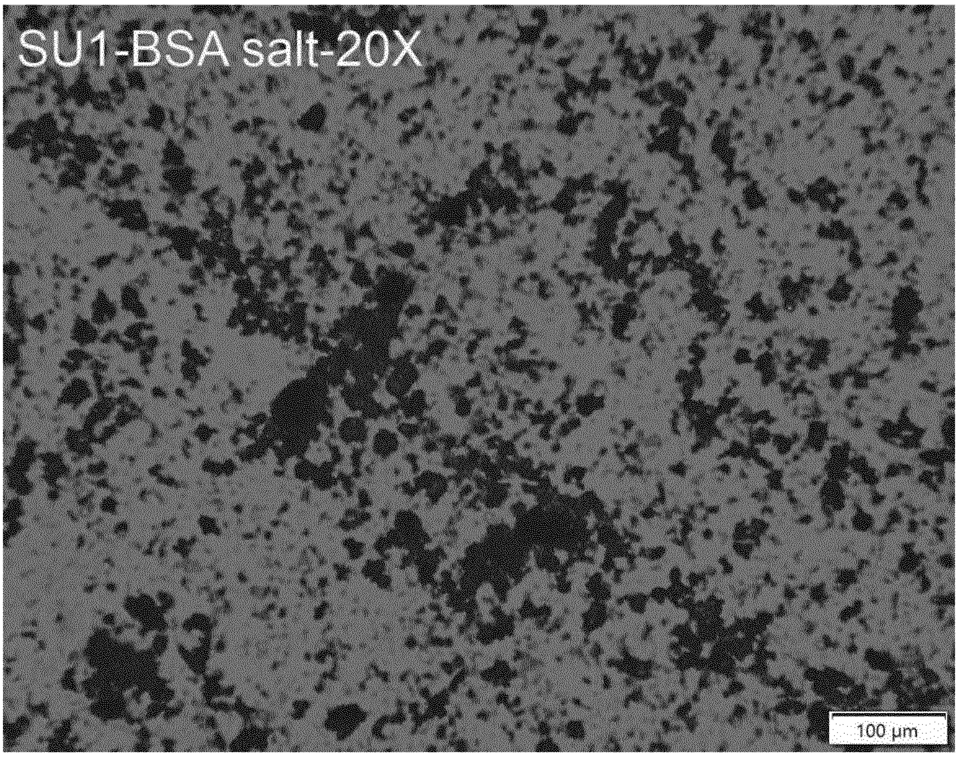


Figure 35

XRPD pattern of the amorphous 1 eq. esylate salt (Sample No. Y11526-28-SU7-methanol-dichloromethane)

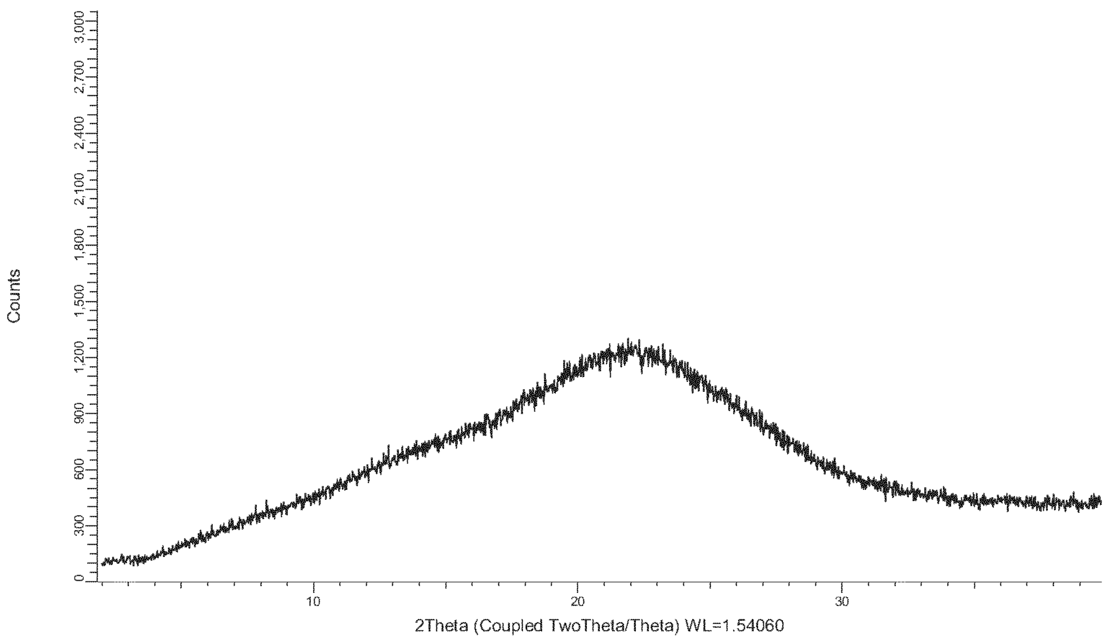


Figure 36

DSC profile of the amorphous 1 eq. esylate salt (Sample No. Y11526-28-SU7-methanol-dichloromethane)

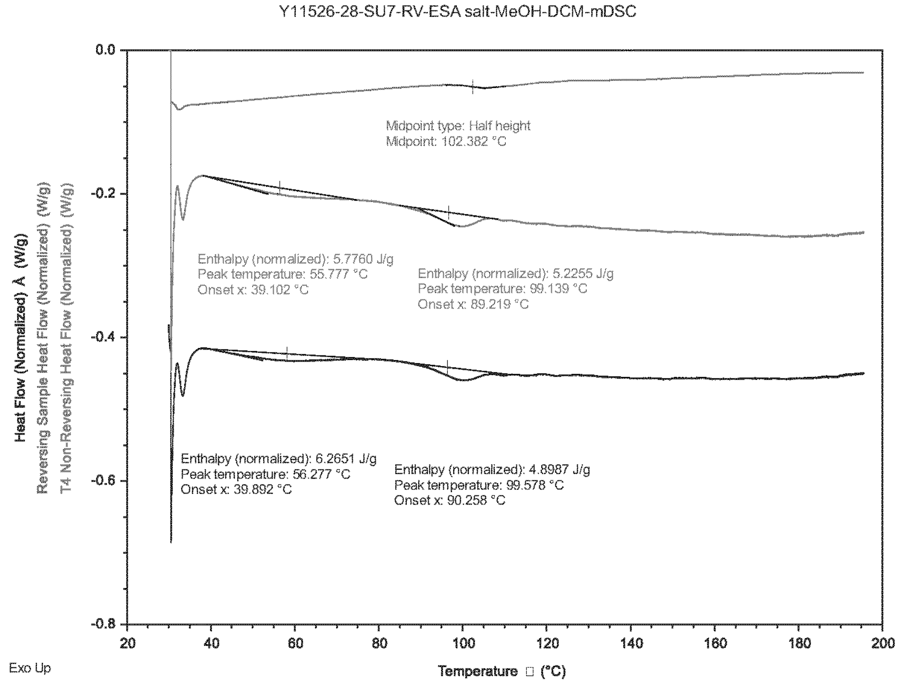


Figure 37

TGA profile of the amorphous 1 eq. esylate salt (Sample No. Y11526-28-SU7-methanol-dichloromethane)

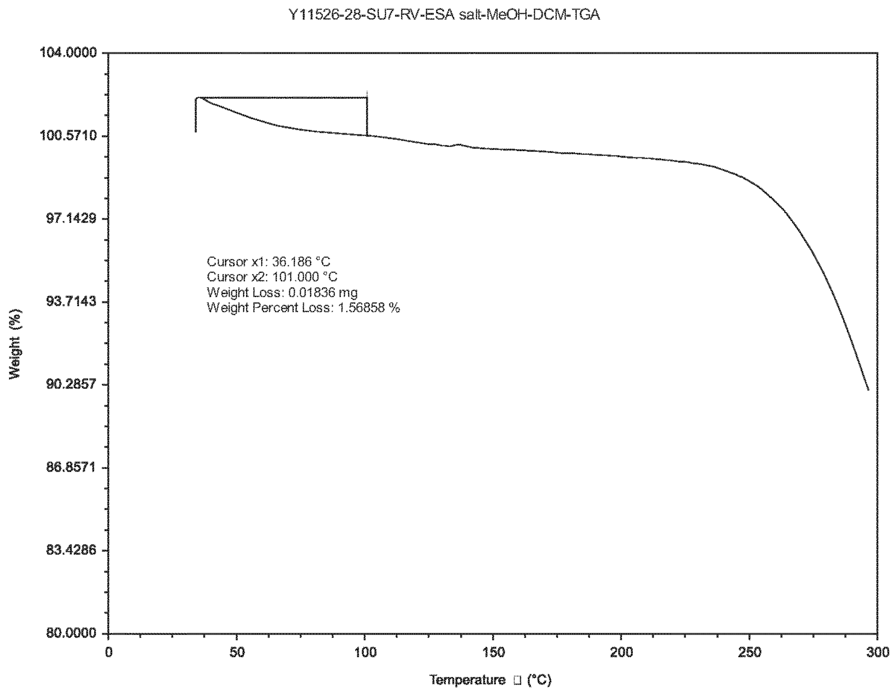


Figure 38

$^1$H-NMR spectrum of the amorphous 1 eq. esylate salt (Sample No. Y11526-28-SU7-methanol-dichloromethane)

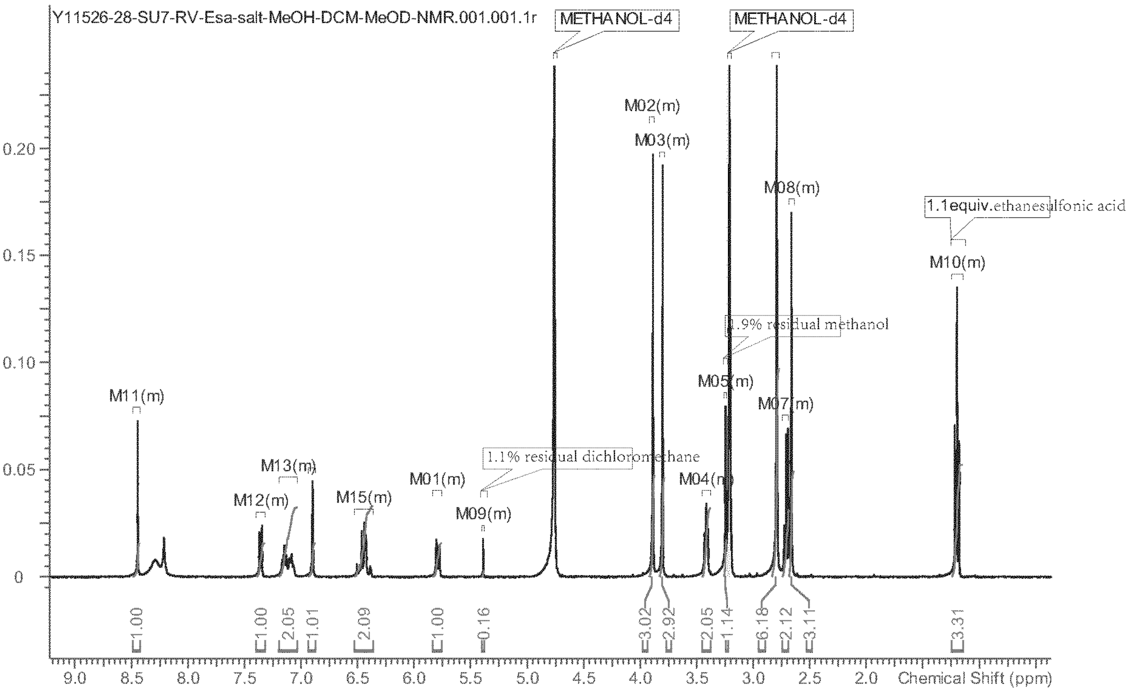


Figure 39

PLM photograph of the amorphous 1 eq. esylate salt (Sample No. Y11526-28-SU7-methanol-dichloromethane)

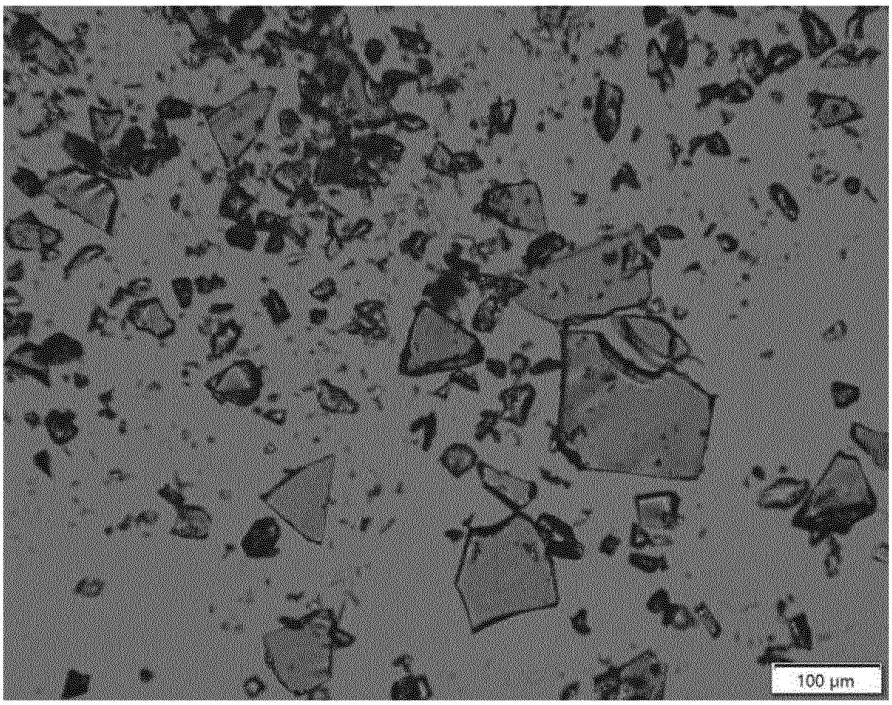


Figure 40

XRPD pattern of the amorphous 1 eq. maleate salt (Sample No. Y11526-30-SU2-water)

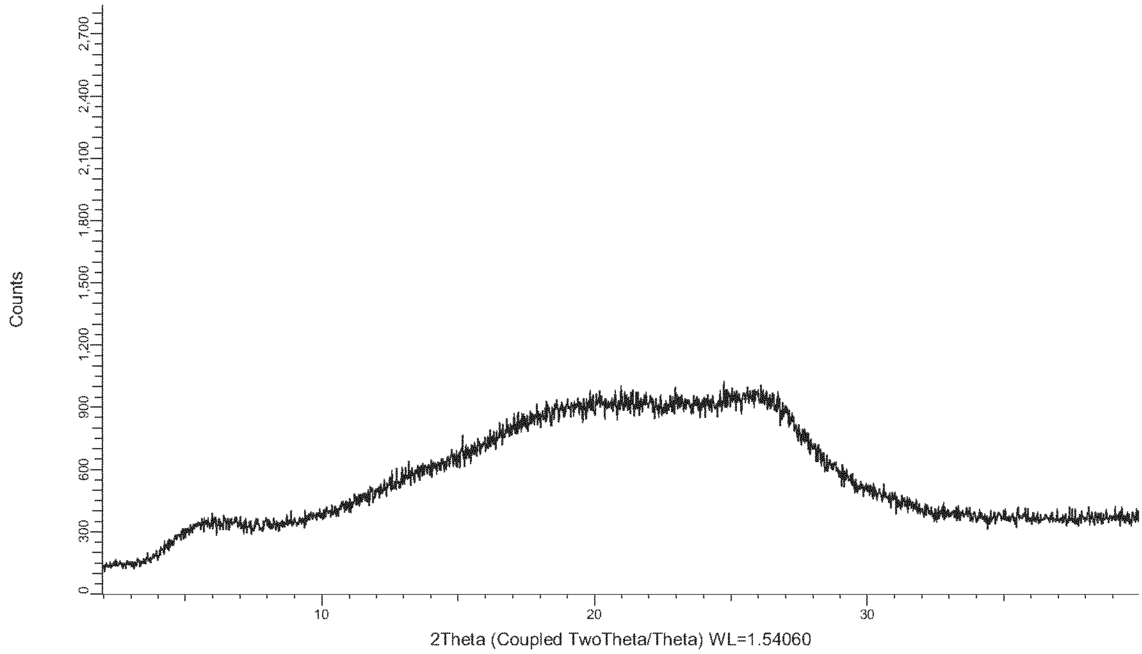


Figure 41

DSC profile of the amorphous 1 eq. maleate salt (Sample No. Y11526-30-SU2-water)

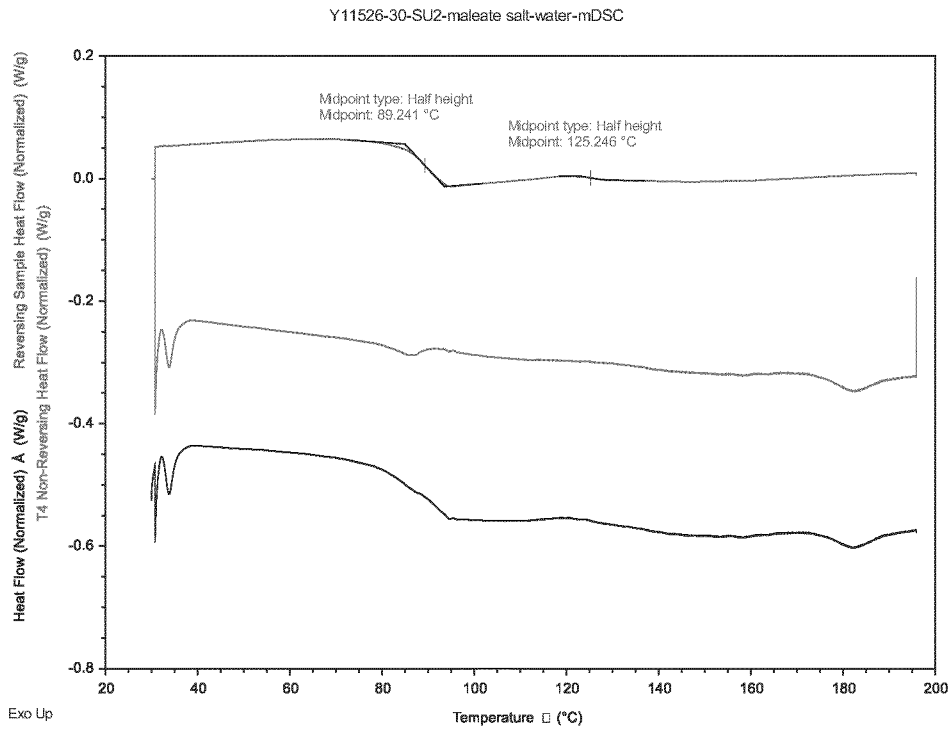


Figure 42

TGA profile of the amorphous 1 eq. maleate salt (Sample No. Y11526-30-SU2-water)

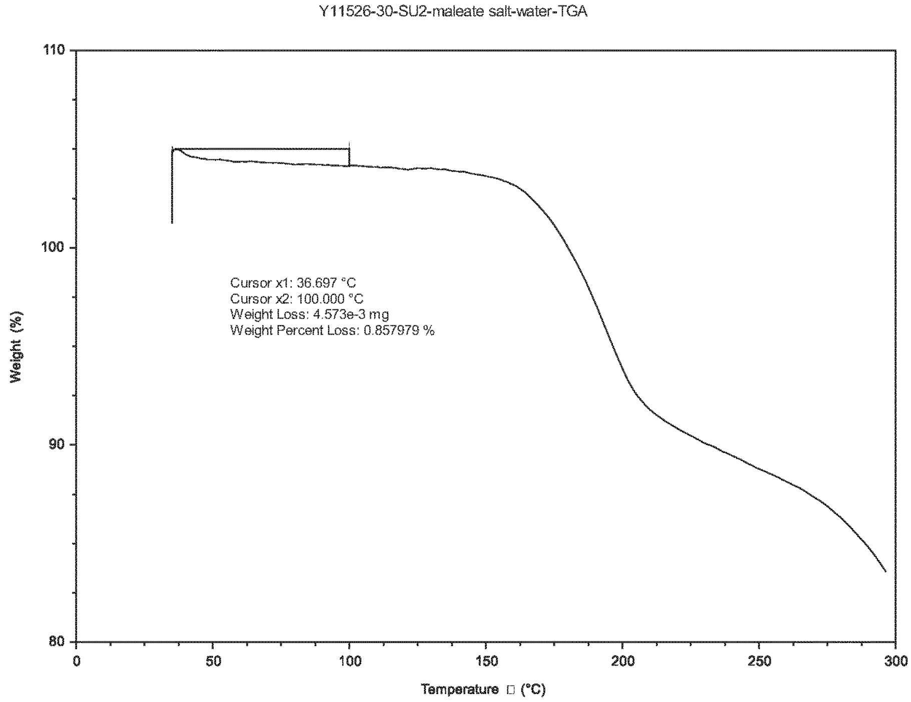


Figure 43

$^1$H-NMR spectrum of the amorphous 1 eq. maleate salt (Sample No. Y11526-30-SU2-water)

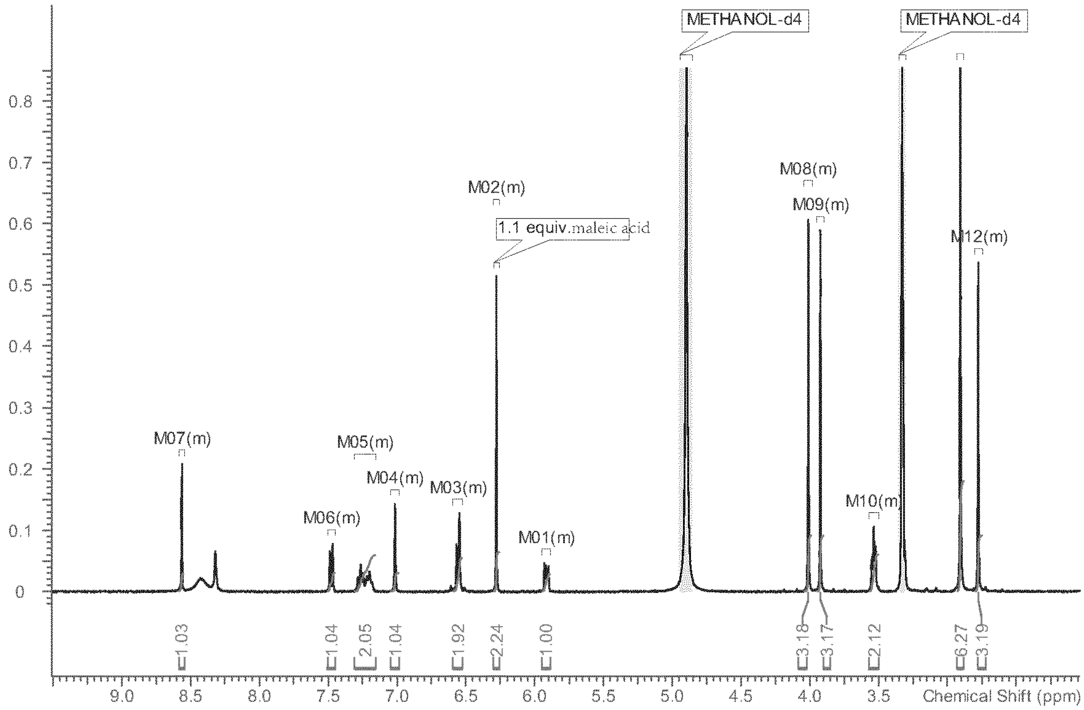


Figure 44

PLM photograph of the amorphous 1 eq. maleate salt (Sample No. Y11526-30-SU2-water)

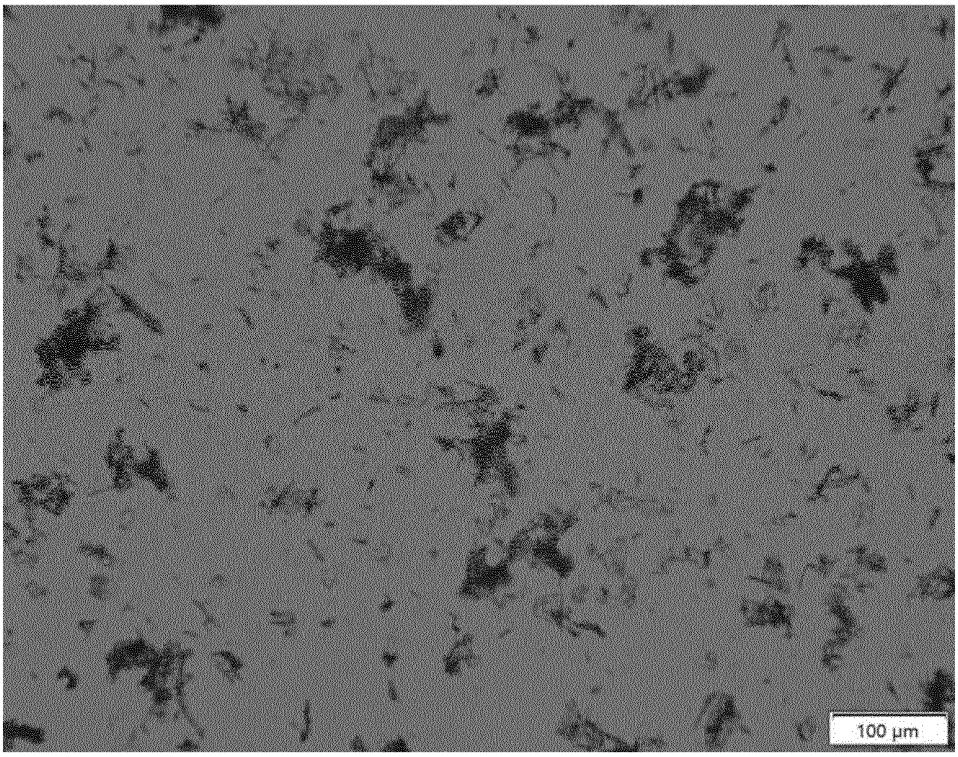


Figure 45

XRPD pattern of the crystalline 1 eq. hydrobromide salt (Sample No. Y11526-33-SU8-methanol-dichloromethane)

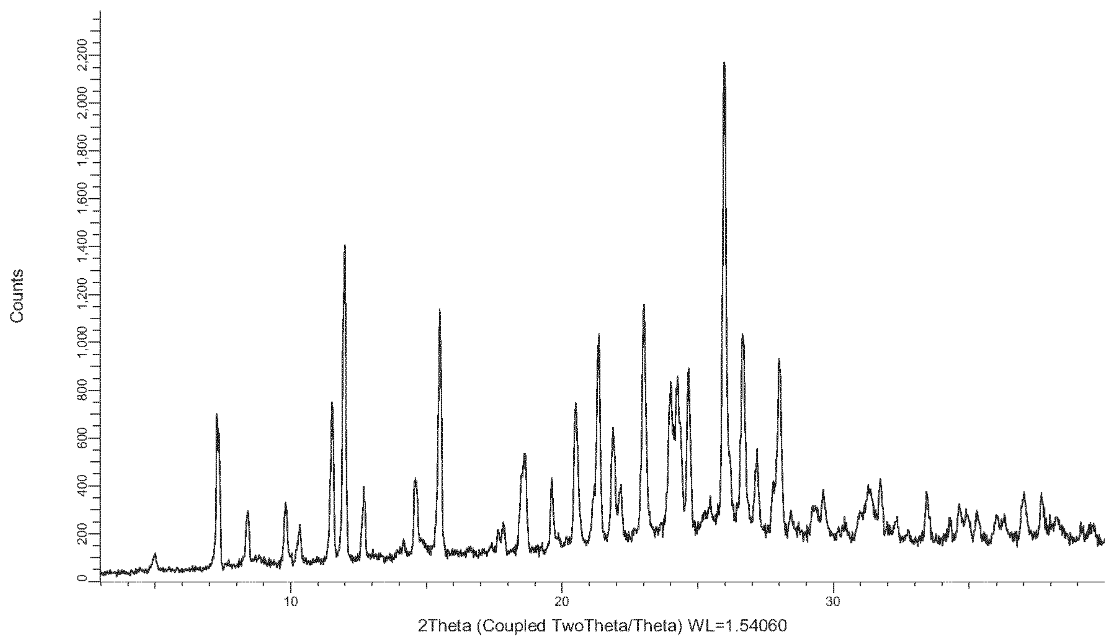


Figure 46

DSC profile of the crystalline 1 eq. hydrobromide salt (Sample No. Y11526-33-SU8-methanol-dichloromethane)

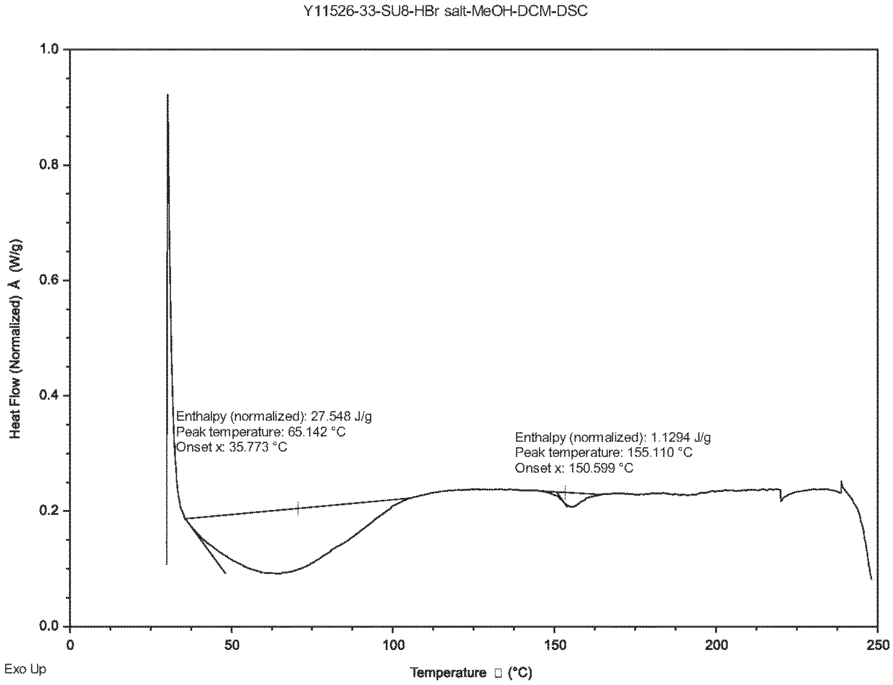


Figure 47

TGA profile of the crystalline 1 eq. hydrobromide salt (Sample No. Y11526-33-SU8-methanol-dichloromethane)

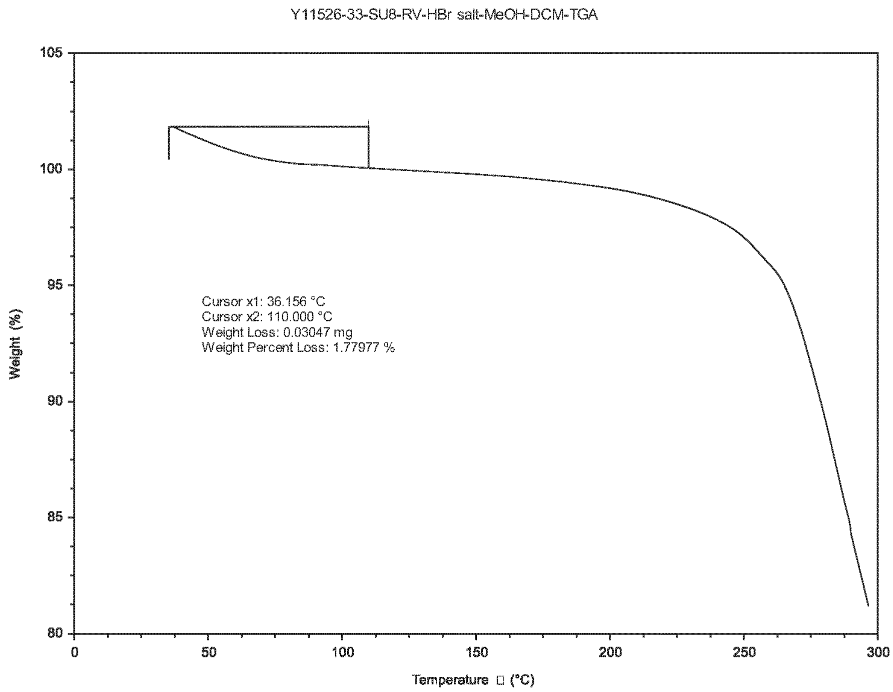


Figure 48

$^1$H-NMR spectrum of the crystalline 1 eq. hydrobromide salt (Sample No. Y11526-33-SU8-methanol-dichloromethane)

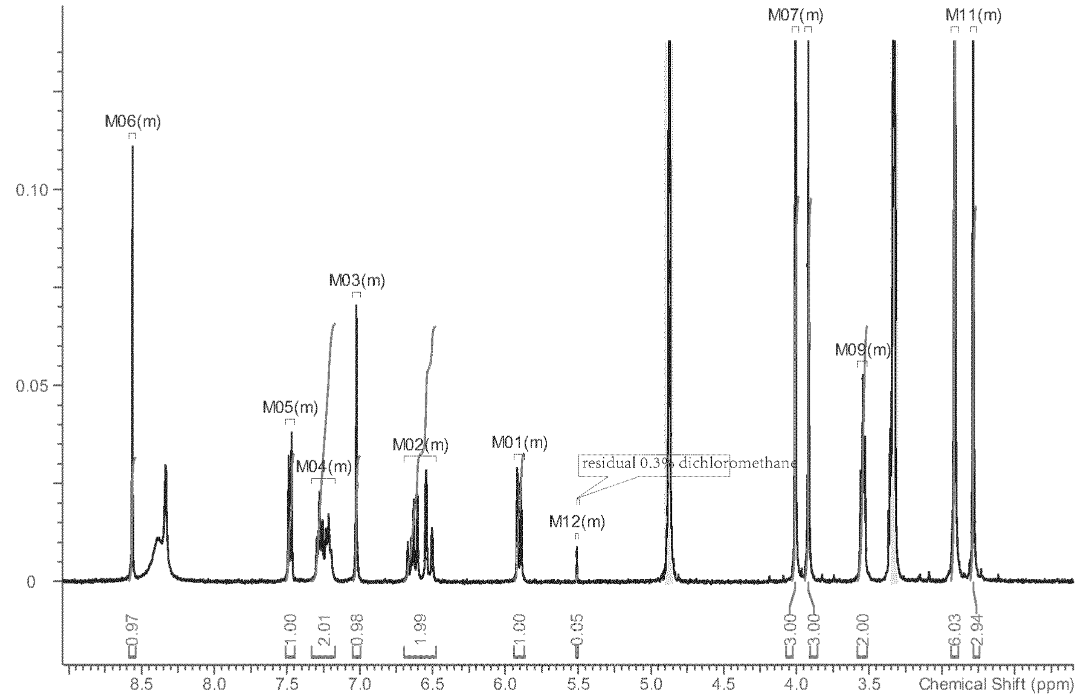


Figure 49

PLM photograph of the crystalline 1 eq. hydrobromide salt (Sample No. Y11526-33-SU8-methanol-dichloromethane)

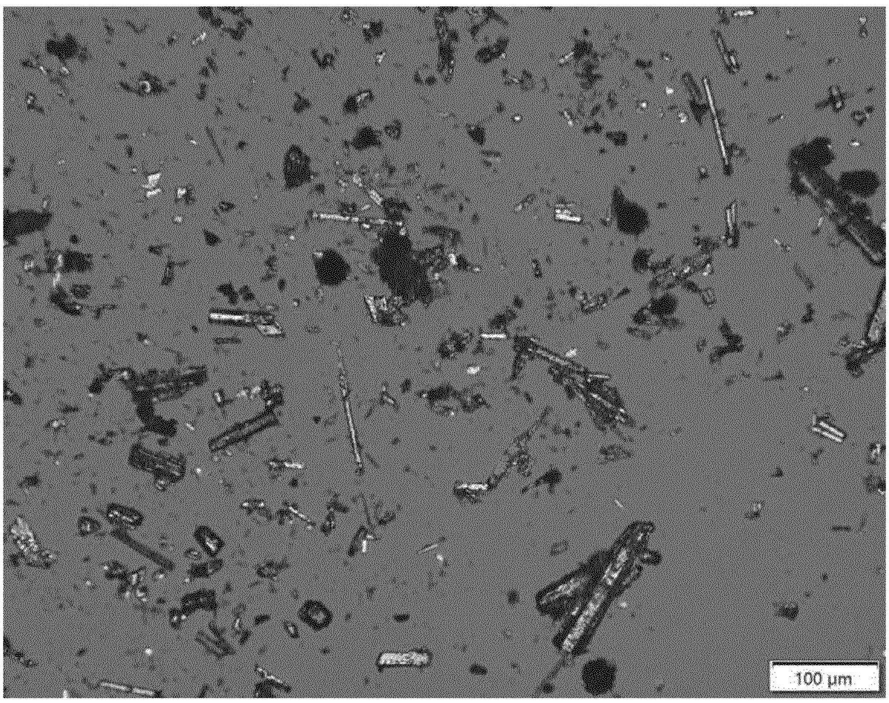


Figure 50

XRPD pattern of the amorphous nitrate salt (Sample No. Y11526-18-RV7-methanol-dichloromethane)

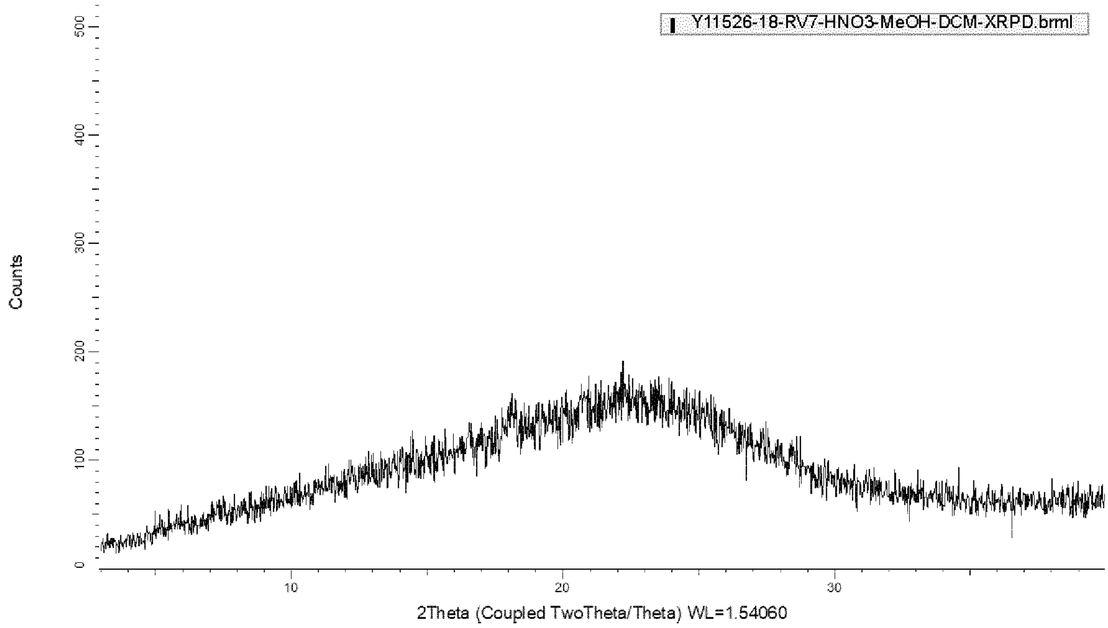


Figure 51

$^1$H-NMR spectrum of the amorphous nitrate salt (Sample No. Y11526-18-RV7-methanol-dichloromethane)

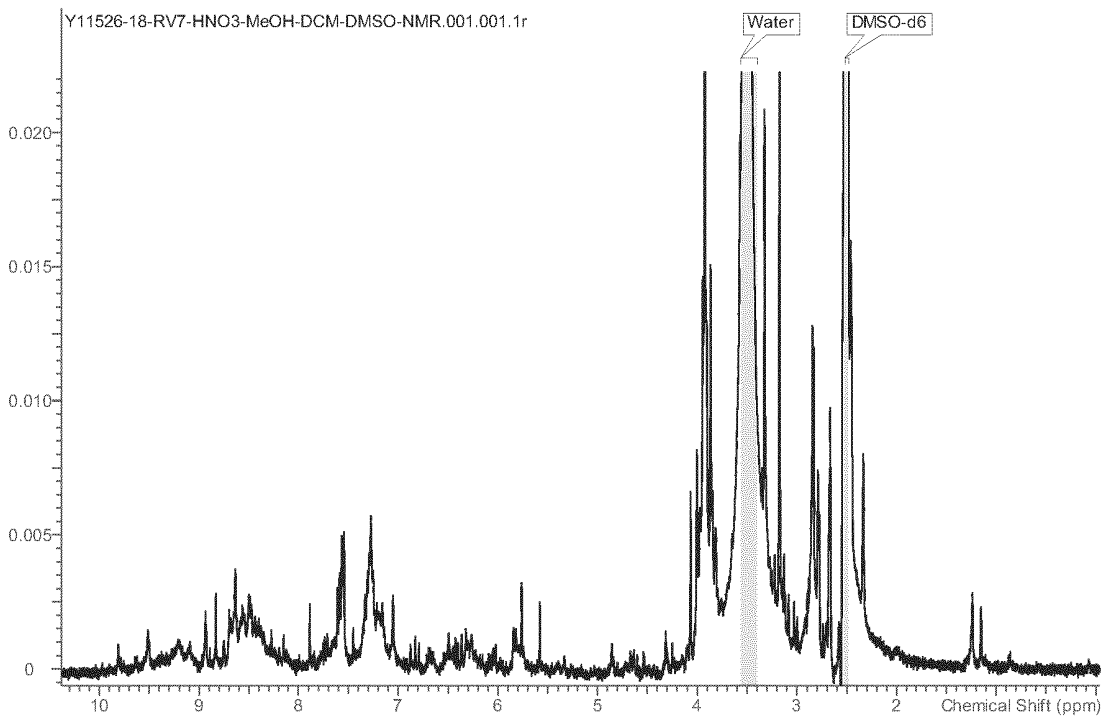


Figure 52

XRPD pattern of the amorphous sulfate salt (Sample No. Y11526-15-RV3-methanol-dichloromethane)

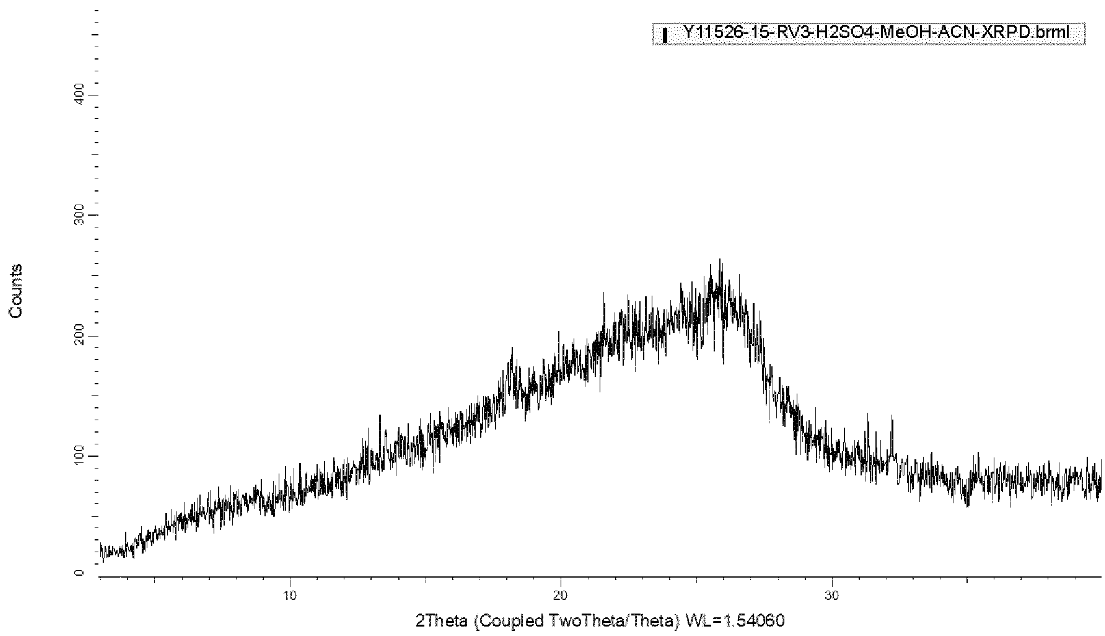


Figure 53

DSC profile of the amorphous sulfate salt (Sample No. Y11526-15-RV3-methanol-acetonitrile)

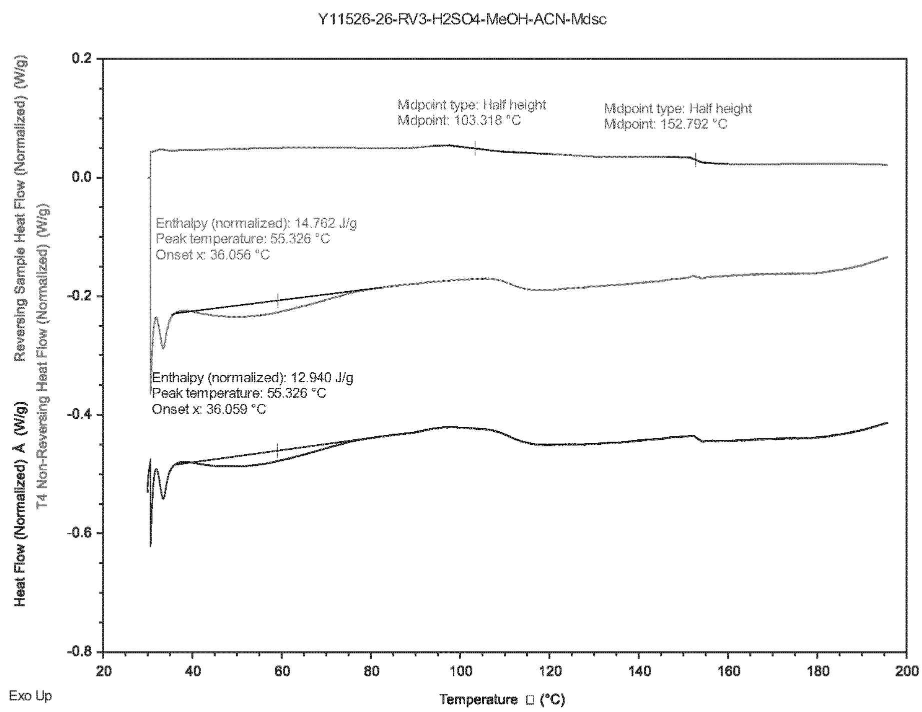


Figure 54

TGA profile of the amorphous sulfate salt (Sample No. Y11526-15-RV3-methanol-dichloromethane)

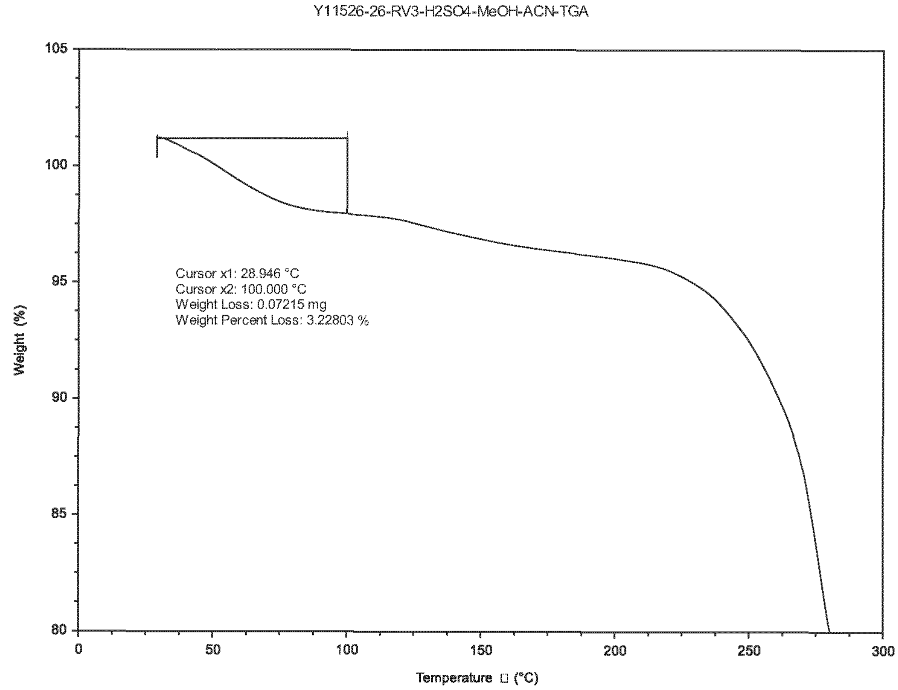


Figure 55

$^1$H-NMR spectrum of the amorphous sulfate salt (Sample No. Y11526-15-RV3-methanol-acetonitrile)

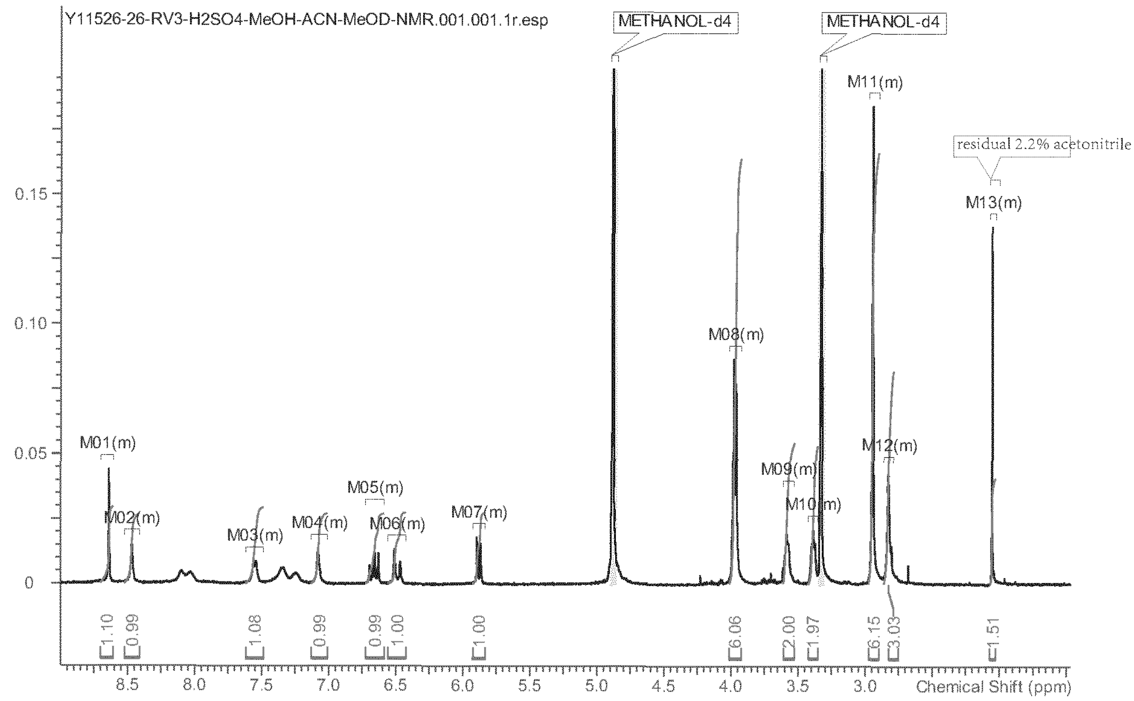


Figure 56

PLM photograph of the amorphous sulfate salt (Sample No. Y11526-15-RV3-methanol-acetonitrile)

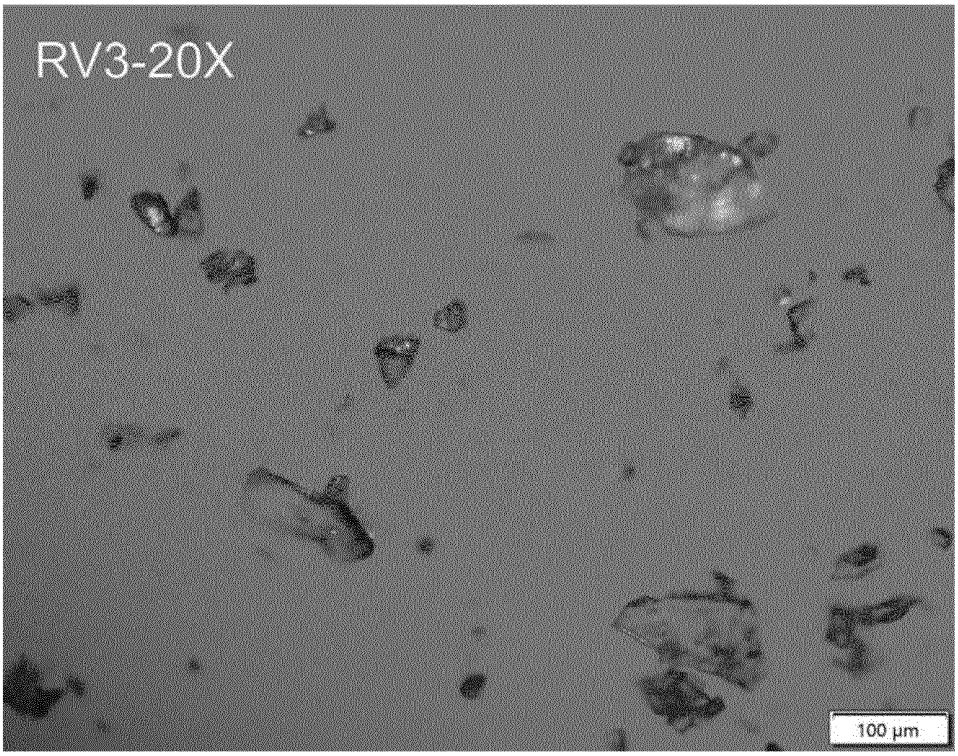


Figure 57

XRPD pattern of the amorphous 2 eq. p-tosylate salt (Sample No. Y11526-23-FD11-water)

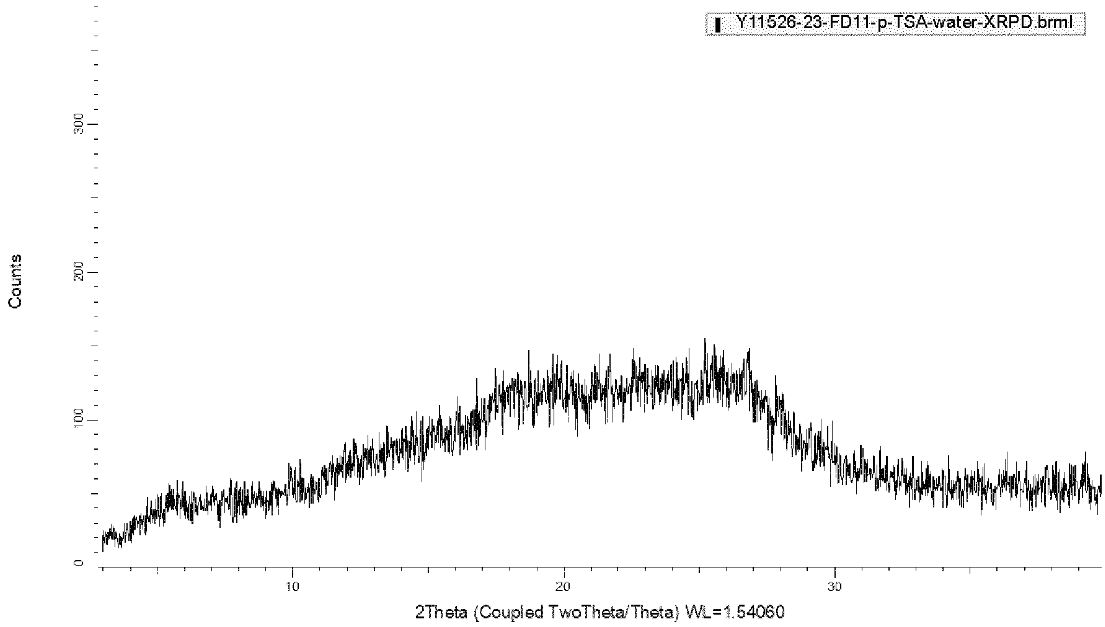


Figure 58

DSC profile of the amorphous 2 eq. p-tosylate salt (Sample No. Y11526-23-FD11-water)

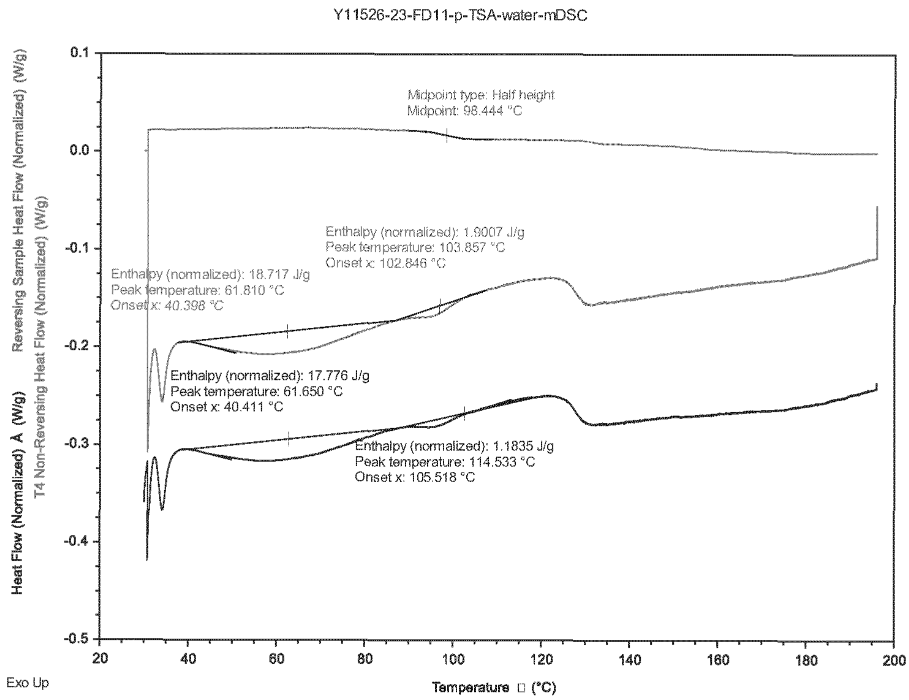


Figure 59

TGA profile of the amorphous 2 eq. p-tosylate salt (Sample No. Y11526-23-FD11-water)

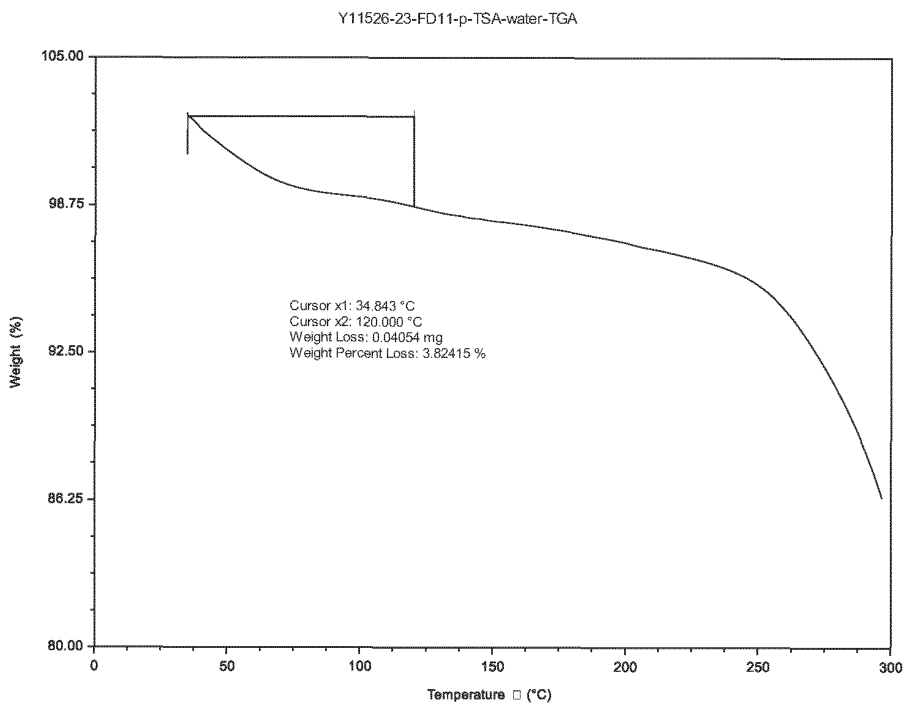


Figure 60

$^{1}$H-NMR spectrum of the amorphous 2 eq. p-tosylate salt (Sample No. Y11526-23-FD11-water)

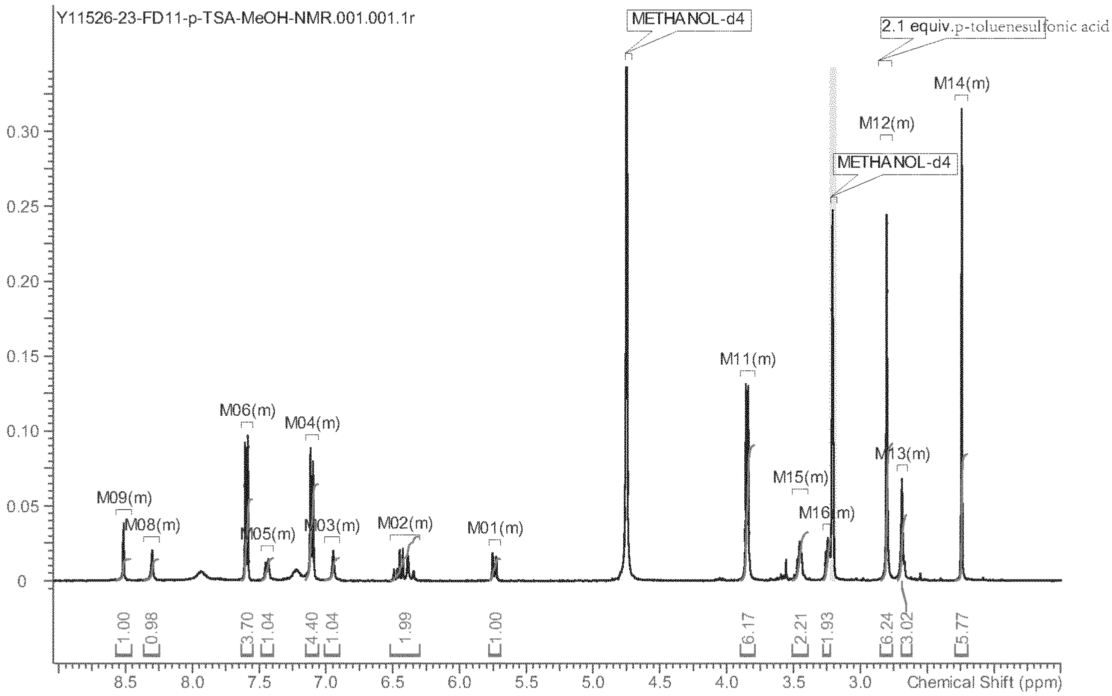


Figure 61

PLM photograph of the amorphous 2 eq. p-tosylate salt (Sample No. Y11526-23-FD11-water)

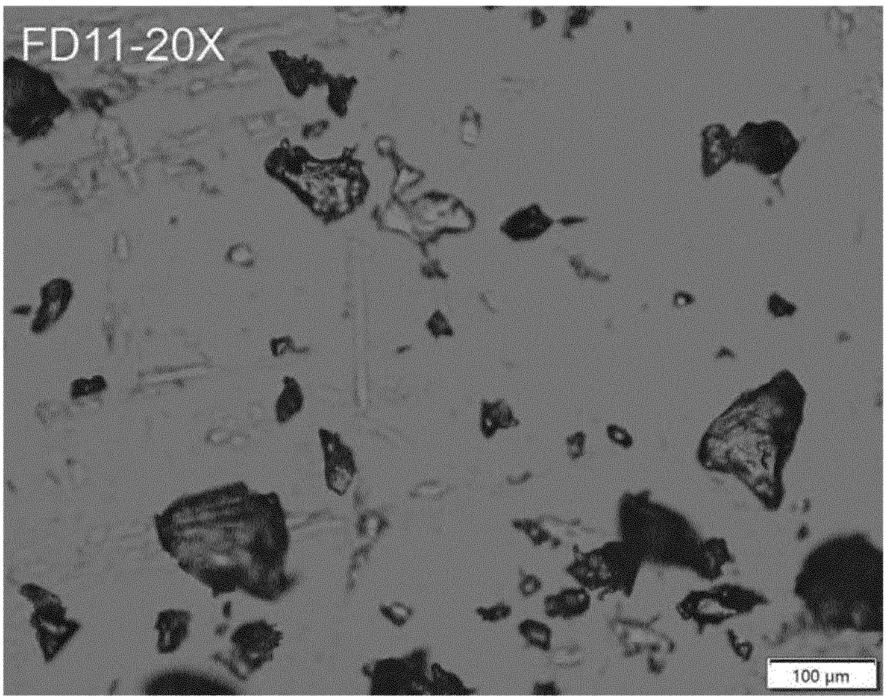


Figure 62

Photograph of the amorphous 2 eq. p-tosylate salt (Sample No. Y11526-23-FD11-water)

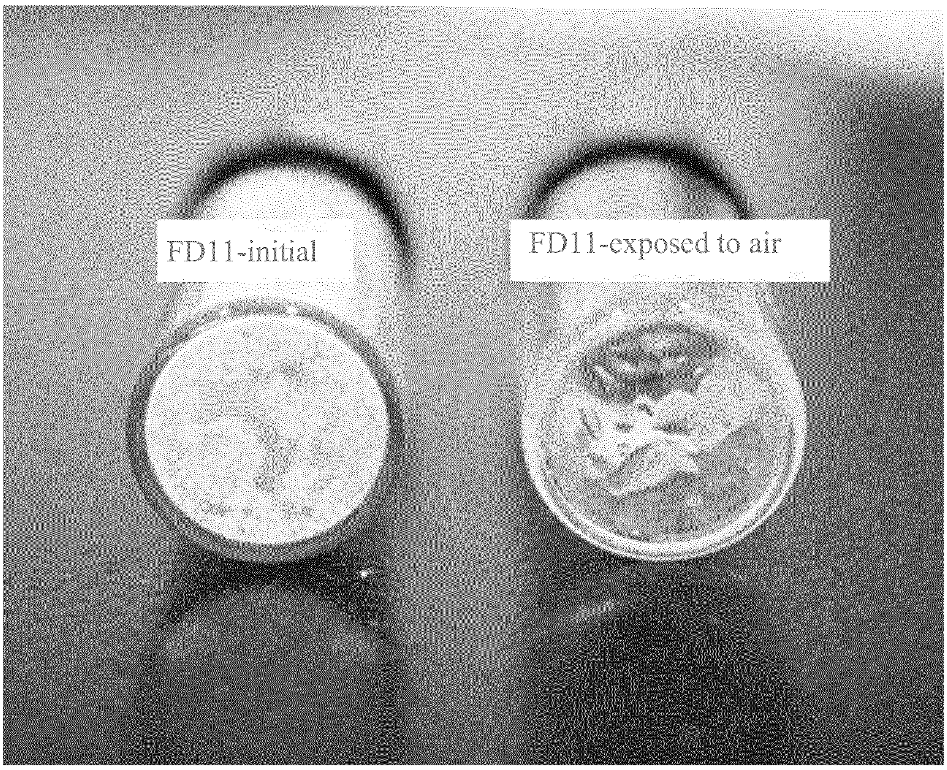


Figure 63

XRPD pattern of the amorphous sulfosalicylate salt (Sample No. Y11526-25-FD13-1,4-dioxane)

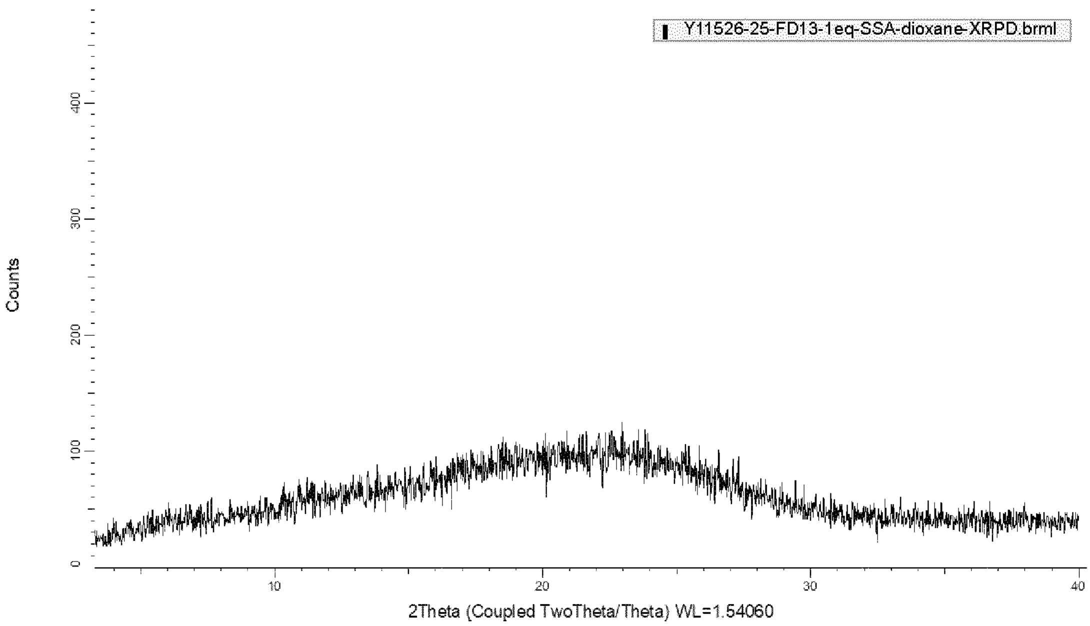


Figure 64

DSC profile of the amorphous sulfosalicylate salt (Sample No. Y11526-25-FD13-1,4-dioxane)

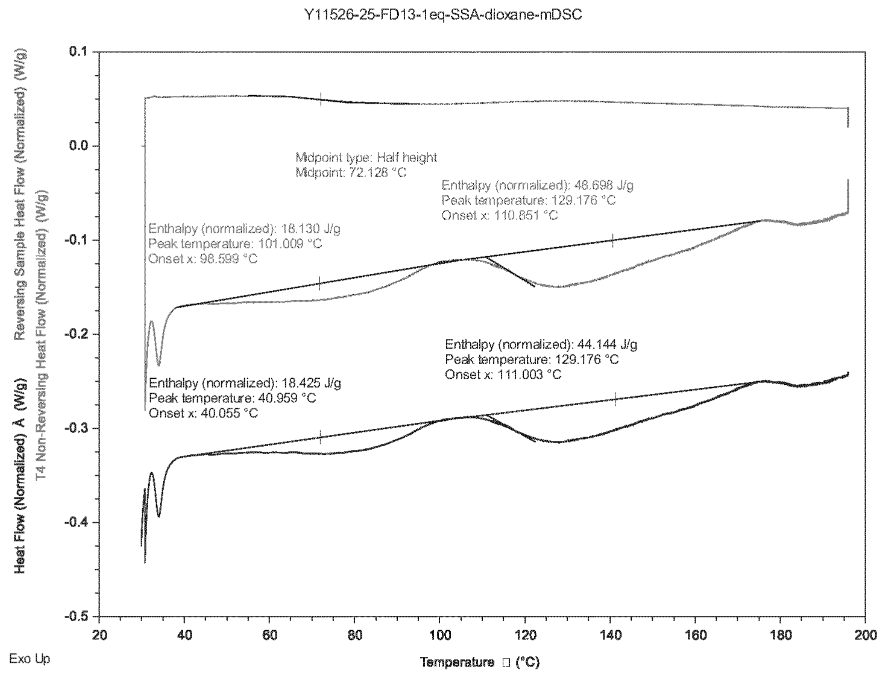


Figure 65

TGA profile of the amorphous sulfosalicylate salt (Sample No. Y11526-25-FD13-1,4-dioxane)

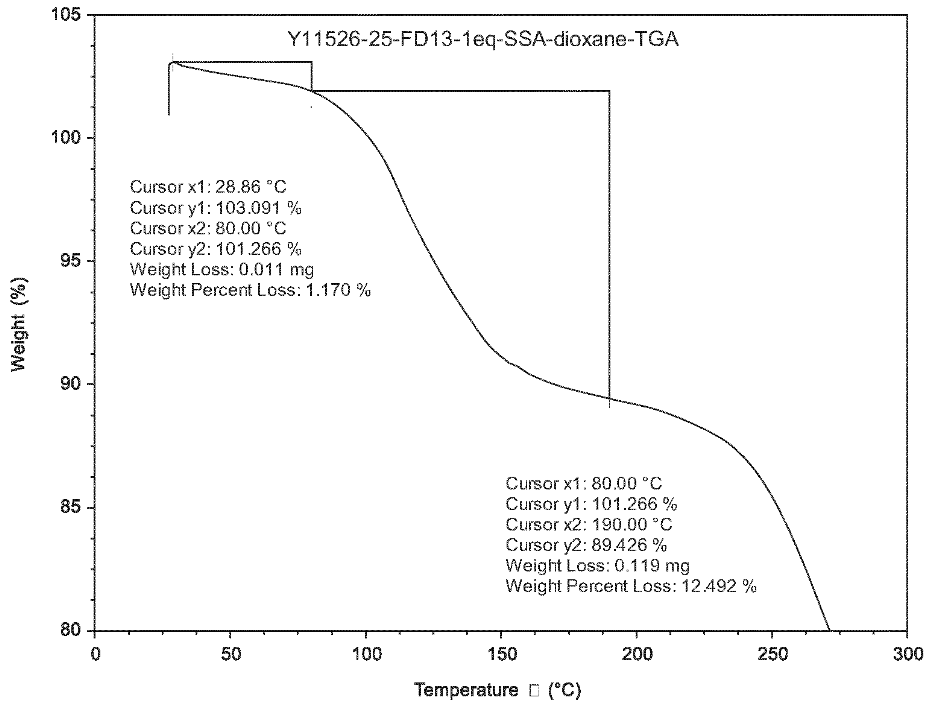


Figure 66

$^1$H-NMR spectrum of the amorphous sulfosalicylate salt (Sample No. Y11526-25-FD13-1,4-dioxane)

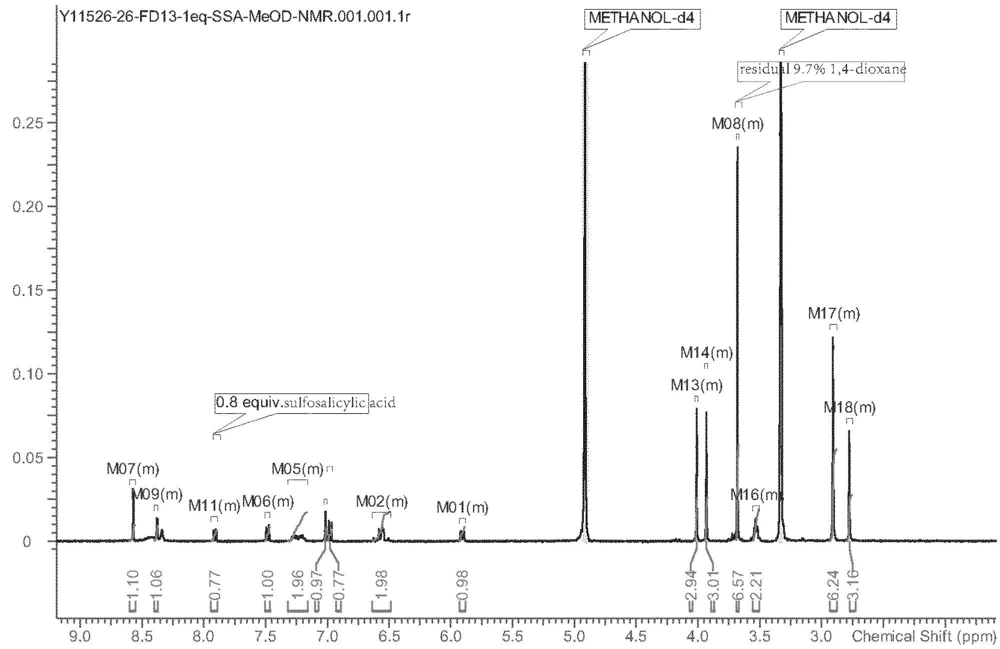


Figure 67

PLM photograph of the amorphous sulfosalicylate salt (Sample No. Y11526-25-FD13-1,4-dioxane)

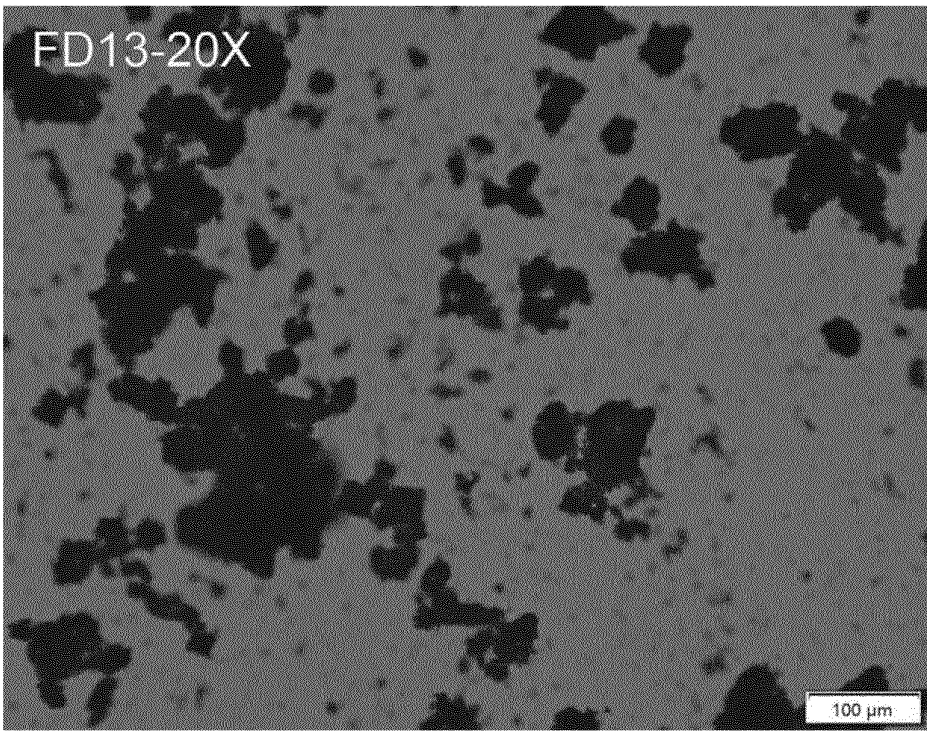


Figure 68

XRPD pattern of the amorphous sulfosalicylate salt (Sample No. Y11526-25-FD12-1,4-dioxane)

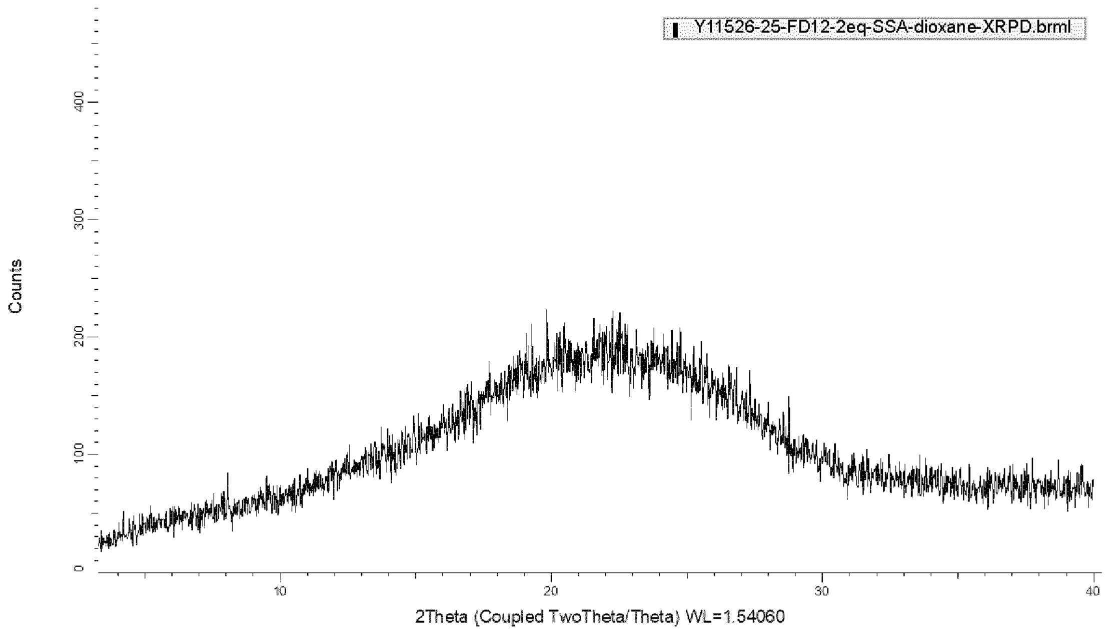


Figure 69

DSC profile of the amorphous sulfosalicylate salt (Sample No. Y11526-25-FD12-1,4-dioxane)

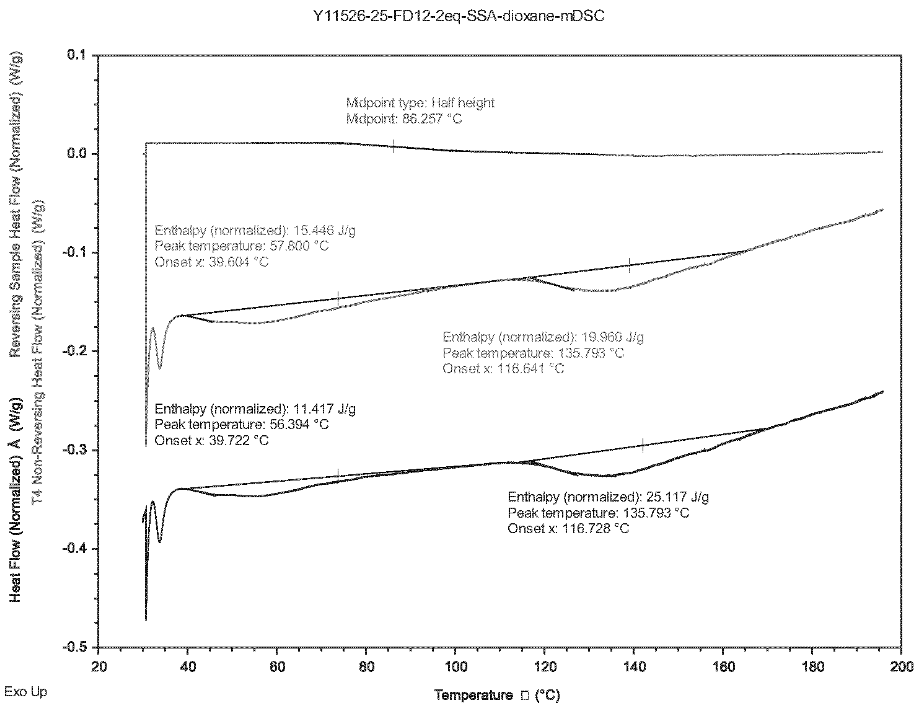


Figure 70

TGA profile of the amorphous sulfosalicylate salt (Sample No. Y11526-25-FD12-1,4-dioxane)

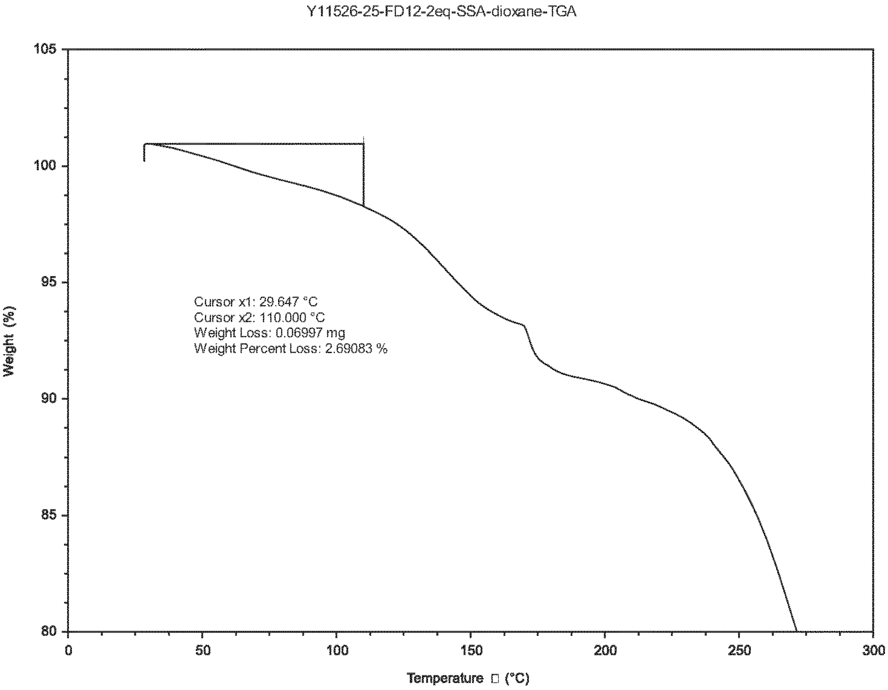


Figure 71

$^1$H-NMR spectrum of the amorphous sulfosalicylate salt (Sample No. Y11526-25-FD12-1,4-dioxane)

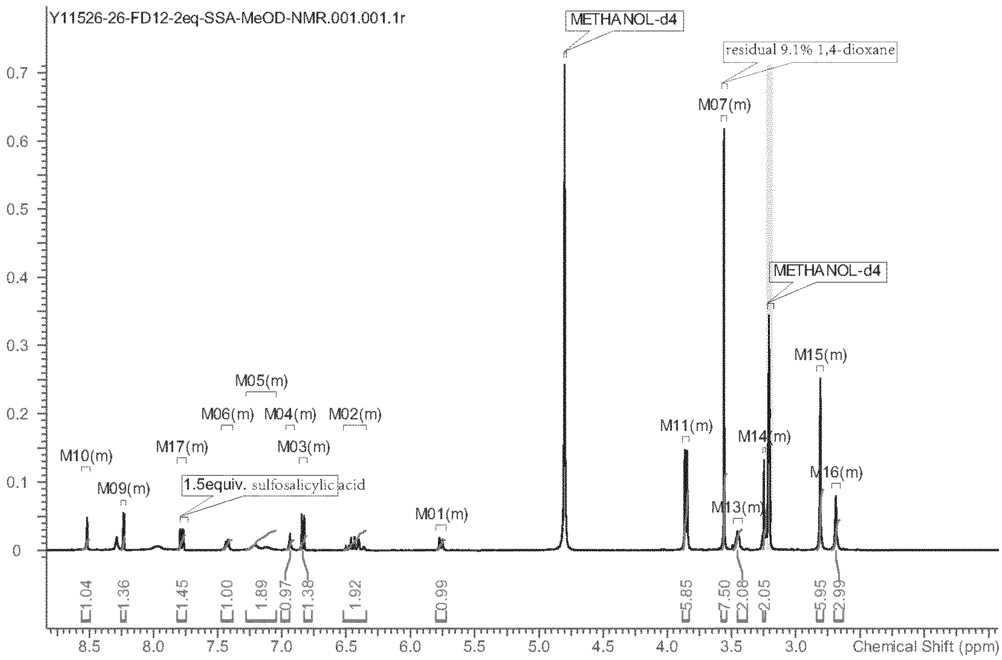


Figure 72

PLM photograph of the amorphous sulfosalicylate salt (Sample No. Y11526-25-FD12-1,4-dioxane)

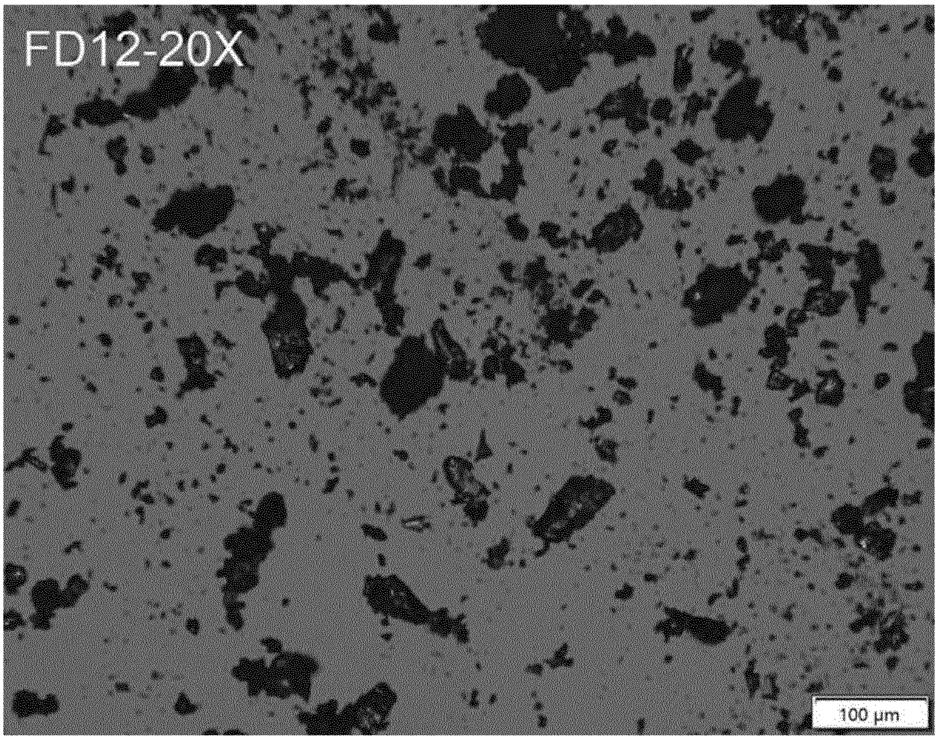


Figure 73

XRPD pattern of the amorphous L-malate salt (Sample No. Y11526-17-RV6-methanol-dichloromethane)

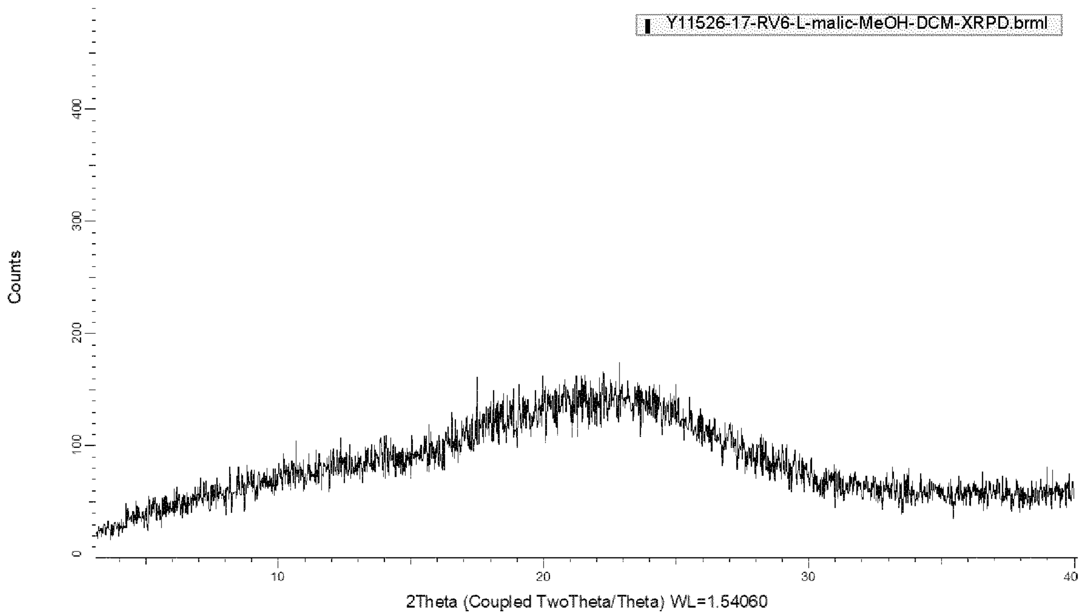


Figure 74

DSC profile of the amorphous L-malate salt (Sample No. Y11526-17-RV6-methanol-dichloromethane)

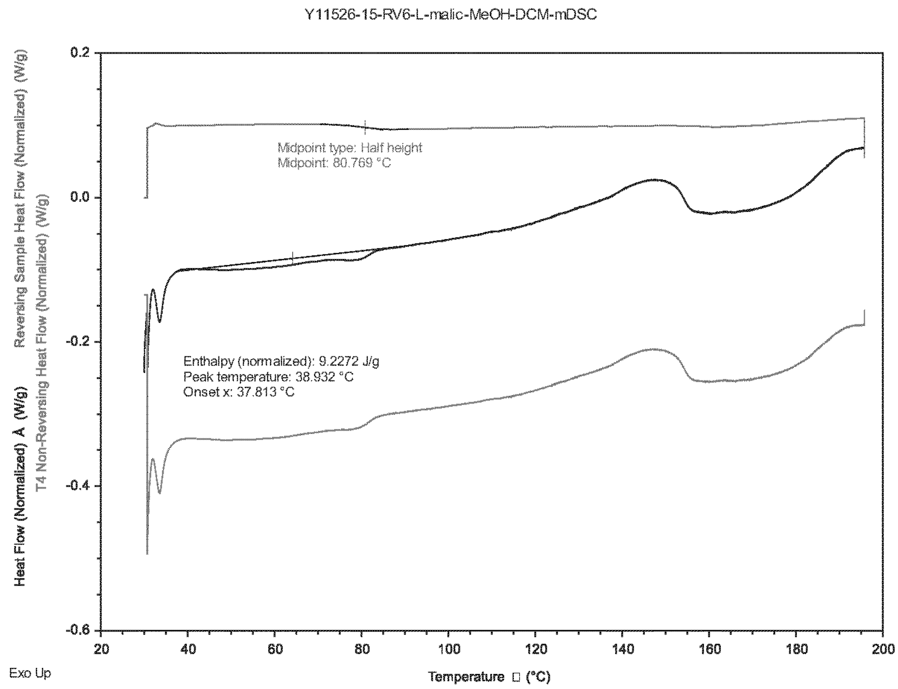


Figure 75

TGA profile of the amorphous L-malate salt (Sample No. Y11526-17-RV6-methanol-dichloromethane)

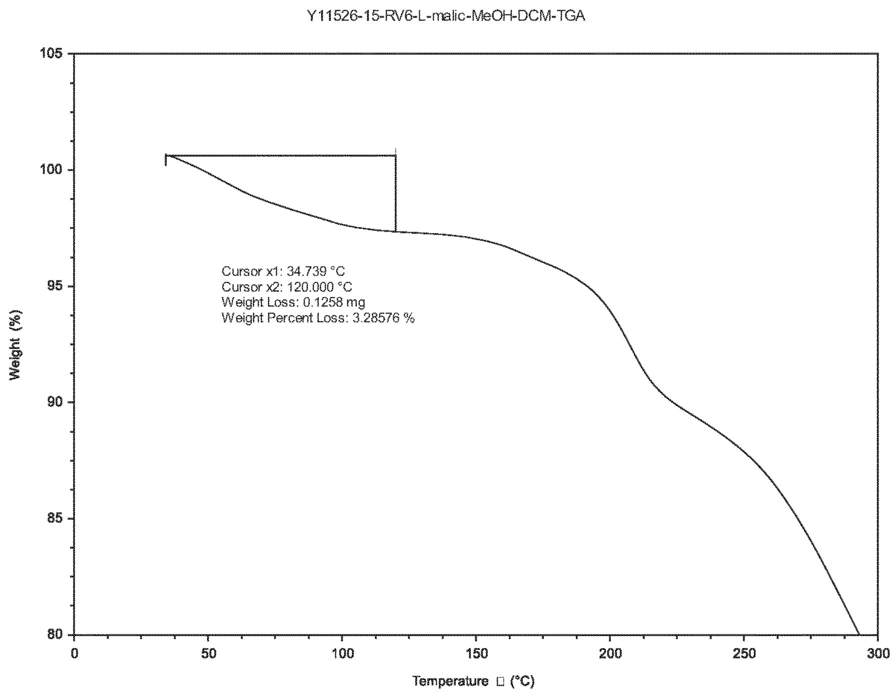


Figure 76

$^1$H-NMR spectrum of the amorphous L-malate salt (Sample No. Y11526-17-RV6-methanol-dichloromethane)

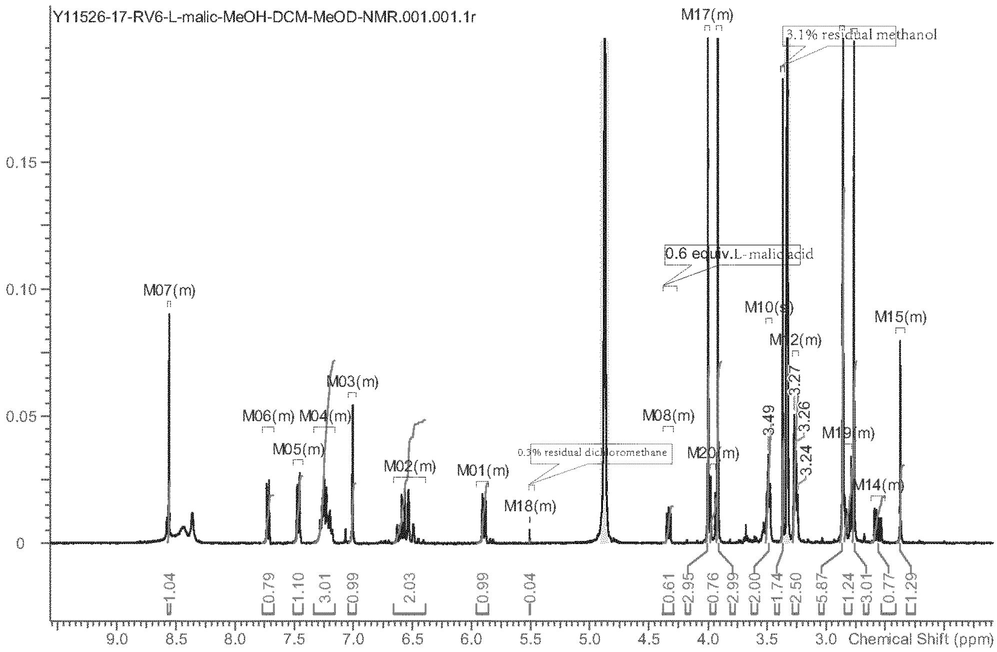


Figure 77

PLM photograph of the amorphous L-malate salt (Sample No. Y11526-17-RV6-methanol-dichloromethane)

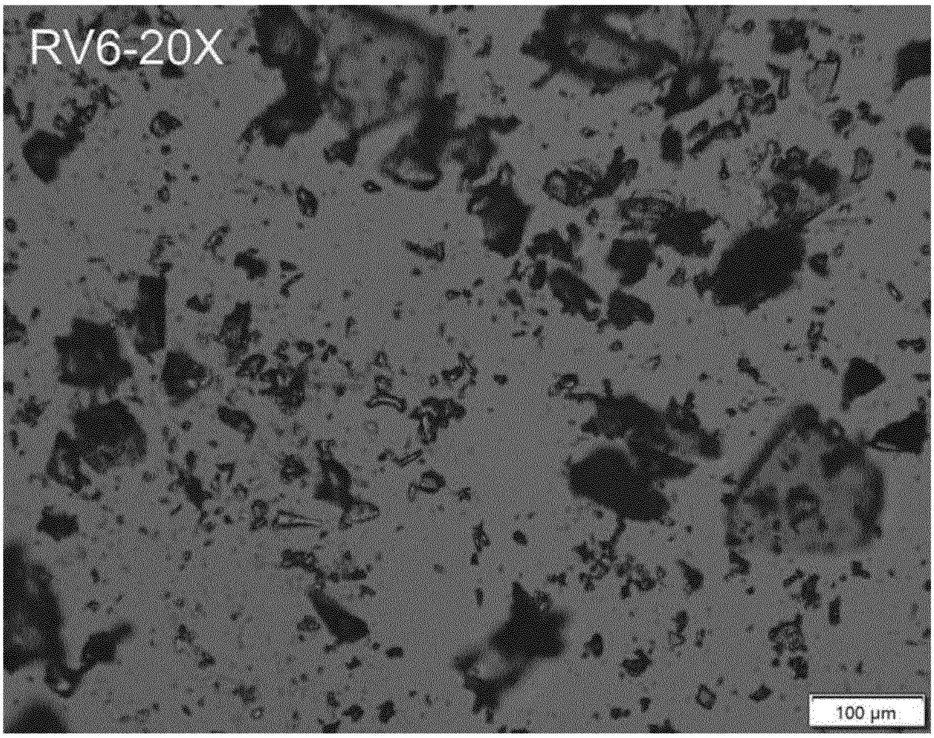


Figure 78

XRPD pattern of the amorphous 1 eq. citrate salt (Sample No. Y11526-10-FD6-1,4-dioxane)

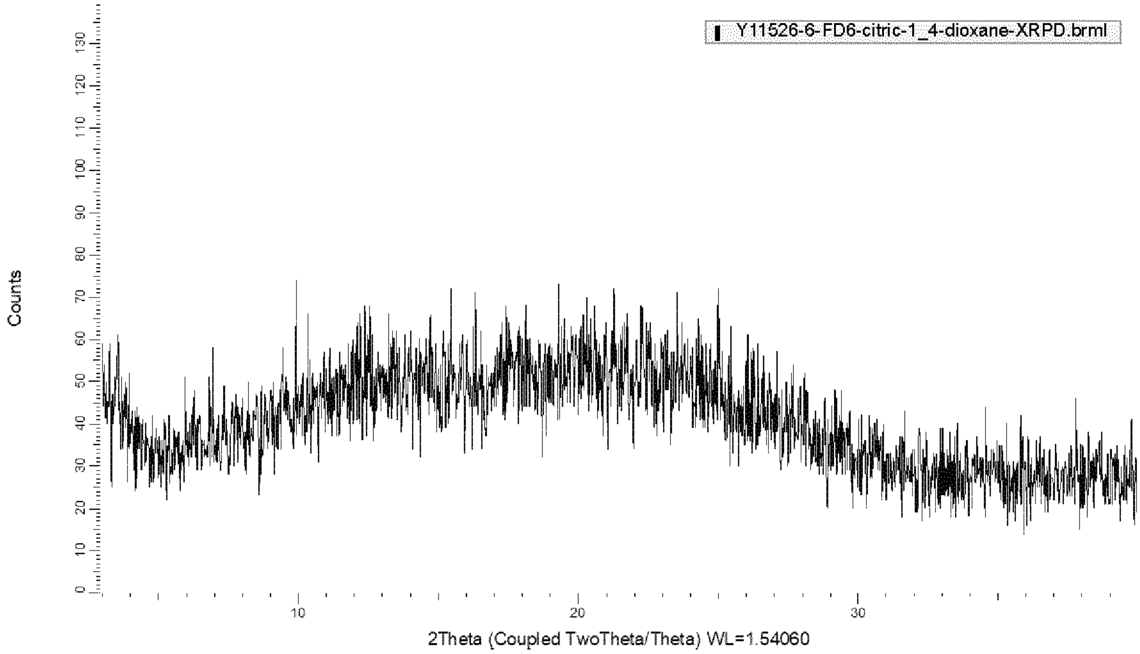


Figure 79

DSC profile of the amorphous 1 eq. citrate salt (Sample No. Y11526-10-FD6-1,4-dioxane)

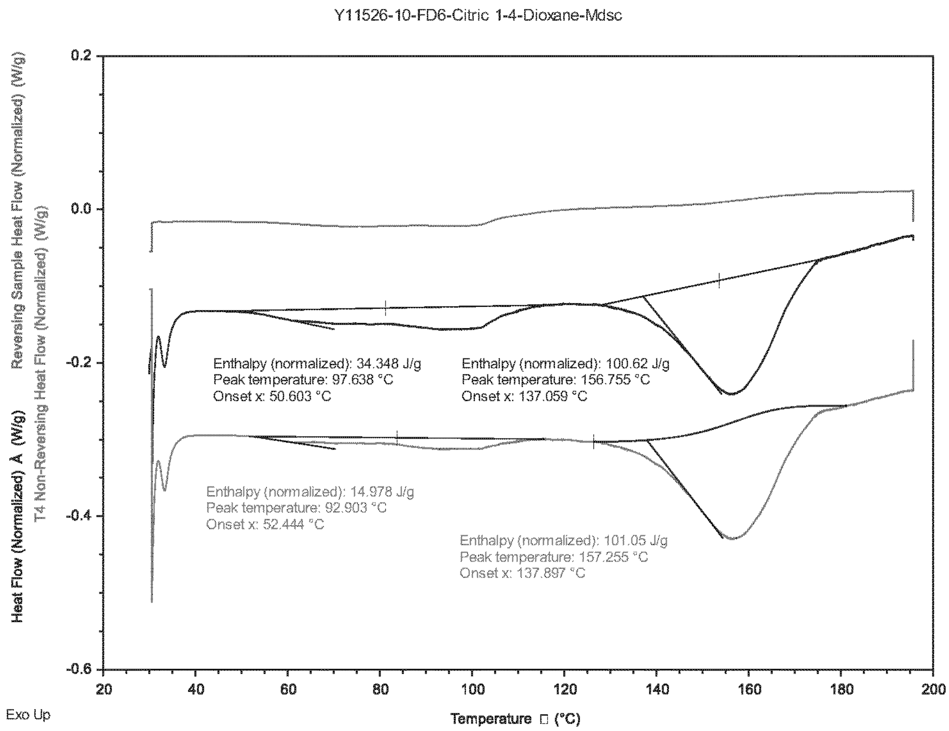


Figure 80

TGA profile of the amorphous 1 eq. citrate salt (Sample No. Y11526-10-FD6-1,4-dioxane)

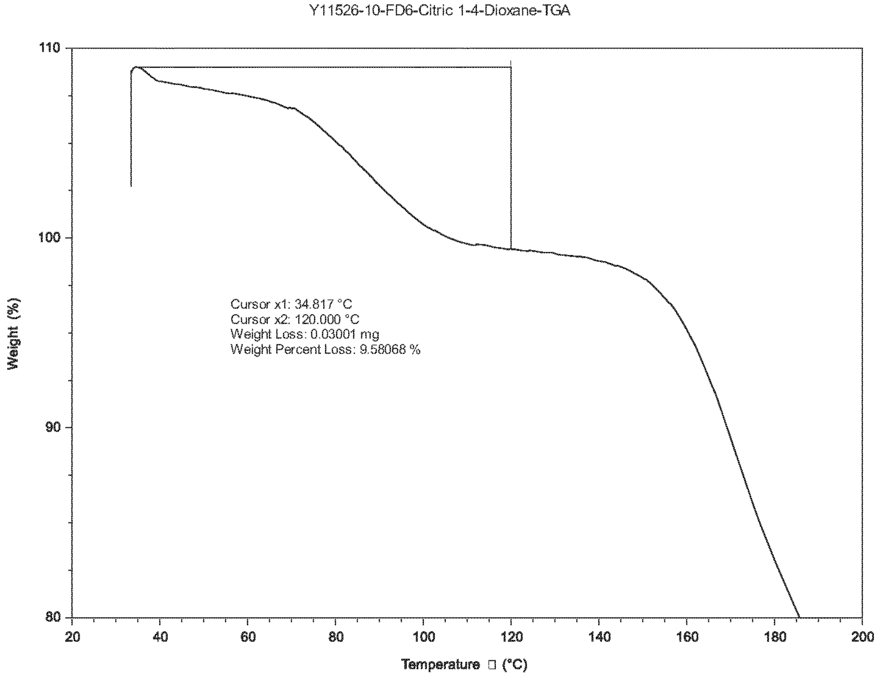


Figure 81

$^1$H-NMR spectrum of the amorphous 1 eq. citrate salt (Sample No. Y11526-10-FD6-1,4-dioxane)

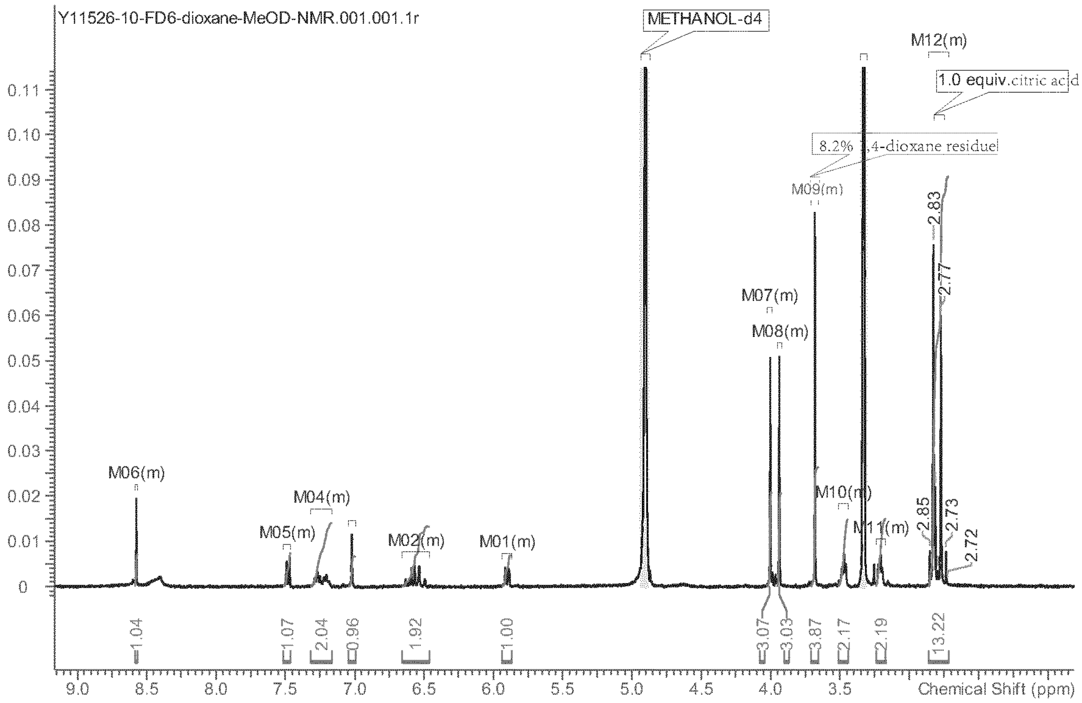


Figure 82

PLM photograph of the amorphous 1 eq. citrate salt (Sample No. Y11526-10-FD6-1,4-dioxane)

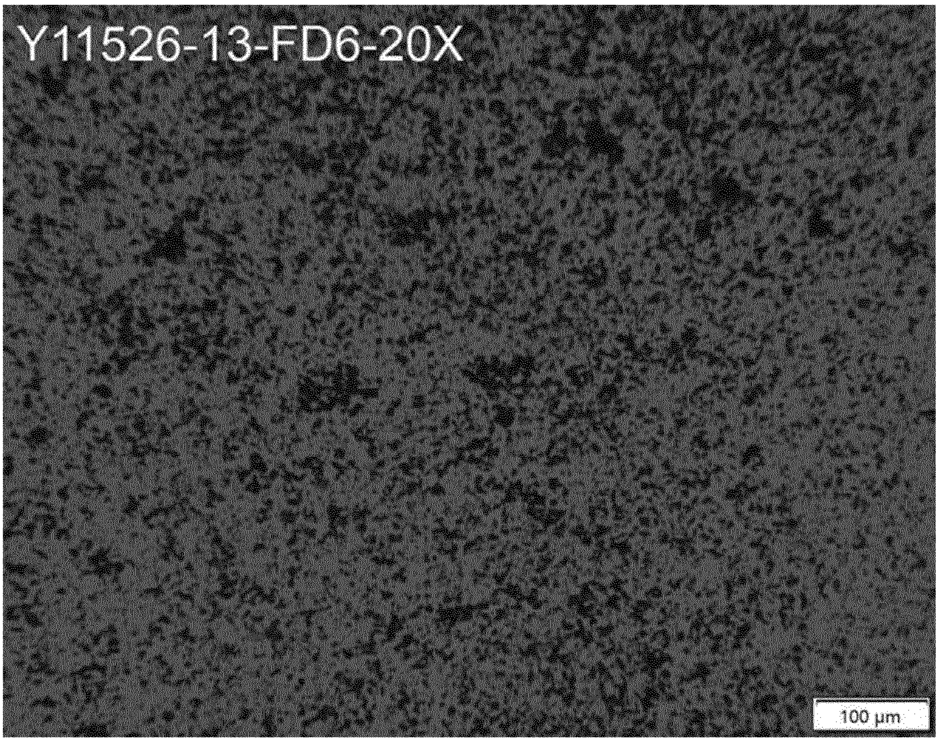


Figure 83

XRPD pattern of the amorphous 1 eq. L-tartrate salt (Sample No. Y11526-10-FD7-1,4-dioxane)

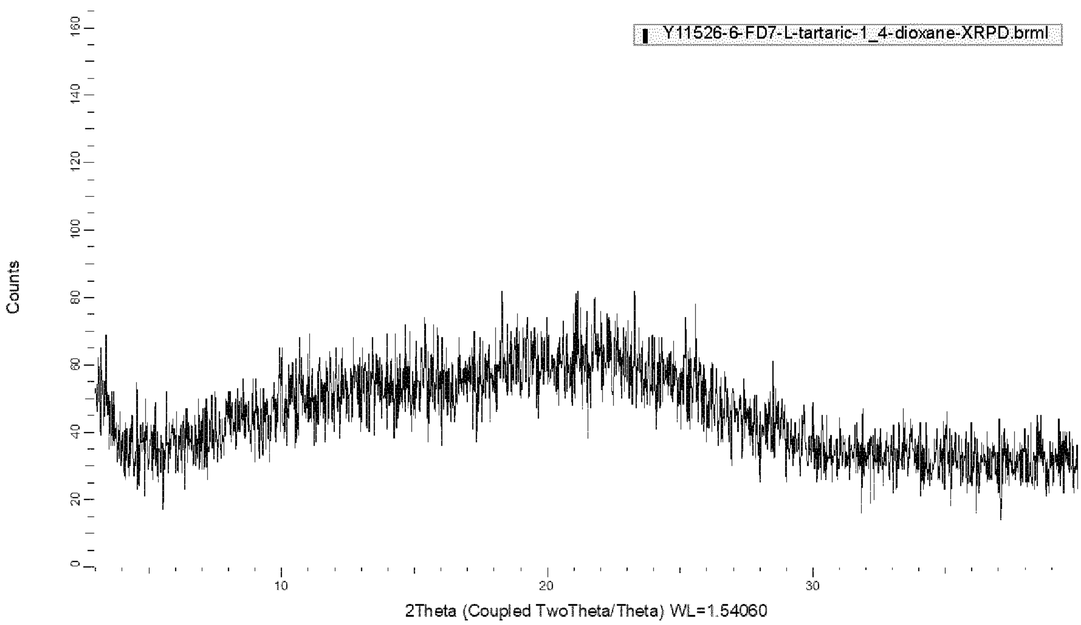


Figure 84

DSC profile of the amorphous 1 eq. L-tartrate salt (Sample No. Y11526-10-FD7-1,4-dioxane)

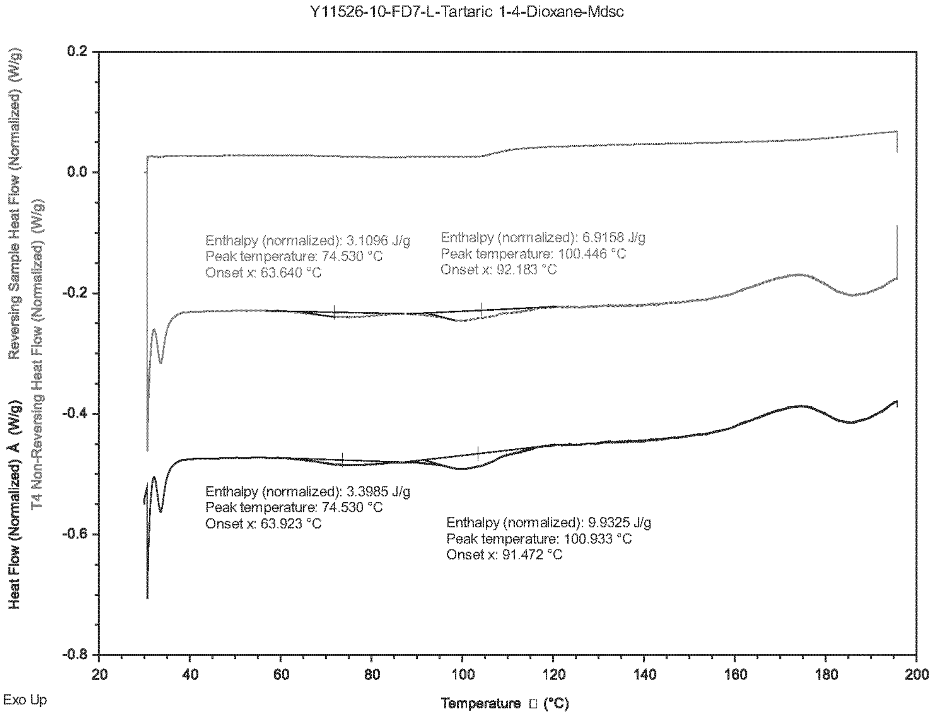


Figure 85

TGA profile of the amorphous 1 eq. L-tartrate salt (Sample No. Y11526-10-FD7-1,4-dioxane)

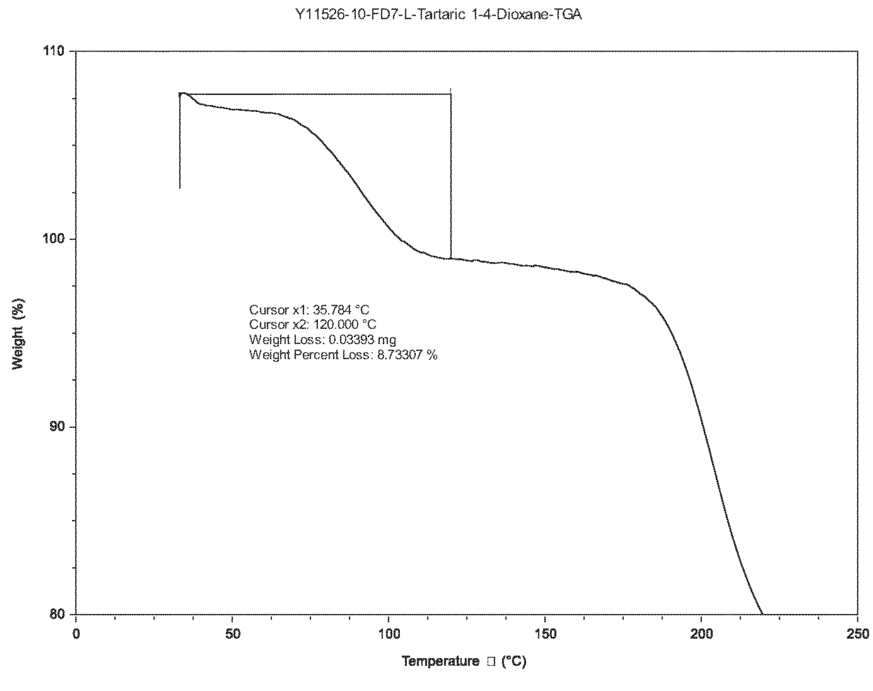


Figure 86

$^1$H-NMR spectrum of the amorphous 1 eq. L-tartrate salt (Sample No. Y11526-10-FD7-1,4-dioxane)

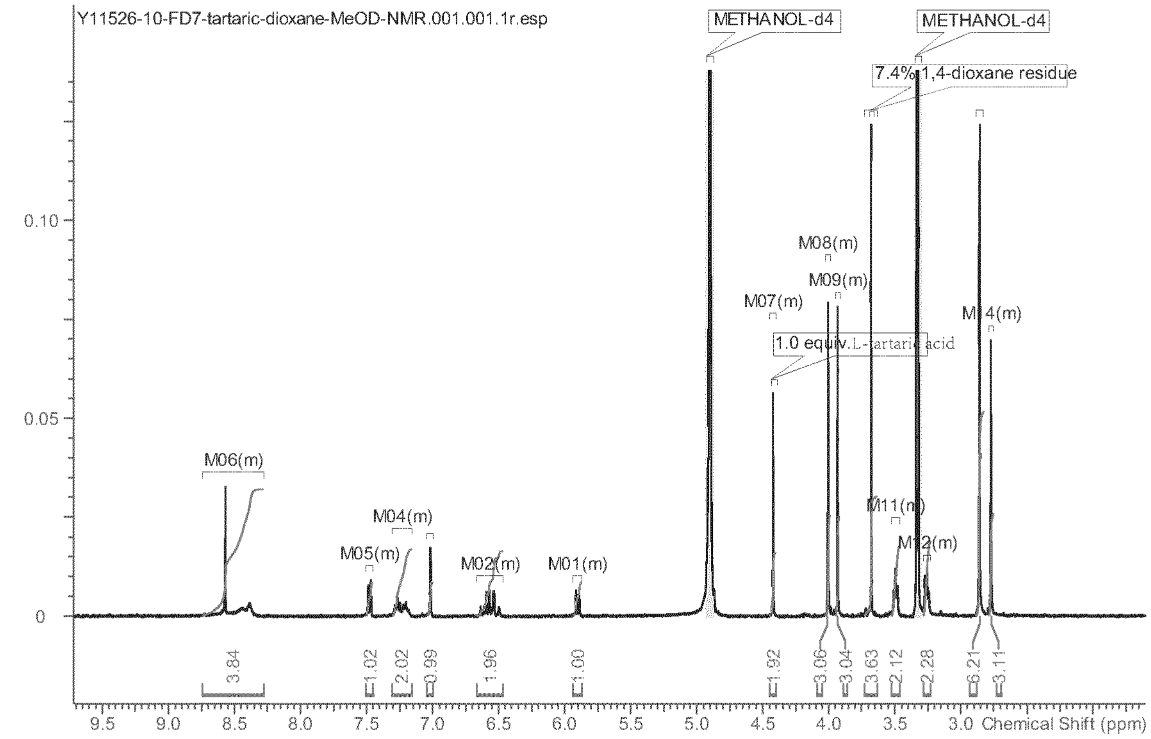


Figure 87

HPLC chromatogram overlay of the amorphous 1 eq. L-tartrate salt (Sample No. Y11526-10-FD7-1,4-dioxane)

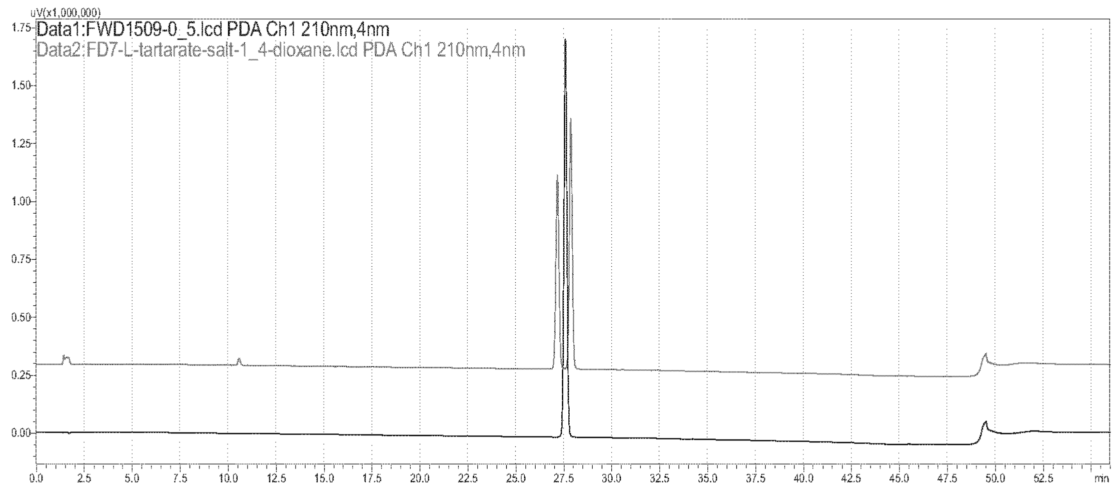


Figure 88

XRPD overlay of the amorphous free base in a solid storage stability test

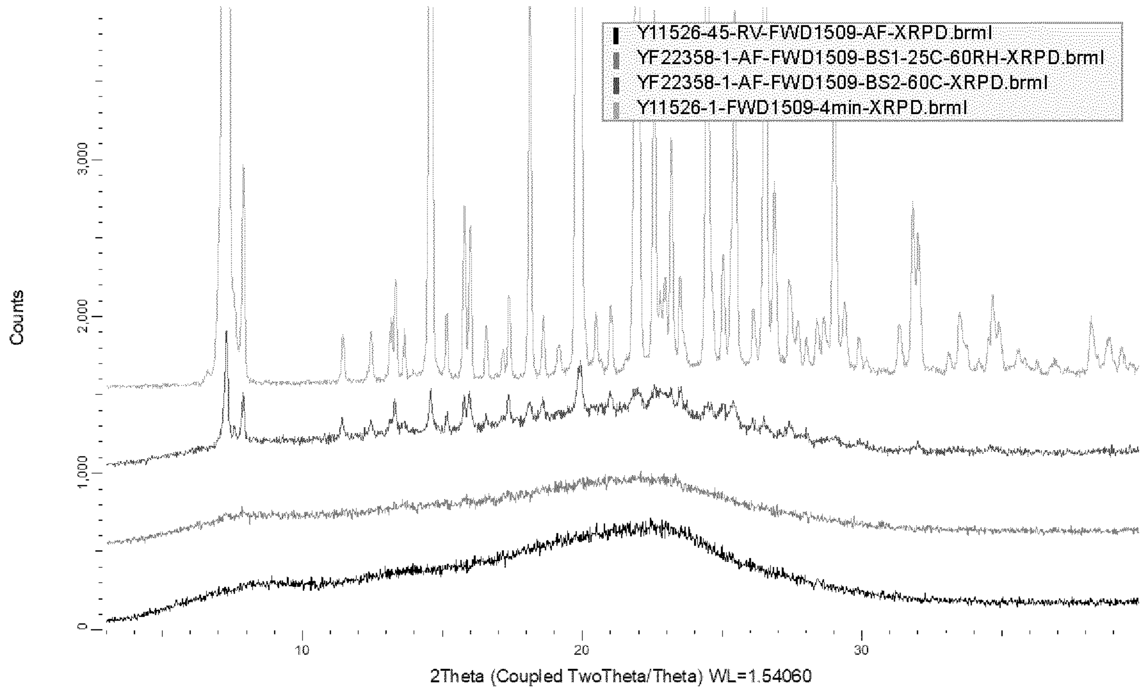


Figure 89

XRPD overlay of the amorphous 1 eq. hydrochloride salt in a solid storage stability test

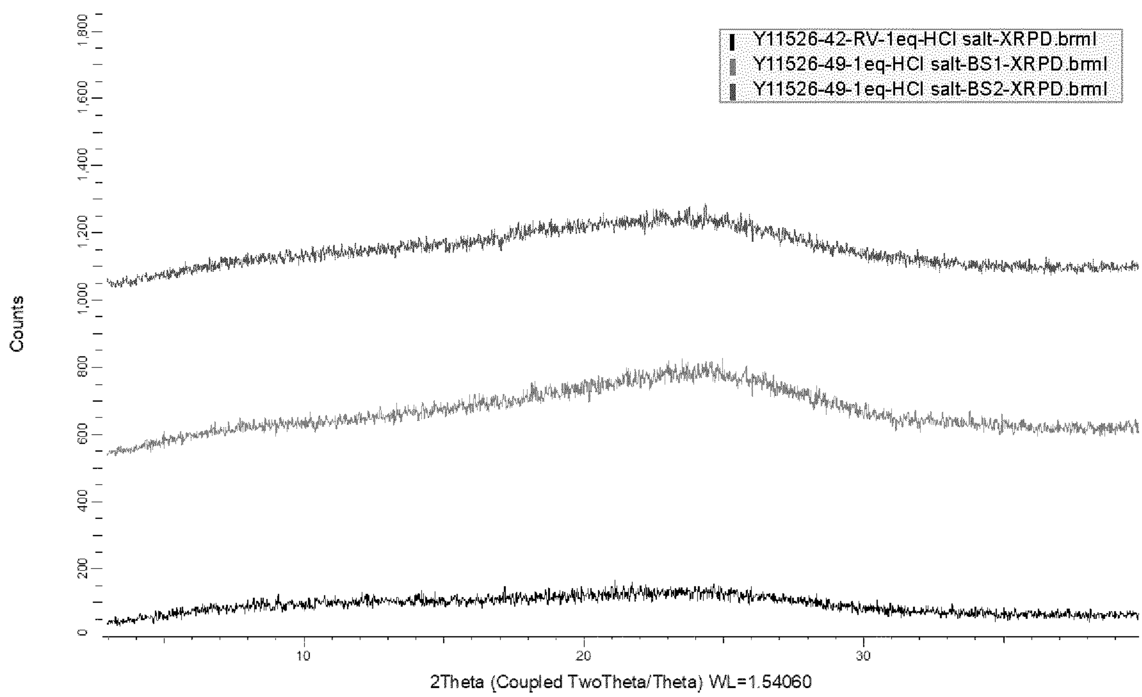


Figure 90

XRPD overlay of the amorphous 2 eq. hydrochloride salt in a solid storage stability test

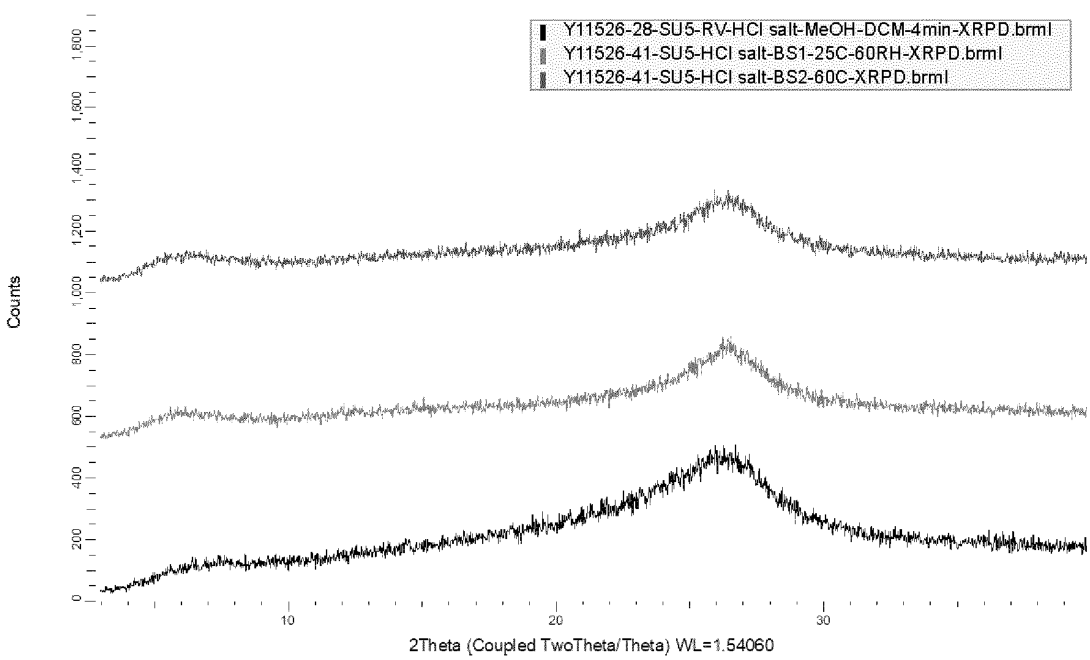


Figure 91

Photograph of the amorphous 2 eq. hydrochloride salt in a solid storage stability test

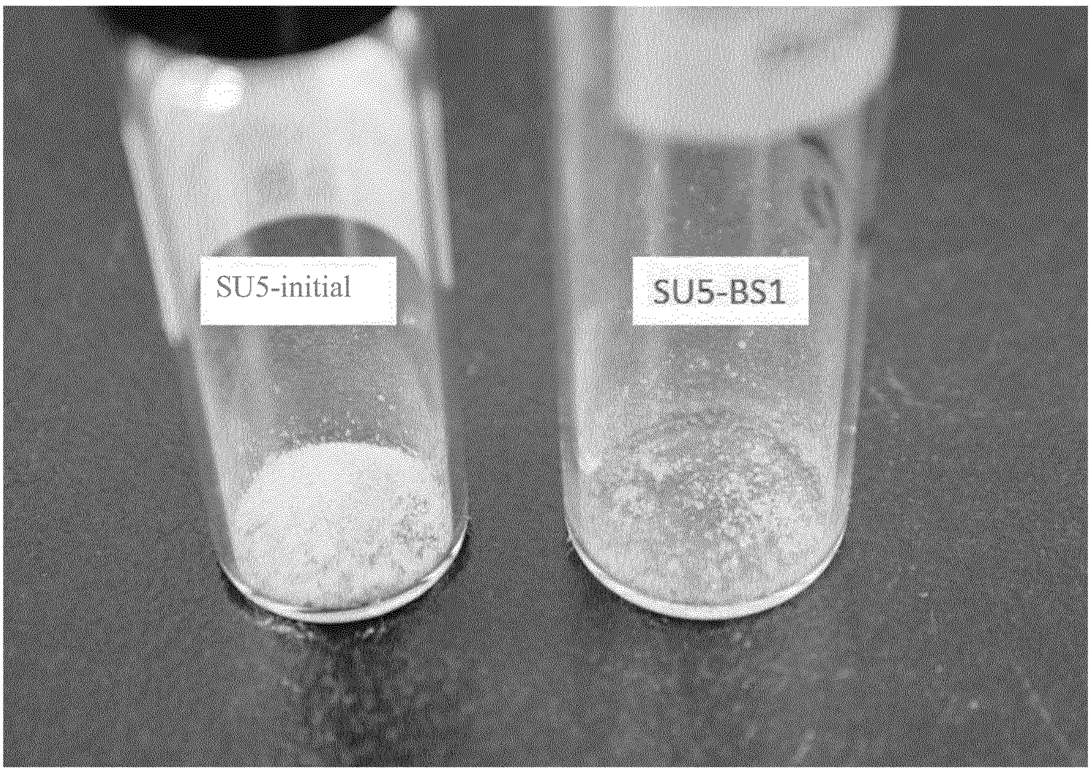


Figure 92

XRPD overlay of the amorphous 1 eq. mesylate salt in a solid storage stability test

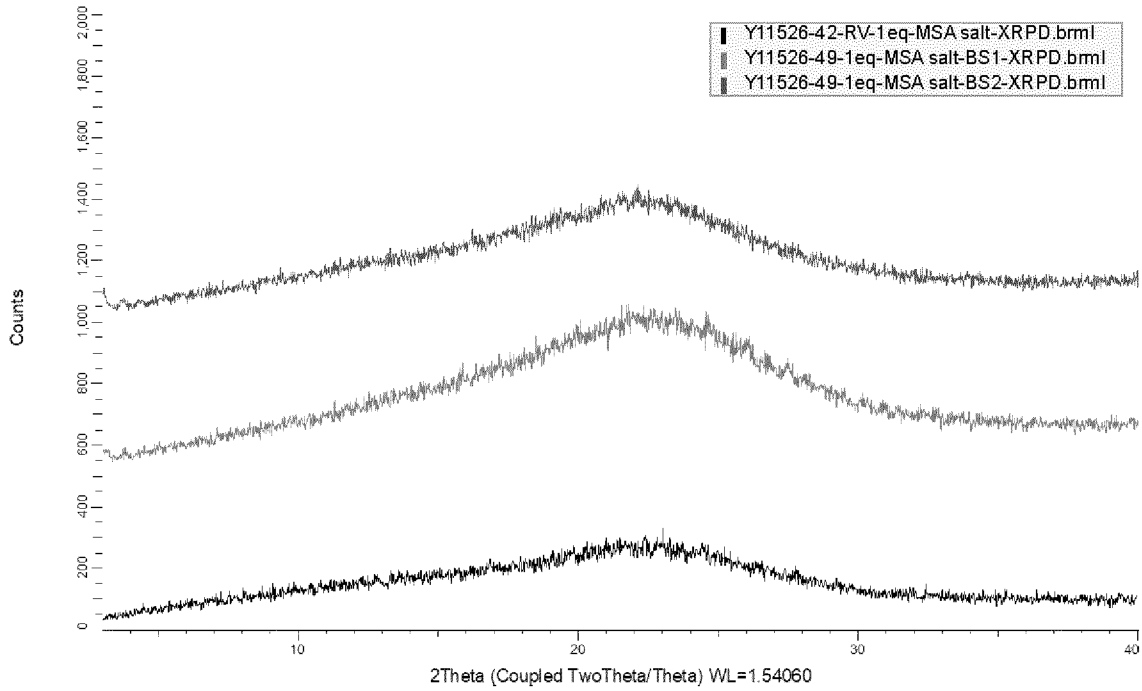


Figure 93

XRPD overlay of the amorphous 2 eq. mesylate salt in a solid storage stability test

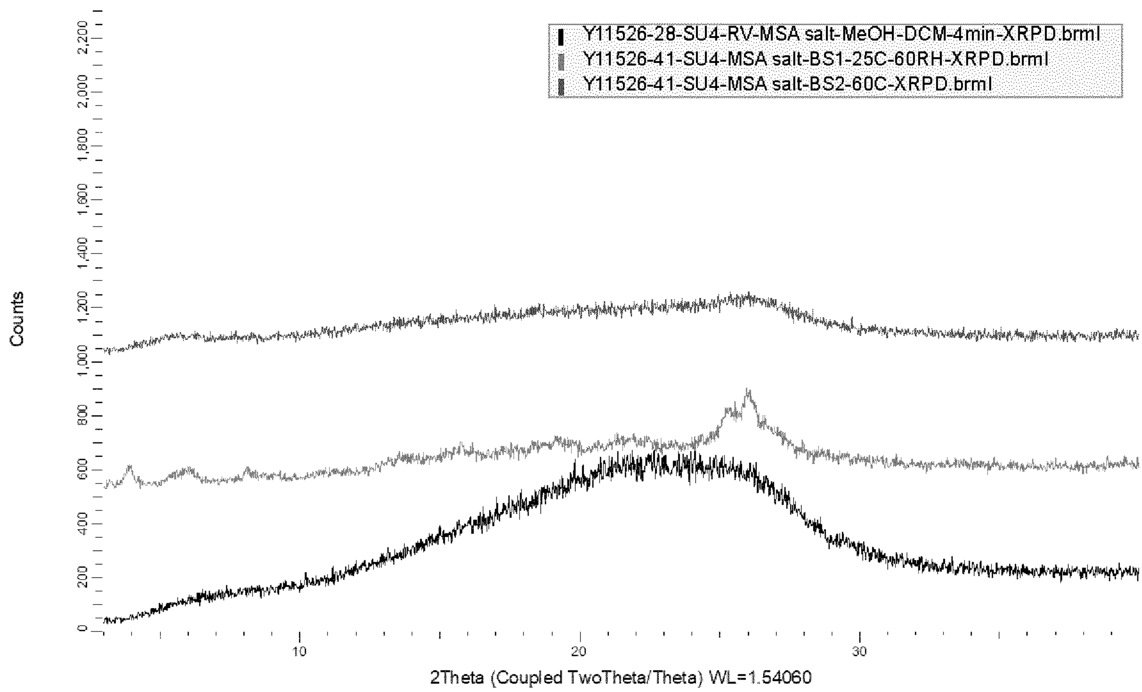


Figure 94

Photograph of the amorphous 2 eq. mesylate salt in a solid storage stability test

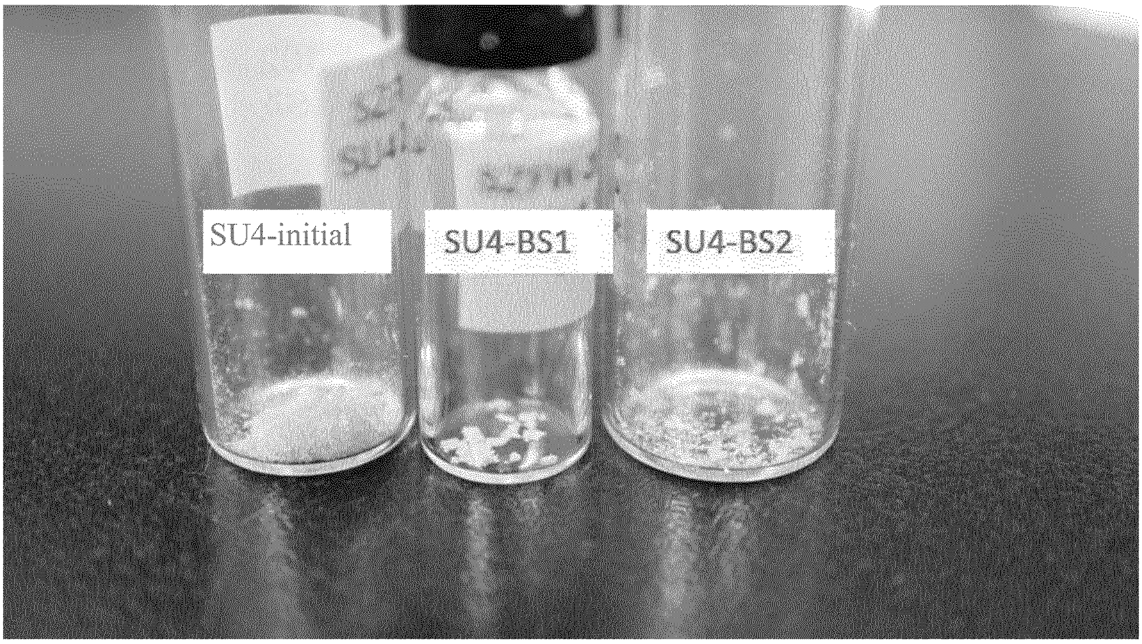


Figure 95

XRPD overlay of the amorphous 1 eq. besylate salt in a solid storage stability test

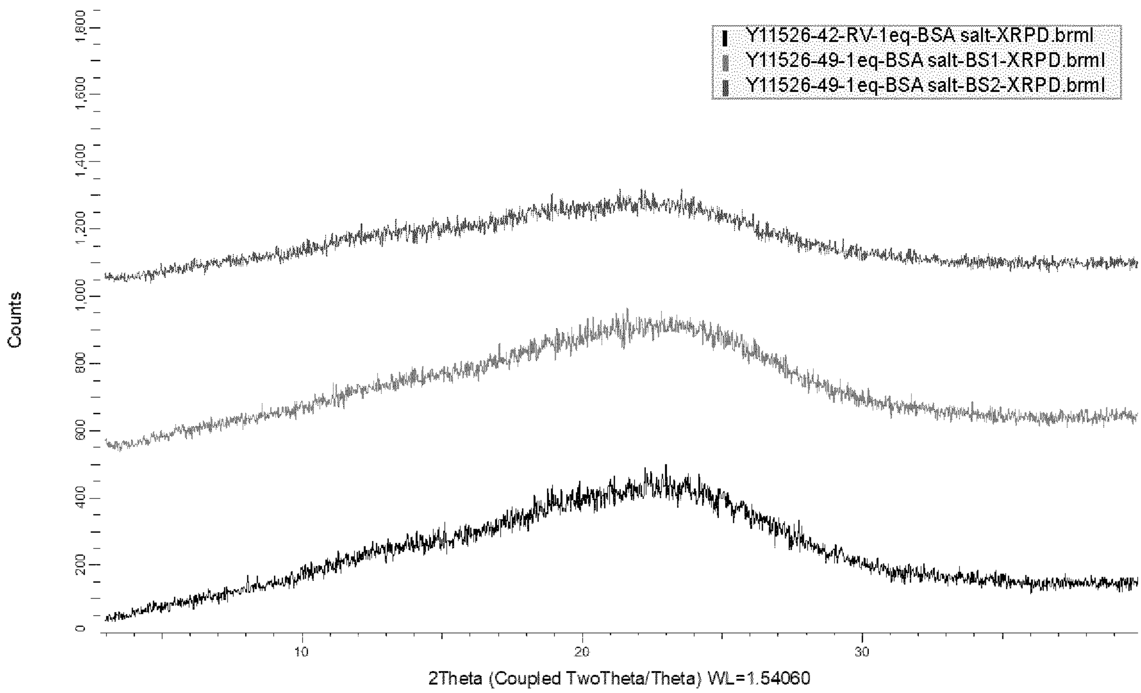


Figure 96

Photograph of the amorphous 2 eq. besylate salt in a solid storage stability test

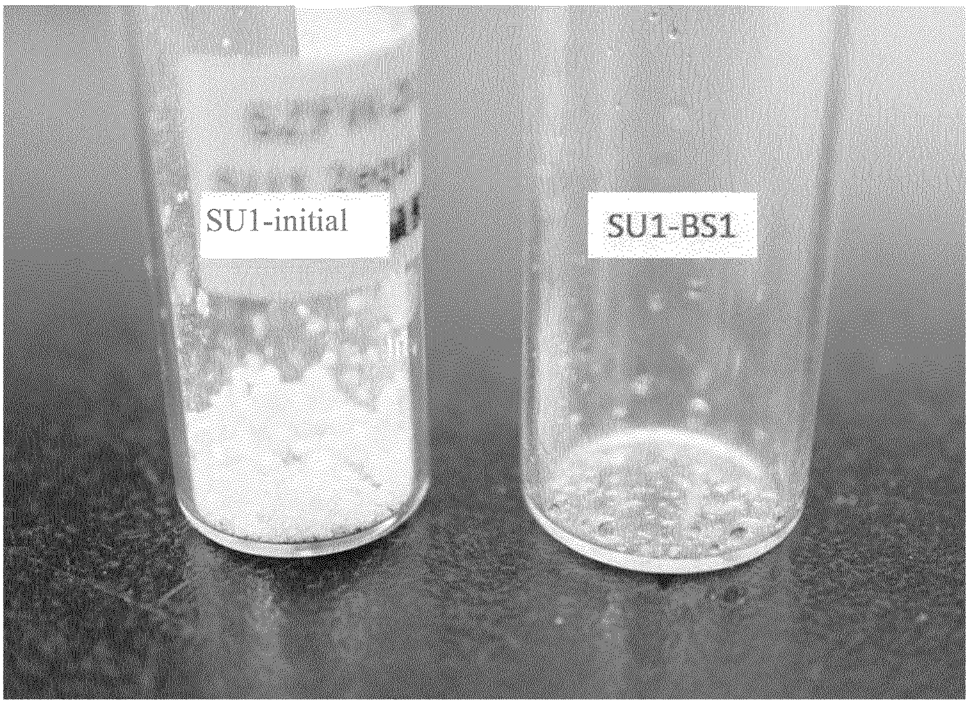


Figure 97

XRPD overlay of the amorphous 2 eq. besylate salt in a solid storage stability test

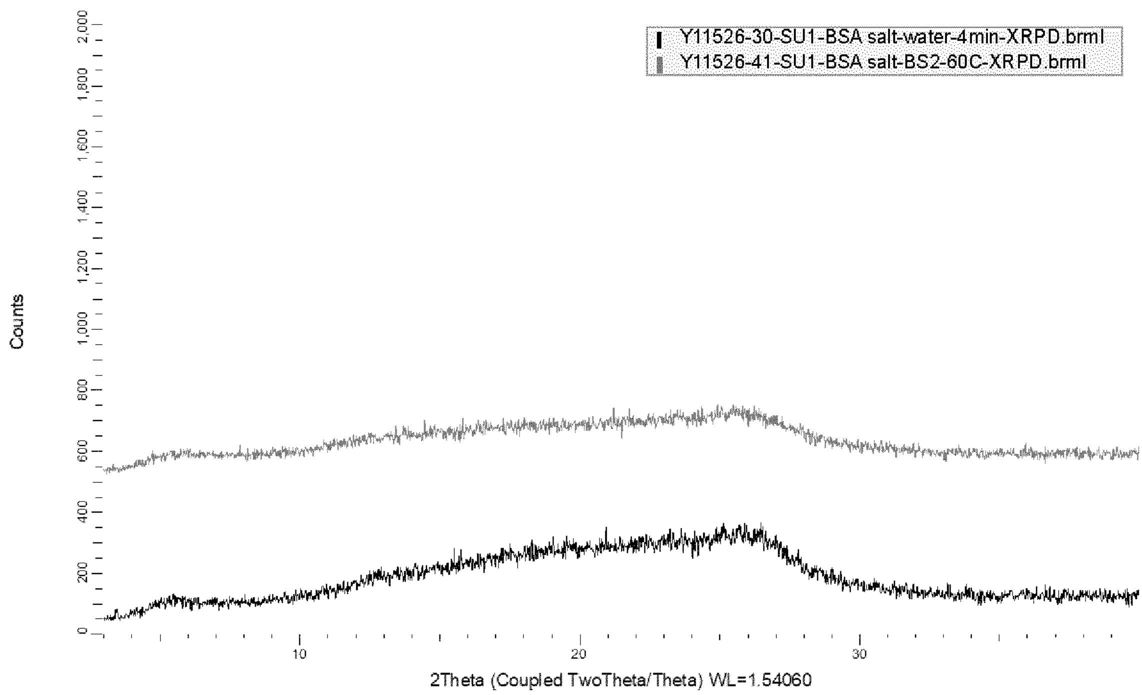


Figure 98

Photograph of the amorphous 1 eq. esylate salt in a solid storage stability test

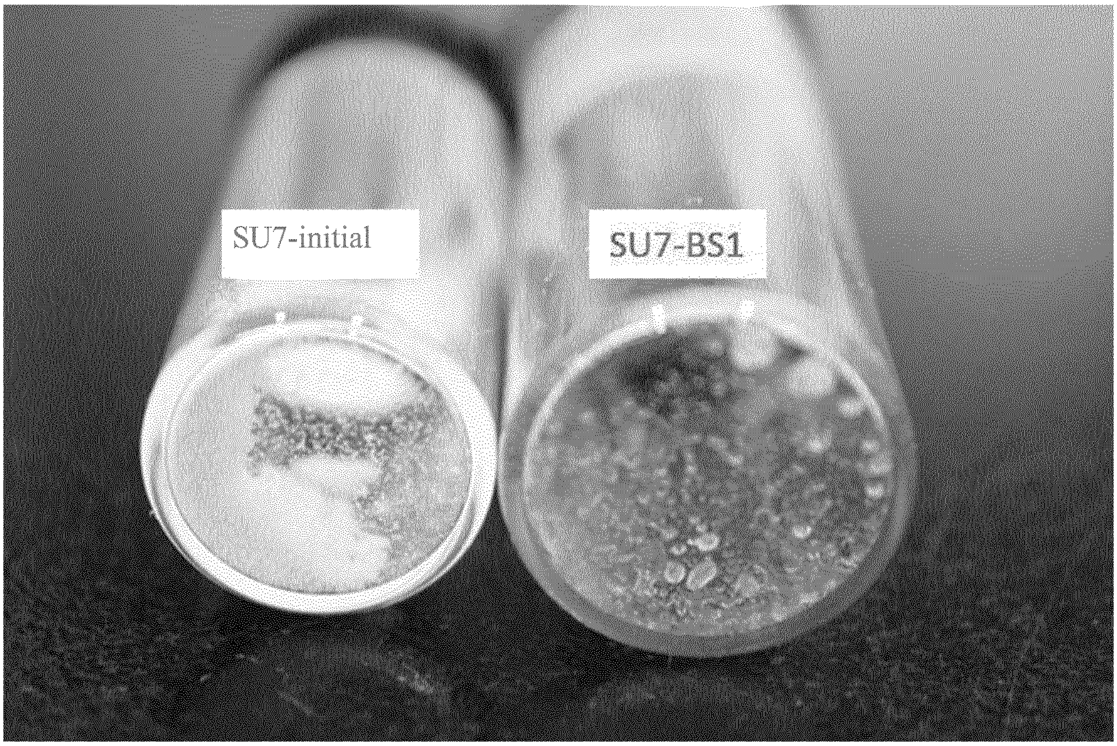


Figure 99

XRPD overlay of the amorphous 1 eq. esylate salt in a solid storage stability test

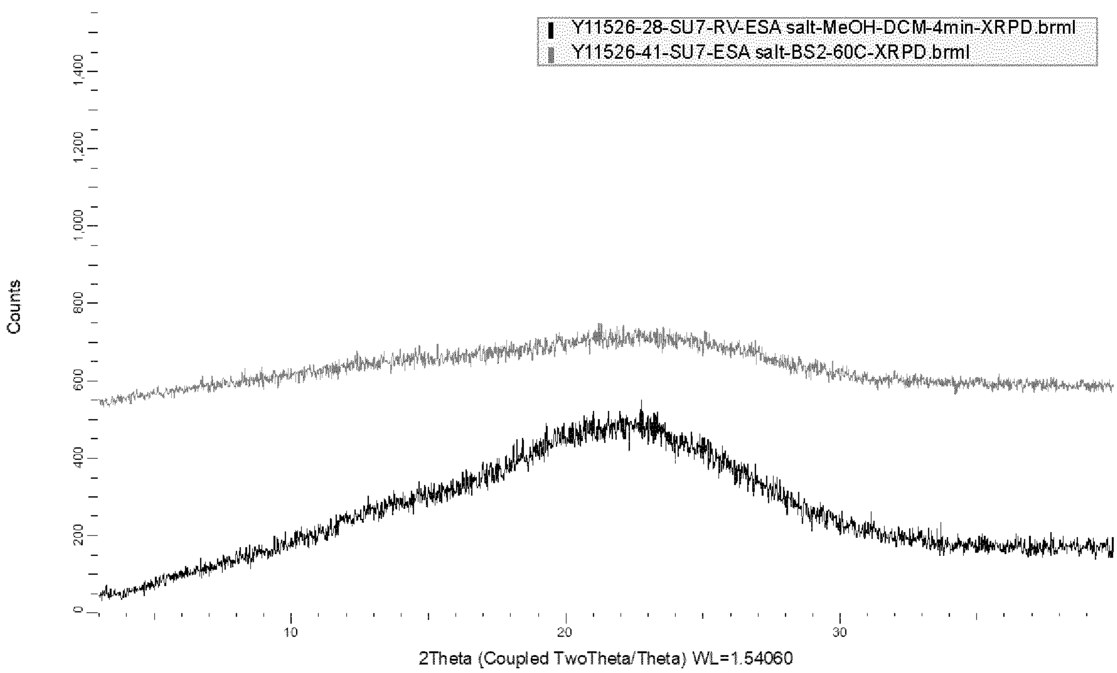


Figure 100

XRPD overlay of the amorphous 1 eq. maleate salt in a solid storage stability test

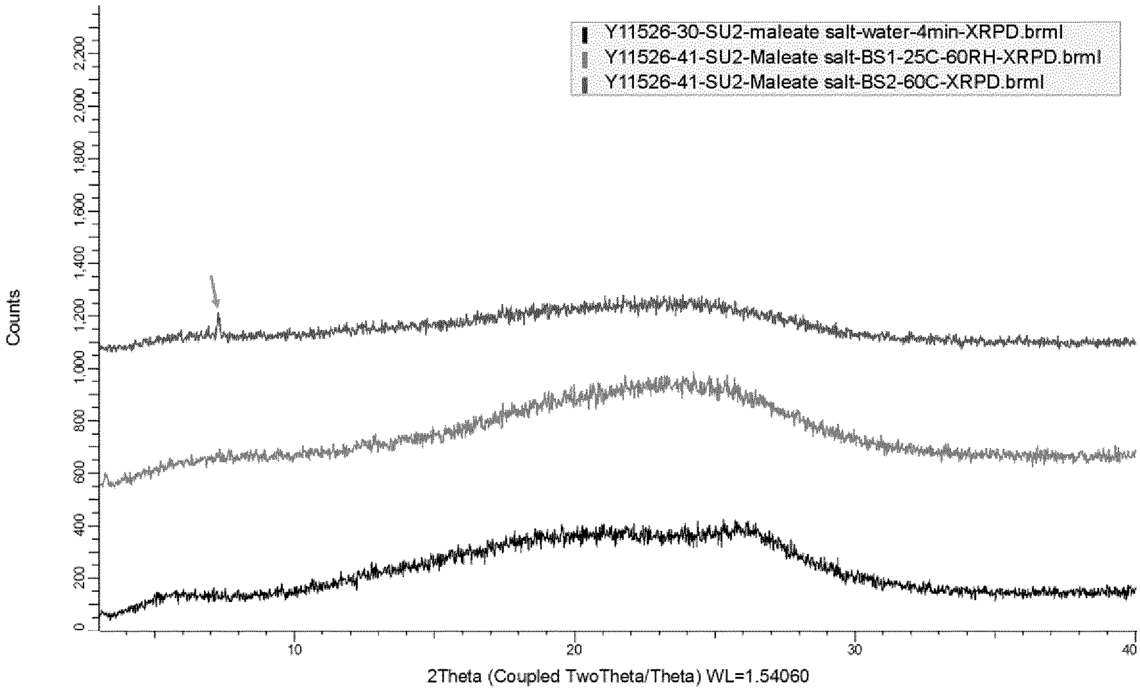


Figure 101

Photograph of the amorphous 1 eq. maleate salt in a solid storage stability test

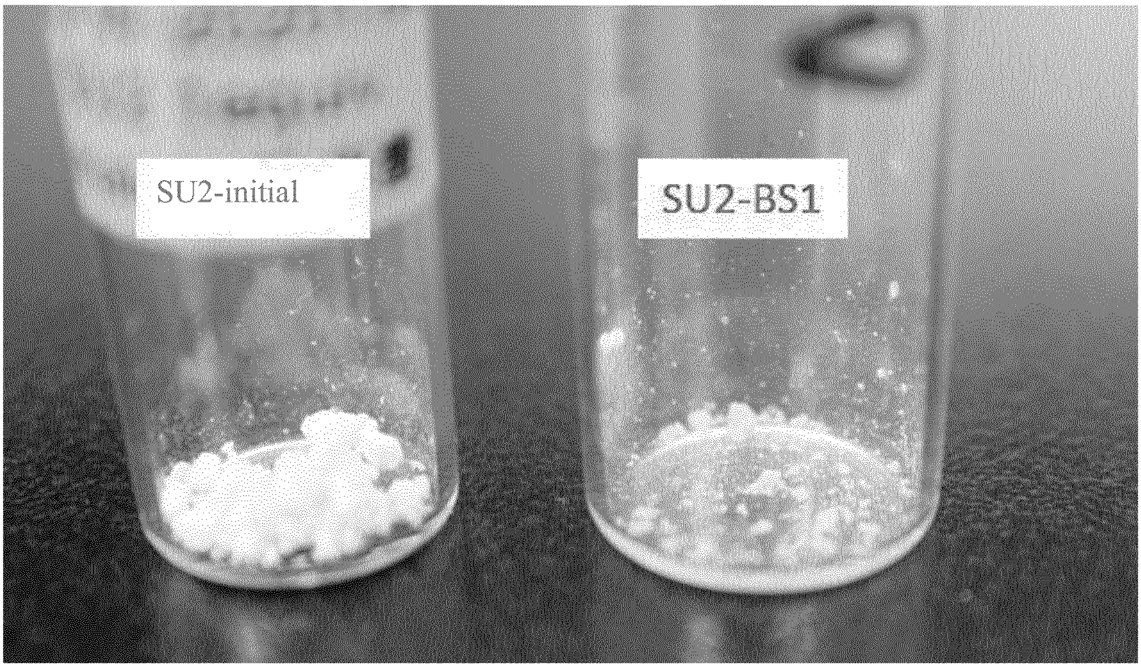


Figure 102

XRPD overlay of the crystalline 1 eq. hydrobromide salt in a solid storage stability test

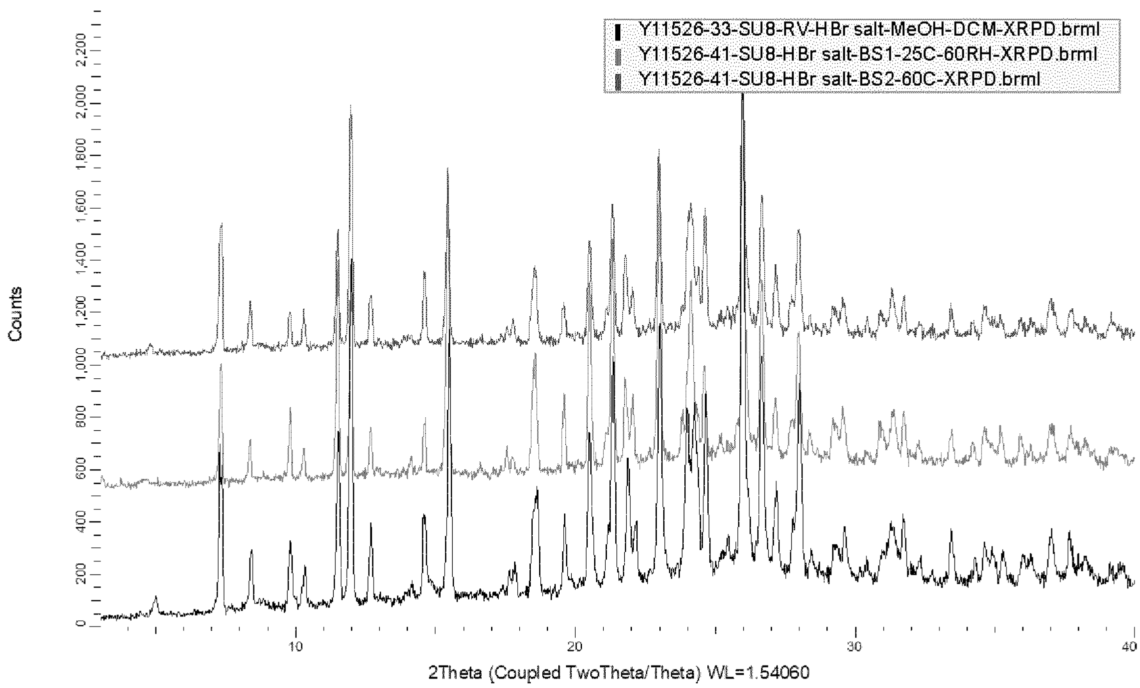


Figure 103

XRPD pattern of the solid of the crystalline 1 eq. hydrobromide salt obtained in a 2 h solid solubility test

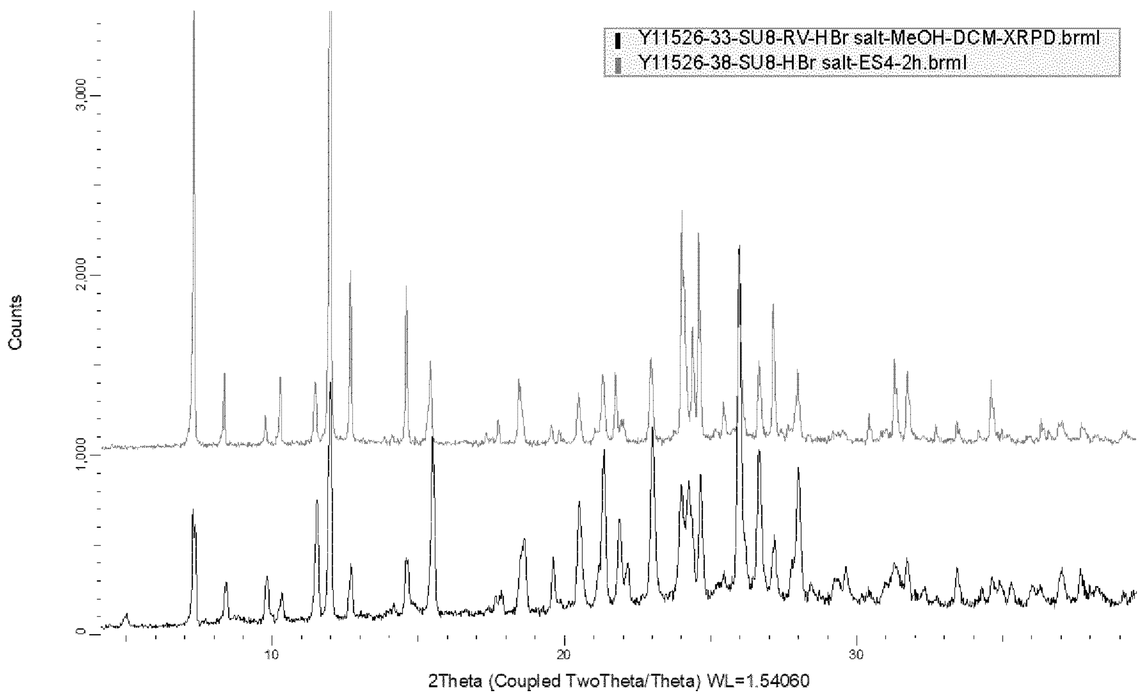


Figure 104

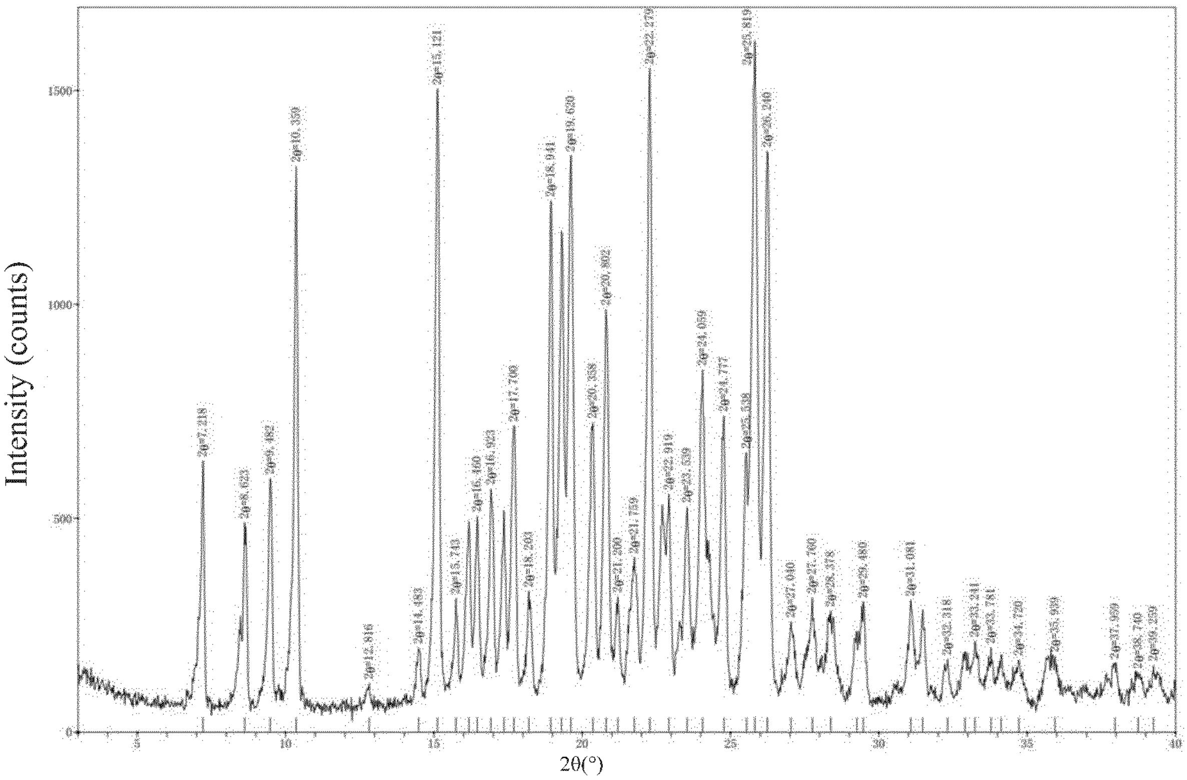


Figure 105

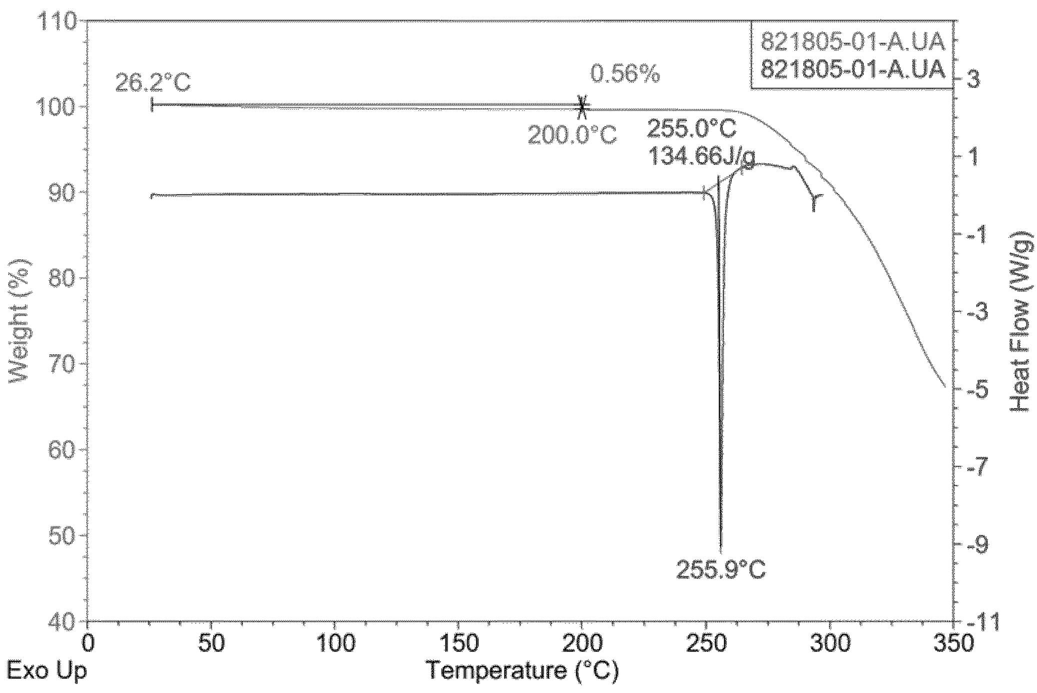


Figure 106

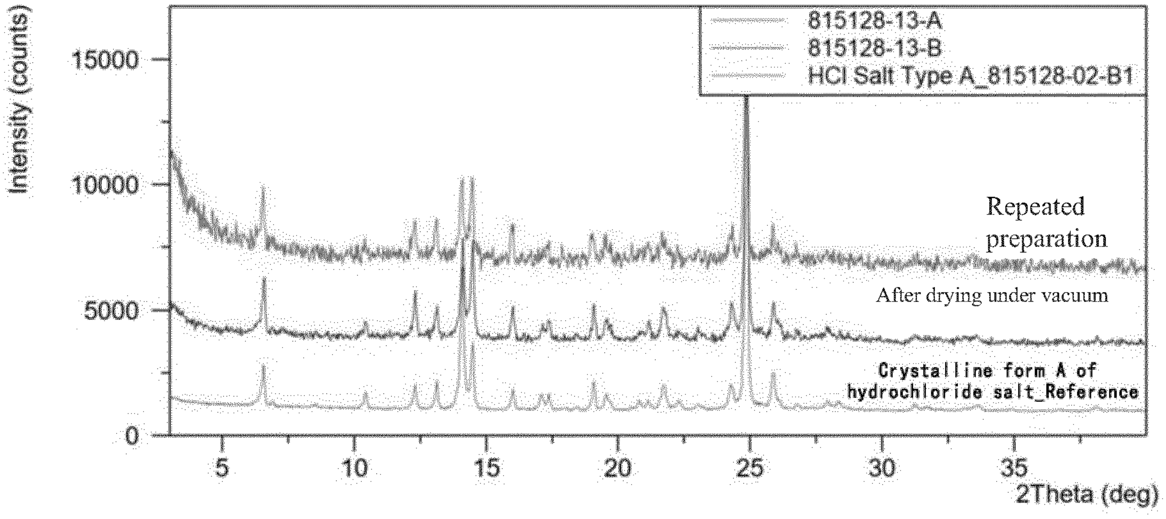


Figure 107

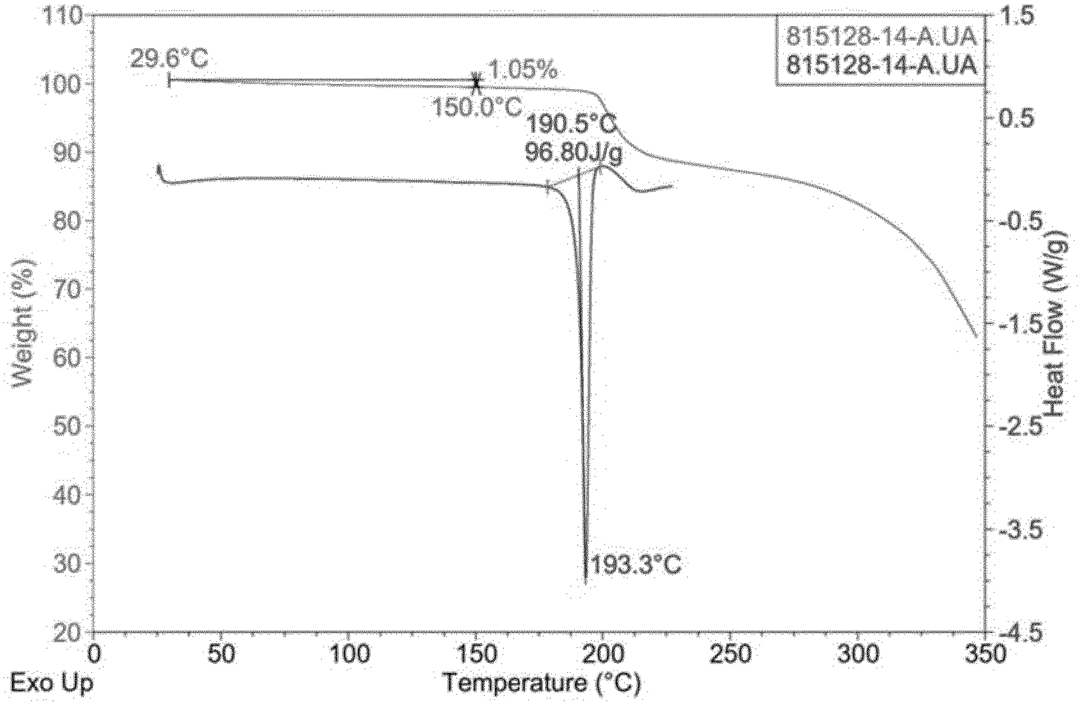


Figure 108

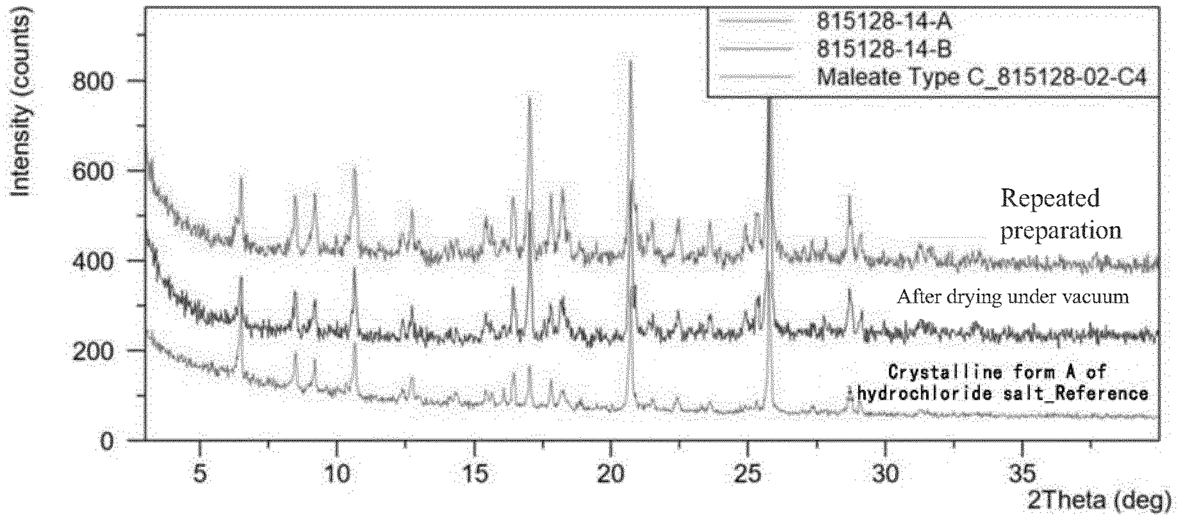


Figure 109

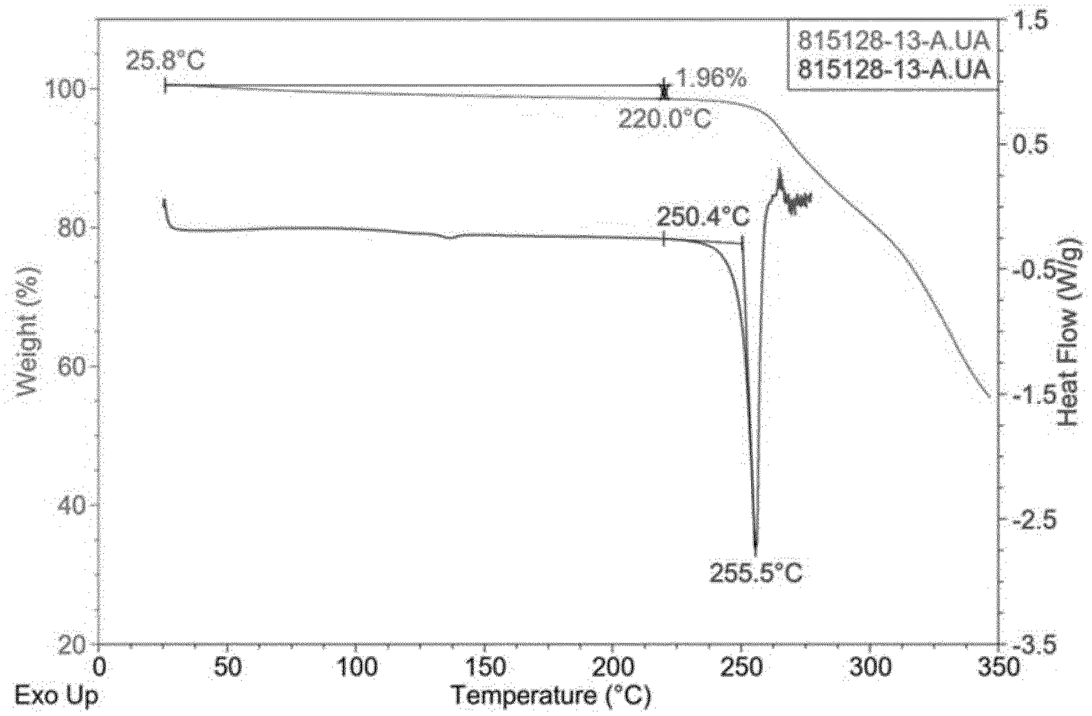


Figure 110

## INTERNATIONAL SEARCH REPORT

International application No.
**PCT/CN2021/101779**

**A. CLASSIFICATION OF SUBJECT MATTER**

C07D 403/04(2006.01)i； A61K 31/53(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D403/-; A61K31/-; A61P35/-

Documentation searched other than minimum documentation to the extent that such documents are included in the fields searched

Electronic data base consulted during the international search (name of data base and, where practicable, search terms used)

CNPAT, CNKI, EPODOC, WPI, REGISTRY(STN), CAPLUS(STN): triazine , crystal?, 三嗪, 晶型, 晶体, 结晶, STN structural formula search, CAS number search, reaction formula search

**C. DOCUMENTS CONSIDERED TO BE RELEVANT**

| Category* | Citation of document, with indication, where appropriate, of the relevant passages | Relevant to claim No. |
|---|---|---|
| X | CN 106928200 A (HUNAN FUWO PHARMACEUTICAL CO., LTD.) 07 July 2017 (2017-07-07) see claims 1, 7, 9, 11-12, description paragraphs [0071], [0079], [0125]-[0128], embodiment 1 | 1-9 |
| Y | CN 106928200 A (HUNAN FUWO PHARMACEUTICAL CO., LTD.) 07 July 2017 (2017-07-07) see claims 1, 7, 9, 11-12, description paragraphs [0071], [0079], [0125]-[0128], embodiment 1 | 10 |
| X | CN 105461695 A (QILU PHARMACEUTICAL CO., LTD.) 06 April 2016 (2016-04-06) see claims 1, 3-4, 6-10, description embodiment 37 | 1-9 |
| Y | CN 105461695 A (QILU PHARMACEUTICAL CO., LTD.) 06 April 2016 (2016-04-06) see claims 1, 3-4, 6-10, description embodiment 37 | 10 |
| Y | CN 102548984 A (AMGEN INC.) 04 July 2012 (2012-07-04) see description paragraphs [0615], [0742] | 10 |
| PX | CN 111747931 A (SHENZHEN FORWARD PHARMACEUTICAL CO., LTD.) 09 October 2020 (2020-10-09) see claims 1, 5-10, description embodiment 1 | 1-9 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 August 2021** | **24 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/101779**

| C. DOCUMENTS CONSIDERED TO BE RELEVANT | | |
|---|---|---|
| Category* | Citation of document, with indication, where appropriate, of the relevant passages | Relevant to claim No. |
| PY | CN 111747931 A (SHENZHEN FORWARD PHARMACEUTICAL CO., LTD.) 09 October 2020 (2020-10-09)<br>see claims 1, 5-10, description embodiment 1 | 10 |

Form PCT/ISA/210 (second sheet) (January 2015)

## INTERNATIONAL SEARCH REPORT

International application No.
**PCT/CN2021/101779**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8**
because they relate to subject matter not required to be searched by this Authority, namely:

   [1] Claim 8 relates to a method for treatment of the living human or animal body under PCT Rule 39.1(4). The present search report is provided on the basis of the pharmaceutical uses of a corresponding compound or a pharmaceutical composition described in claim 8.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/101779**

| Patent document cited in search report | | | Publication date (day/month/year) | Patent family member(s) | | | Publication date (day/month/year) |
|---|---|---|---|---|---|---|---|
| CN | 106928200 | A | 07 July 2017 | None | | | |
| CN | 105461695 | A | 06 April 2016 | CN | 105461695 | B | 27 March 2018 |
| CN | 102548984 | A | 04 July 2012 | US | 8772480 | B2 | 08 July 2014 |
| | | | | PE | 20121159 | A1 | 19 September 2012 |
| | | | | AR | 076486 | A1 | 15 June 2011 |
| | | | | WO | 2010126895 | A1 | 04 November 2010 |
| | | | | EP | 2424859 | B1 | 08 April 2015 |
| | | | | CR | 20110634 | A | 06 January 2012 |
| | | | | MA | 34207 | B1 | 02 May 2013 |
| | | | | JP | 5697662 | B2 | 08 April 2015 |
| | | | | AU | 2010241723 | B2 | 13 December 2012 |
| | | | | NZ | 595572 | A | 26 July 2013 |
| | | | | TW | 201103902 | A | 01 February 2011 |
| | | | | CO | 6440596 | A2 | 15 May 2012 |
| | | | | JP | 2012525395 | A | 22 October 2012 |
| | | | | MX | 2011011335 | A | 18 November 2011 |
| | | | | ZA | 201108101 | B | 25 July 2012 |
| | | | | UY | 32582 | A | 30 November 2010 |
| | | | | EP | 2424859 | A1 | 07 March 2012 |
| | | | | IL | 215731 | D0 | 31 January 2012 |
| | | | | US | 2010273764 | A1 | 28 October 2010 |
| | | | | SG | 175364 | A1 | 28 November 2011 |
| | | | | CA | 2758986 | C | 27 May 2014 |
| | | | | CA | 2758986 | A1 | 04 November 2010 |
| | | | | BR | PI1015262 | A2 | 03 May 2016 |
| | | | | EA | 201101583 | A1 | 30 May 2012 |
| | | | | HK | 1167862 | A1 | 14 December 2012 |
| | | | | KR | 20120007540 | A | 20 January 2012 |
| | | | | CN | 102548984 | B | 25 November 2015 |
| | | | | EA | 019700 | B1 | 30 May 2014 |
| | | | | US | 8362241 | B2 | 29 January 2013 |
| | | | | AU | 2010241723 | A1 | 17 November 2011 |
| | | | | US | 2013079303 | A1 | 28 March 2013 |
| | | | | CL | 2011002691 | A1 | 16 March 2012 |
| CN | 111747931 | A | 09 October 2020 | CN | 111747950 | A | 09 October 2020 |
| | | | | WO | 2020200158 | A1 | 08 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 105461695 B